# EUROPEAN PATENT APPLICATION

(11) **EP 4 006 525 A1**
(43) Date of publication of application: **01.06.2022**
(21) Application number: 20855165.5
(22) Date of filing: 20.08.2020
(51) Int. Cl.: G01N 15/12, G01N 15/14, C12M 1/34, G01N 21/64, C12Q 1/02, G01N 33/48, C12N 11/02

(54) **CELL ANALYZER SYSTEM AND CELL ANALYSIS METHOD**

(30) Priority: 21.08.2019 JP 2019151212
(71) Applicant: WASEDA UNIVERSITY, Shinjuku-ku Tokyo 169-8050 (JP)
(72) Inventor: YASUDA, Kenji, Tokyo 169-8050 (JP)
(74) Representative: Engelhard, Markus
(86) International application number: PCT/JP2020/031491
(87) International publication number: WO 2021/033750

(57) **Abstract**

The present disclosure provides a technique for separating and identifying an abnormal cell in a cell sample derived from a subject. The present disclosure provides a method for analyzing cells using a cell analyzer by utilizing the functions, either alone or in combination, of the cell analyzer, said cell analyzer having a function of continuously concentrating cells, a function of successively arranging the cells in a specific region of a flow channel continuously, a function of simultaneously recognizing the shape of each cell, in a single cell unit on an image base, in the bright field and the shape of fluorescence, and a function of separating and purifying the cells having been recognized on the basis of the shape thereof obtained by correcting the aforesaid shape in accordance with the flow rate of the cells and the light emission data of the fluorescence.

## Description

### [Technical Field]

The present disclosure relates to a device system for analyzing a cell and a cell analysis method.

### [Background Art]

In a biological tissue of a multicellular organism, various cells serve different roles to maintain an overall harmonious function. Alternatively, some of the cells becomes a neoplasm that is different from the surrounding regions upon oncogenic transformation (cancers including tumors are collectively referred to as cancer herein). Meanwhile, a cancerous region cannot be necessarily separated from normal tissue portions that are far away by a boundary, as regions surrounding the cancer is also affected to some extent. Thus, it is necessary to separate and analyze a small number of cells, which are present in a region that is narrow for analyzing the function in an organ tissue, in a short period of time in the simplest manner with minimal loss.

An attempt to separate, re-culture, and induce differentiation of organ stem cells in a tissue to regenerate a tissue of interest and thus an organ is ongoing in the field of regenerative medicine.

When identifying or separating cells, cells need to be distinguished in accordance with some type of an indicator. Generally, cells are distinguished by using the following.
1) Morphological cell classification by visual inspection: examples thereof include tests for bladder cancer or urinary tract cancer and blood atypical cell classification using a test for atypical cells manifested in urine, cancer test using cytodiagnosis in a tissue, and the like.
2) Cell classification using cell surface antigen (marker) staining through a fluorescent antibody technique: a cell surface antigen, which is generally known as a CD marker, is stained with a fluorescently labeled antibody that is specific thereto, and the antigen is used in cell separation using a cell sorter, flow cytometer or tissue staining for a cancer test, or the like. Obviously, they are frequently used not only for medical applications, but also for cell biology research and industrial cell use.
3) Alternatively, examples of stem cell separation include roughly separating cells including stem cells using a fluorescent dye taken up into a cell as a reporter and then actually culturing the cells to isolate the stem cells of interest. Since an effective marker for stem cells has not been established, this method actually cultures cells and uses only cells that are induced to differentiate, to substantially isolate cells of interest.

Separation and collection of a specific cell in a culture in this manner is an important technology for biological and medical analysis. Cells to be separated by the difference in specific gravity of cells can be separated by sedimentation velocity analysis. However, if there is hardly any difference in the specific gravity of cells for visually distinguishing unsensitized cells from sensitized cells, cells needs to be separated one by one based on information from staining with a fluorescent antibody or information from visual inspection.

Examples of technologies for separating cells include cell sorters. A cell sorter is a technology for isolating and dropping fluorescently stained cells in a charged droplet in a unit of a single cell, and applying a high electric field in any direction in the direction of the plane that is normal to the direction of fall while the droplet falls, based on the presence/absence of fluorescence in the cell in the droplet or the size of light scatter, to control the direction of fall of the droplet for fractionating and collecting cells in a plurality of containers placed below (Non Patent Literature 1).

However, the devices for such a technology have problems such as high cost, large size of the device, need for a high electric field of thousands of volts, need for a large amount of sample that are concentrated to a certain concentration or higher, possibility of damaging cells upon preparation of droplets, and samples cannot be directly observed. In recent years, to solve such problems, a fine channel is created using a microprocessing technology to develop a cell sorter, which separates cells flowing in a laminar flow within the channel while allowing direct observation of the cells with a microscope (Non Patent Literatures 2 to 3).

### [Citation List]

### [Patent Literature]

[PTL 1] Japanese Patent No. 3898103
[PTL 2] Japanese Patent No. 4420900
[PTL 3] Japanese Patent No. 4630015
[PTL 4] Japanese Patent No. 4677254
[PTL 5] Japanese Patent No. 5170647
[PTL 6] Japanese Patent No. 5320510
[PTL 7] Japanese Patent No. 5580117
[PTL 8] Japanese Patent No. 5712396

### [Non Patent Literature]

[NPL 1] Kamarck, M.E., Methods Enzymol. Vol.151, p 150-165 (1987)
[NPL 2] A. Wolff et al., Micro Total Analysis, 98, pp. 77-80 (Kluwer Academic Publishers, 1998)
[NPL 3] S. Fiedler et al., Analytical Chemistry, 70, pp. 1909-1915 (1998)

### [Summary of Invention]

### [Solution to Problem]

In view of the heavy focus on confirming the presence of circulating tumor cells (CTCs) in relation to cancer metastasis in clinical settings, the present disclosure has provided and established diagnostic criteria through use thereof as an indicator of cancer metastasis. The present disclosure has solved the need for a very high detection sensitivity in conventional methods, which deem a sample after blood collection as a homogeneous tissue and test the presence/absence of a rare mutated gene therein in view of the diversity/rarity thereof.

In particular, because a gene within a cell and expression were analyzed without confirming whether a fluorescently labeled cancer cell in blood is forming a cell mass with other cells or an isolated single cell in the past, information was obtained as a population average including information on cells other than the target cancer cells, so that accurate information on target cancer cells could not be obtained. Such a problem has been solved by the present disclosure.

The present disclosure does not require diagnosis with a higher S/N ratio by means for sorting and concentrating rare cells and then diagnosing a gene and analyzing the expression with a small amount of cell units, in addition to the collection means in a unit of a single cell.

For cancer cells in blood based on cells in the past, the focus was only on the presence/absence of cells satisfying certain criteria such as the cell size or multinucleate cells, but the present disclosure focused on the change in the distribution of cell sizes obtained by full testing, such as the cell size distribution in blood and the like.

While an approach to test cancer cells include analysis means using image recognition on a cell cluster already filed by the inventors (Patent Literatures 1 to 8), the present disclosure does not require acquiring the shape, circumferential length, and area of a cell more accurately based on an image, and simultaneously acquiring the accurate flow rate of cells and constantly optimizing image acquisition means or cell collection means of a cell sorter in accordance with the obtained flow rate in order to more accurately collect cells.

The present disclosure does not require a preprocessing technology that can continuously concentrate only target candidate sized cells and cell clusters without clogging to observe the full amount in a shorter period of time, in order to observe the full amount of target cells through an image. The present disclosure also does not require a processing technology for constructing an upright microstructure for the manufacture of a member to be used, which was considered necessarily for materializing this technology.

Furthermore, the present disclosure does not require preprocessing for filling cells into a capsule for encapsulating cells in order to prevent contamination upon post-separation/collection genetic analysis or re-culture of cells collected by image observation.

The present disclosure also does not require means for simultaneously and continuously acquiring fluorescence images or light absorption of more wavelengths of cells in order to simultaneously acquire more detailed cell information in image acquisition.

Specifically, the present disclosure provides a technology for separating/identifying abnormal cells in a cell sample derived from a subject.

The present disclosure does not require a separation method that does not damage samples and has a faster response, which was needed for practical application of a cell sorter prepared by using a microprocessing technology with a slow response rate in separating a sample with respect to observation means. The present disclosure has also solved a problem, in which efficiency of separation of a device cannot be improved sufficiently at a low cell concentration unless the cell concentration in a sample solution to be used is increased in advance to a certain concentration or higher. The present disclosure has also solved the problem, in which the concentration of a small amount of sample with another device leads to not only difficulty in collection of the concentrate without any loss, but also an undesirable problem in regenerative medicine or the like such as cell contamination in a complex preprocessing stage.

Cell analysis and separation devices (Patent Literatures 1 to 8) capable of readily analyze/separate cell samples without damaging the samples to be collected by fractionating samples based on the microstructure of the samples and the distribution of fluorescence in the samples by utilizing microprocessing technologies, which were developed by the inventor to overcome such problems, are sufficiently practical cell sorter at the laboratory level, but the present disclosure, above and beyond such devices, can be practically used more effectively with improved efficiency and provides a new technical development for a method of accurately measuring the flow rate of a cell flowing in a microchannel, a method of acquiring an image, a method of image processing, a method of transporting or collecting cells, sample preparation, and other preprocessing.

While detection of cancer tissue has dramatically improved by improvements in MRI (magnetic resonance imaging) and CT (computed tomography), the present disclosure provides an approach for identifying benign/malignant tumor beyond evaluation by biopsy. It is known that malignant tumor metastasizes to other organs due to the ability of cancer cells themselves to infiltrate a blood vessel or lymph node from tissue as an issue with malignant tumor. Malignant tumor cells that circulate peripheral blood in this manner are known as Circulating Tumor Cells (CTCs). It is understood that there are about several hundred cancer cells in 100 thousand blood cells (including red blood cells). Cancinostatic agents for a specific target have been developed one after another in recent years. If the type of malignant tumor in blood can be identified, a carcinostatic agent that effectively kills such cells can be selected. The present disclosure is materialized by a technology for monitoring CTCs flowing in blood, and provides the world's first approach that can quantitatively measure the presence of malignant tumor cells that cause metastatic cancer flowing in blood and therefore can continuously evaluate the effect of the administered carcinostatic agent quantitatively, and not only prevent unneeded administration of a carcinostatic agent or excessive administration of a carcinostatic agent, but also detect the presence/absence of recurrence.

In this manner, the inventor provides a cell analysis device system that can identify the type, condition, and number (blood concentration) of cancer cells flowing in blood with metastatic capability at high speed.

More specifically, the present disclosure provides a cell analysis device system having a function for continuously concentrating cells, a function for continuously disposing the cells in a specific region of a channel, a function for simultaneously recognizing a bright field shape and fluorescence shape by a single cell unit based on an image, and a function for separating/purifying cells through recognition based on information on a shape obtained by correcting the shape to match the flow rate of the cells and emission of fluorescence, and a cell analysis method used in said device.

More specifically, the present disclosure provides the following devices, systems, and methods.

The present disclosure provides a cell analysis device system, comprising:
(A) a first device for processing of purification, concentration, stain, and/or wash of cells in a candidate size region from a cell sample solution from a subject;
(B) a second device for preparing a capsule particle encapsulating the cells processed by the first device in a capsule;
(C) a third device for acquiring and distinguishing images of the cells processed by the first device or the cells encapsulated in a capsule particle by the second device, continuously acquiring a flow rate of the cells as flow rate data, acquiring an accurate cell shape based on the flow rate data, continuously analyzing information in the images of the cells based on the cell shape, outputting a distribution of cell information for an entire amount of test sample, and distinguishing and collecting a target cell; and
(D) a control/determination unit for controlling an operation of each of the first to third devices to perform determination on the cell sample solution.

In the cell analysis device system of the present disclosure,
(a) the first device comprises a chamber comprising a membrane filter for concentrating, staining, and washing cells obtained from a cell sample solution from a subject, containers respectively housing a solution comprising the cells, a staining solution, and a detergent, and a cell concentration/staining/washing mechanism for sequentially introducing each solution in each of the containers into the chamber, or
(a') the first device comprises an alternating electric field application mechanism comprising: a pillar array capable of selectively and continuously fractionating the cells in the cell sample solution by size, wherein the pillar array has a space adjusted to match a cell size to be fractionated and is disposed in a microchannel with a slope with respect to a flow in a channel; and a pair of electrodes, which can apply a sinusoidal alternating electric field to a microchannel, disposed to oppose both side wall surfaces that are orthogonal to the microchannel;
(b) the second device comprises a capsule particle construction mechanism for constructing capsule particles of alginic acid comprising cells by discharging a solution comprising alginic acid in a sol state and the cells from an outlet of a microtube into a solution comprising a divalent ion, and comprises a capsule particle size sorting mechanism for applying a small current to the inside and outside of the microtube, and measuring and controlling a discharge rate from a change in a value of resistance to align particle sizes of capsule particles of alginic acid, and a cell/capsule particle collection mechanism for distinguishing a capsule particle comprising a cell and a capsule particle that does not comprise a cell to selectively collect a capsule particle comprising a cell; and
(c) the third device is a module having an image detecting single cell separation/purification unit (also described as "cell sorting unit" hereinafter) comprising a channel for allowing a sample solution of cells comprising a target cell or capsule particle containing a target cell to flow, the channel comprising a merging region where the channel merges with a sheath solution from both sides for allowing cells or capsule particles arranged in one line to flow downstream, a detection/sorting region where the cells or capsule particles aligned in one line are detected and the target cell is sorted, a combination of channels for collecting target cells by applying an ionic current to move cells or capsule particles comprising a cell and selectively moving the cells or capsule particles to a branched channel continuing from the merging region, and an ionic current application tool for applying an ionic current, wherein the module can comprise, at the merging region, an optical tool for determining a flow rate of a cell and an analysis tool for acquiring and analyzing a characteristic from an image of a cell corrected based on an optically obtained flow rate of a cell.

In the cell analysis device system of the present disclosure,
the third device is a module having an image detecting single cell separation/purification unit comprising a channel for allowing a sample solution comprising a target cell or capsule particle to flow, the channel having a merging region where the channel merges with a sheath solution from both sides for allowing the cells or capsule particles arranged in one line to flow downstream, and an observation region where the cells or capsule particles aligned in one line are detected,
wherein the module can comprise: a light source that can be temporally controlled so that light is emitted during an irradiation period in an irradiation region for irradiating light of two or more different wavelengths for a shorter period of time than an interval of image capture time with a camera, with each different length of time; a condenser optical system for irradiating light from the light source onto the irradiation region; an image capturing camera mechanism for splitting an obtained image of two or more different wavelengths by a difference in wavelengths and acquiring images as images of each wavelength; a cell flow rate acquisition mechanism for acquiring a flow rate of a cell from comparing a difference in irradiation periods of the light source and lengths of the resulting images of two or more wavelengths in a direction of flow of a cell; a cell shape correction mechanism for correcting obtained cell shape information from the acquired flow rate of a cell; a cell analysis tool for acquiring and analyzing a characteristic of a cell from a shape of a cell corrected based on an optically obtained flow rate of a cell; and an image blur suppression mechanism for suppressing an image blur by controlling a flash time of a light source in a relation of "flash time of a light source = pixel size of a camera acquiring an image/obtained flow rate of a cell" from the obtained flow rate.

In the cell analysis device system of the present disclosure,
the third device is a module having an image detecting single cell separation/purification unit comprising, downstream of the observation region, a combination of channels for collecting target cells or capsule particles comprising target cells by applying an ionic current to move cells or capsule particles and selectively moving the cells or capsule particles to a branched channel continuing from the merging region, and an ionic current application tool for applying an ionic current, wherein the module can comprise an application timing controlling tool for controlling a timing of applying an ionic current to a cell or capsule particle to be collected based on a flow rate acquired by the cell flow rate acquisition mechanism in the merging region.

In the cell analysis device system of the present disclosure,
the third device can use fluorescence for the light source and an observed image.

In the cell analysis device system of the present disclosure,
the third device is a module having an image detecting single cell separation/purification unit comprising a channel for allowing a sample solution comprising cells or capsule particles to flow, the channel having a merging region where the channel merges with a sheath solution from both sides for allowing the cells arranged in one line to flow downstream, and an observation region where the cells or capsule particles aligned in one line are detected,
wherein the module can comprise an image reconstruction mechanism having a condenser optical system for continuously irradiating light for observing cells or capsule particles onto the irradiation region; a flow rate measuring one dimensional photosensor array disposed along a flow of cells or capsule particles on an image acquiring surface for forming the resulting image; and a cell image acquiring one dimensional photosensor array with a length that can cover a channel width in an orientation that is orthogonal to a flow of a cell and acquire all cell images at a bottom end thereof, wherein a flow rate is computed from measured moving rate information on a cell image of the flow rate measuring one dimensional photosensor array and the rate information and data acquisition time are combined to reconstruct two dimensional image information from information of the cell image acquiring one dimensional photosensor array.

In the cell analysis device system of the present disclosure,
the third device is a module having an image detecting single cell separation/purification unit comprising, downstream of the observation region, a combination of channels for collecting target cells or capsule particles comprising target cells by applying an ionic current to move cells or capsule particles and selectively moving the cells or capsule particles to a branched channel continuing from the merging region, and an ionic current application tool for applying an ionic current, wherein the module can comprise an application timing controlling tool for controlling a timing of applying an ionic current to cells or capsule particles to be collected based on a flow rate acquired by calculating the flow rate in the merging region.

In the cell analysis device system of the present disclosure,
the third device can use fluorescence for the light source and an observed image.

In the cell analysis device system of the present disclosure,
the third device can have an image blur suppression mechanism that can simultaneously acquire images at different image formation heights by disposing and arranging in parallel at one or more different heights, in addition to the cell image acquiring one dimensional photosensor array on the image acquiring surface.

In the cell analysis device system of the present disclosure,
the third device can have an image splitting mechanism 1 that can simultaneously acquire images of a plurality of different wavelength bands by splitting a wavelength of the light source into a plurality of wavelengths, and disposing a plurality of cell image acquiring one dimensional photosensor arrays on the image acquiring surface in addition to the cell image acquiring one dimensional photosensor array and disposing a band-pass filter that allow only light with a specific wavelength to pass through on each one dimensional photosensor array.

In the cell analysis device system of the present disclosure,
the third device can have a wavelength spectrum separation mechanism for separating a wavelength of the light source into a plurality of wavelengths and separating a linear light of a band-like region that is orthogonal to an obtained flow as a wavelength spectrum, and an image splitting mechanism **2** that can simultaneously acquire images of a plurality of different wavelength bands by disposing the wavelength spectrum and each cell image acquiring one dimensional photosensor array at a position of respective wavelength spectrum on the image acquiring surface.

The present disclosure also provides a cell analysis method for measuring a distribution of sizes, circumferential lengths, and/or particle amount ratios of an internal microstructure of a shape of a cell or microparticle in a solution at a full amount to determine the presence/absence of an abnormality from a change in the distribution.

The present disclosure also provides the following.

### (Item A1)

A method of analyzing a cell derived from a subject, the method comprising the steps of:
a) acquiring an image of the cell;
b) generating flow rate data for the cell from the acquired image;
c) generating accurate cell shape data based on the flow rate data;
d) continuously analyzing information on a cell based on the cell shape data;
e) outputting a distribution of cell information on the entire test sample from information on a cell based on the cell shape data; and
f) distinguishing an abnormality in a cell of the subject from the distribution of the cell information.

### (Item A2)

A computer program for causing a computer to execute processing of a method of analyzing a cell derived from a subject, the method comprising the steps of:
a) causing the computer to acquire an image of the cell;
b) causing the computer to generate flow rate data for the cell from the acquired image;
c) causing the computer to generate accurate cell shape data based on the flow rate data;
d) causing the computer to continuously analyze information on a cell based on the cell shape data;
e) causing the computer to output a distribution of cell information on the entire test sample from information on a cell based on the cell shape data; and
f) causing the computer to distinguish an abnormality in a cell of the subject from the distribution of the cell information.

### (Item A3)

A recording medium for storing a computer program for causing a computer to execute processing of a method of analyzing a cell derived from a subject, the method comprising the steps of:
a) causing the computer to acquire an image of the cell;
b) causing the computer to generate flow rate data for the cell from the acquired image;
c) causing the computer to generate accurate cell shape data based on the flow rate data;
d) causing the computer to continuously analyze information on a cell based on the cell shape data;
e) causing the computer to output a distribution of cell information on the entire test sample from information on a cell based on the cell shape data; and
f) causing the computer to distinguish an abnormality in a cell of the subject from the distribution of the cell information.

### (Item A4)

A system for analyzing a cell derived from a subject, comprising:
a) means for acquiring an image of the cell;
b) means for generating flow rate data for the cell from the acquired image;
c) means for generating accurate cell shape data based on the flow rate data;
d) means for continuously analyzing information on a cell based on the cell shape data;
e) means for outputting a distribution of cell information on the entire test sample from information on a cell based on the cell shape data; and
f) means for distinguishing an abnormality in a cell of the subject from the distribution of the cell information.

### (Item B)

Use of at least one indicator selected from the group consisting of a size of a cell, a shape of a cell, presence/absence of formation of a population, i.e., whether cells form an aggregate (cluster), a population size (number and type of constituent cells), a size of a nucleus within cells, and presence/absence of a multinucleated cell, for cell analysis.

### (Item C1)

A method of determining whether a cell is a nucleated cell and/or a multinucleated cell, comprising simultaneously acquiring an image of a bright field cell shape of the cell and a fluorescence image in the cell as an image of at least one wavelength.

### (Item C2)

The method of item C1, further comprising acquiring a shape or size of a cell, presence/absence of cells in a clumped state, a rough number of cells in a cell population when forming a cell mass, or the like.

### (Item C3)

The method of item C1 or C2, further comprising finding the number of nuclei inside a cell and an area of each nucleus by using a fluorescent dye that specifically stains a nucleus.

### (Item C4)

The method of items C1 to C3, further comprising identifying the presence of a cancer cell by identifying a multinucleated cell from information on fluorescence of a nucleus of a cell in an acquired cell cluster.

### (Item C5)

The method of item C4, which is a diagnostic method of cancer.

### (Item D)

A method of distinguishing a cell mass, comprising combining:
acquiring background image data from when cells are not flowing;
acquiring bright field image data from when cells are flowing;
extracting an image of only a cell mass by subtracting the background image data from the bright field image data; and
acquiring a length of a boundary line of the extracted image (circumferential line of a cell or a cell mass) and an area of a region surrounded by the boundary line;
to extract data for a cell mass.

### (Item E1)

A method of identifying a cancer cell in blood, comprising at least one step selected from the group consisting of:
(1) identifying a cell cluster (mass), which is not present in healthy blood, as the presence of a cancer cell in blood;
(2) identifying a multinucleated cell, which is not present in healthy blood, as the presence of a cancer cell in blood;
(3) identifying a giant cell, which is not present in healthy blood, as the presence of a cancer cell in blood; and
(4) identifying a size distribution that is characteristic to a metastatic cancer patient, which is different from a characteristic of a healthy individual, from a size distribution diagram of white blood cells in blood (all cells remaining after removing red blood cell components from blood) as the presence of a cancer cell; and optionally
(5) identifying a cancer cell by analysis combining the presence of a fluorescence intensity of a fluorescent antibody to one or more biomarkers (e.g., EpCam antibody, K-ras antibody, cytokeratine antibody, or the like) of a cancer cell measured from fluorescence intensity.

### (Item E2)

The method of item E2, wherein the method is characterized by using one of:
(1) an area of a nucleus of about 150 µm² or greater in a cell (cluster) is measured from an acquired image;
(2) an area of about 250 µm² or greater in a cell (cluster) is measured from an acquired image; and
(3) the presence of three of more nuclei in a cell (cluster) is measured from an acquired image;
or a combination of the three conditions described above, i.e., (1) and (2), (1) and (3), (2) and (3), or (1) and (2) and (3), as criteria for determining the presence of a cancer cell in blood.

### (Item E3)

The method of Item E1 or E2, further comprising at least one of ultimately identifying whether a cell is a cancer cell or what characteristic a cancer cell has if the cell is a cancer cell by measuring a genetic mutation in combination with gene analysis means such as a PCR analysis technology for small cells, or re-culturing a cell to evaluate a cellular function.

### (Item F1)

A method of analyzing a cell derived from a subject, comprising the steps of:
(A) processing a cell contained in a cell sample solution derived from a subject;
(B) preparing capsule particles by encapsulating the processed cell in a capsule;
(C) acquiring an image of the processed cell or the cell encapsulated in a capsule particle; and
(D) performing the method of item A1 on the image for determination.

### (Item F2)

The method of item F1, wherein the step of processing comprises purifying, concentrating, staining, and/or washing a cell of a candidate size region.

### (Item F3)

The method of item F1 or F2, wherein the step of processing selectively and continuously fractionates cells in the cell sample solution by size.

### (Item F4)

The method of any one of items F1 to F3, wherein the step of preparing capsule particles constructs capsule particles of alginic acid comprising a cell by mixing a solution comprising alginic acid in a sol state and the cell in a solution comprising a divalent ion.

### (Item F5)

The method of any one of items F1 to F4, wherein the step of preparing capsule particles aligns particle sizes of the capsule particles of alginic acid, and distinguishes a capsule particle comprising a cell and a capsule particle that does not comprise a cell to selectively collect a capsule particle comprising a cell.

### (Item F6)

The method of item F5, wherein the collection collects a target cell or capsule particle comprising a cell by applying an ionic current to a cell or capsule particle comprising a cell.

### (Item F7)

The method of any one of items F1 to F6, wherein the step of distinguishing optically determines a flow rate of a cell, and acquires and analyzes a characteristic from an image of a cell corrected based on the optically obtained flow rate of a cell.

### (Item F8)

The method of any one of items F1 to F7 for measuring a distribution of sizes, circumferential lengths, and/or particle amount ratios of an internal microstructure of a shape of a cell in the cell sample solution at a full amount to determine the presence/absence of an abnormality from a change in the distribution.

### (Item F9)

The method of any one of items F1 to F8 for separating/identifying an abnormal cell in a cell sample derived from a subject.

### (Item G1)

A method of determining the presence/absence of an abnormal cell in a cell sample derived from a subject, comprising the steps of:
(A) processing a cell contained in a cell sample solution derived from a subject;
(B) preparing capsule particles by encapsulating the processed cell in a capsule; and
(C) determining the presence/absence of an abnormal cell in the cell sample derived from the subject, wherein the determination comprises the steps of:
   a) acquiring an image of the processed cell or the cell encapsulated in the capsule particle;
   b) generating flow rate data for the cell from the acquired image;
   c) generating accurate cell shape data based on the flow rate data;
   d) continuously analyzing information on a cell based on the cell shape data;
   e) outputting a distribution of cell information on the entire test sample from information on a cell based on the cell shape data; and
   f) distinguishing an abnormality in a cell of the subject from the distribution of the cell information.

### (Item G2)

The method of item G1, wherein the step of processing comprises purifying, concentrating, staining, and/or washing a cell of a candidate size region.

### (Item G3)

The method of item G1 or G2, wherein the step of processing selectively and continuously fractionates cells in the cell sample solution by size.

### (Item G4)

The method of any one of items G1 to G3, wherein the step of preparing capsule particles constructs capsule particles of alginic acid comprising a cell by mixing a solution comprising alginic acid in a sol state and the cell in a solution comprising a divalent ion.

### (Item G5)

The method of any one of items G1 to G4, wherein the step of preparing capsule particles aligns particle sizes of the capsule particles of alginic acid, and distinguishes a capsule particle comprising a cell and a capsule particle that does not comprise a cell to selectively collect a capsule particle comprising a cell.

### (Item G6)

The method of item G5, wherein the collection collects a target cell or capsule particle comprising a cell by applying an ionic current to a cell or capsule particle comprising a cell.

### (Item G7)

The method of any one of items G1 to G6, wherein the step of distinguishing optically determines a flow rate of a cell, and acquires and analyzes a characteristic from an image of a cell corrected based on the optically obtained flow rate of a cell.

### (Item G8)

A computer program for causing a computer to execute processing of a method of determining the presence/absence of an abnormal cell in a cell sample derived from a subject, the method comprising the steps of:
(A) causing the computer to process a cell contained in a cell sample solution derived from a subject;
(B) causing the computer to preparing a capsule particle by encapsulating the processed cell in a capsule; and
(C) causing the computer to determine the presence/absence of an abnormal cell in a cell sample derived from the subject, wherein the determination comprises the steps of:
   a) causing the computer to acquire an image of the processed cell or the cell encapsulated in a capsule particle;
   b) causing the computer to generate flow rate data for the cell from the acquired image;
   c) causing the computer to generate accurate cell shape data based on the flow rate data;
   d) causing the computer to continuously analyze information on a cell based on the cell shape data;
   e) causing the computer to output a distribution of cell information on the entire test sample from information on a cell based on the cell shape data; and
   f) causing the computer to distinguish an abnormality in a cell of the subject from the distribution of the cell information.

### (Item G9)

A recording medium for storing a computer program for causing a computer to execute processing of a method of determining the presence/absence of an abnormal cell in a cell sample derived from a subject, the method comprising the steps of:
(A) causing the computer to process a cell contained in a cell sample solution derived from a subject;
(B) causing the computer to prepare a capsule particle by encapsulating the processed cell in a capsule; and
(C) causing the computer to determine the presence/absence of an abnormal cell in a cell sample derived from the subject, wherein the determination comprises the steps of:
   a) causing the computer to acquire an image of the processed cell or the cell encapsulated into a capsule particle;
   b) causing the computer to generating flow rate data for the cell from the acquired image;
   c) causing the computer to generate accurate cell shape data based on the flow rate data;
   d) causing the computer to continuously analyze information on a cell based on the cell shape data;
   e) causing the computer to output a distribution of cell information on the entire test sample from information on a cell based on the cell shape data; and
   f) causing the computer to distinguish an abnormality in a cell of the subject from the distribution of the cell information.

### (Item G10)

A system for determining the presence/absence of an abnormal cell in a cell sample derived from a subject, comprising:
(A) means for processing a cell contained in a cell sample solution derived from a subject;
(B) means for preparing a capsule particle by encapsulating the processed cell in a capsule; and
(C) means for determining the presence/absence of an abnormal cell in the cell sample derived from the subject, wherein the determination comprises:
   a) means for acquiring an image of the processed cell or the cell encapsulated into the capsule particle;
   b) means for generating flow rate data for the cell from the acquired image;
   c) means for generating accurate cell shape data based on the flow rate data;
   d) means for continuously analyzing information on a cell based on the cell shape data;
   e) means for outputting a distribution of cell information on the entire test sample from information on a cell based on the cell shape data; and
   f) means for distinguishing an abnormality in a cell of the subject from the distribution of the cell information.

### [Advantageous Effects of Invention]

An abnormal cell in a cell sample derived from a subject can be separated/identified by the present disclosure.

### [Brief Description of Drawings]

[Figure 1] Figure **1** provides schematic diagrams and pictures that schematically show an exemplary summary of a cell sorting technology based on an image performed by a cell analysis device system with regard to cell analysis performed using the cell analysis device system of the present disclosure.
[Figure 2] Figure **2** is a schematic diagram that schematically show an example of the overall process of cell analysis performed using the cell analysis device system of the present disclosure and means within a device corresponding to individual steps.
[Figure 3] Figure **3** is a diagram that schematically shows an example of the overall configuration of the cell analysis device system of the present disclosure in Figure **2****.**
[Figure 4] Figure **4** is a diagram that schematically shows an example of the configuration of the cell concentration/size fractionation/staining/washing module in Figure **3****.**
[Figure 5] Figure **5** is a diagram that schematically shows another example (pillar array form) of the configuration of the cell concentration/size fractionation/staining/washing module in Figure **3****.**
[Figure 6] Figure **6** is a diagram that schematically shows the principle behind the operation of another example (pillar array form) of the configuration of the cell concentration/size fractionation/staining/washing module in Figure **3****.**
[Figure 7] Figure **7** is a diagram that schematically shows an example of a microprocessing technology used in the cell concentration/size fractionation/staining/washing module in Figure **3****.**
[Figure 8] Figure **8** is a diagram that schematically shows the configuration of the cell/cell mass encapsulation module in Figure **3****.**
[Figure 9] Figure **9** is a diagram that schematically shows an example of the configuration of the image detecting single cell separation/purification (cell sorting) module in Figure **3****.**
[Figure 10] Figure **10** is a diagram that schematically shows another example of the configuration of the image detecting single cell separation/purification (cell sorting) module in Figure **3****.**
[Figure 11] Figure **11** is a diagram that schematically shows an example of constituent elements of the image detecting single cell separation/purification (cell sorting) module in Figure **9****.**
[Figure 12] Figure **12** is a diagram that schematically shows another example of a combination of constituent elements of the image detecting single cell separation/purification (cell sorting) module in Figure **10****.**
[Figure 13] Figure **13** is a diagram that schematically shows an example of the configuration of an analysis system that simultaneously performs high speed bright field microscope image acquisition and high speed fluorescence microscope image acquisition.
[Figure 14] Figure **14** is a diagram that schematically shows an example of the configuration of an analysis system that simultaneously performs fluorescence intensity measurement, high speed bright field microscope image acquisition, and high speed fluorescence microscope image acquisition.
[Figure 15] Figure **15** is a diagram that schematically shows the outer appearance of another example of the configuration of an optical module portion in an analysis system that simultaneously performs fluorescence intensity measurement, high speed bright field microscope image acquisition, and high speed fluorescence microscope image acquisition.
[Figure 16] Figure **16** is a diagram that schematically shows an example of the chip configuration of the image detecting single cell separation/purification (cell sorting) module in the present disclosure.
[Figure 17] Figure **17** is a diagram that schematically shows an example of the relationship between an electronic shutter and the timing of light emission from a high speed flash light source of the image detecting single cell separation/purification (cell sorting) module.
[Figure 18] Figure **18** is a diagram that schematically shows an example of the configuration of an optical system for preventing image blur in the image detecting single cell separation/purification (cell sorting) module.
[Figure 19] Figure **19** is a diagram that schematically shows an example of the configuration of a high speed continuous image acquisition system using a line sensor set in the image detecting single cell separation/purification (cell sorting) module.
[Figure 20] Figure **20** is a diagram that schematically shows an example of the configuration of a line sensor array set for simultaneously acquiring a plurality of images of image formation surface and the configuration of a line sensor array set for simultaneously acquiring polychromatic fluorescence in a high speed continuous image acquisition system using a line sensor set in the image detecting single cell separation/purification (cell sorting) module.
[Figure 21] Figure **21** is a diagram describing the process of image processing after simultaneously acquiring a high speed bright field microscope image and a high speed fluorescence microscope image and pictures showing exemplary images from simultaneously acquiring a high speed bright field microscope image and high speed fluorescence microscope image of a fluorescently stained nucleus.

### [Description of Embodiments]

The embodiments of the present disclosure include a cell analysis device system and a cell analysis method using the cell analysis device system. The preferred embodiments thereof are described hereinafter with a description of the Examples and descriptions of the drawing.

The entirety of all of the documents mentioned herein is incorporated herein by reference. The Examples described herein are provided for exemplification of the embodiments of the present disclosure and should not be interpreted as limitation of the scope of the present disclosure.

### <1. Summary of the concept of cell sorting technology performed by a cell analysis device system>

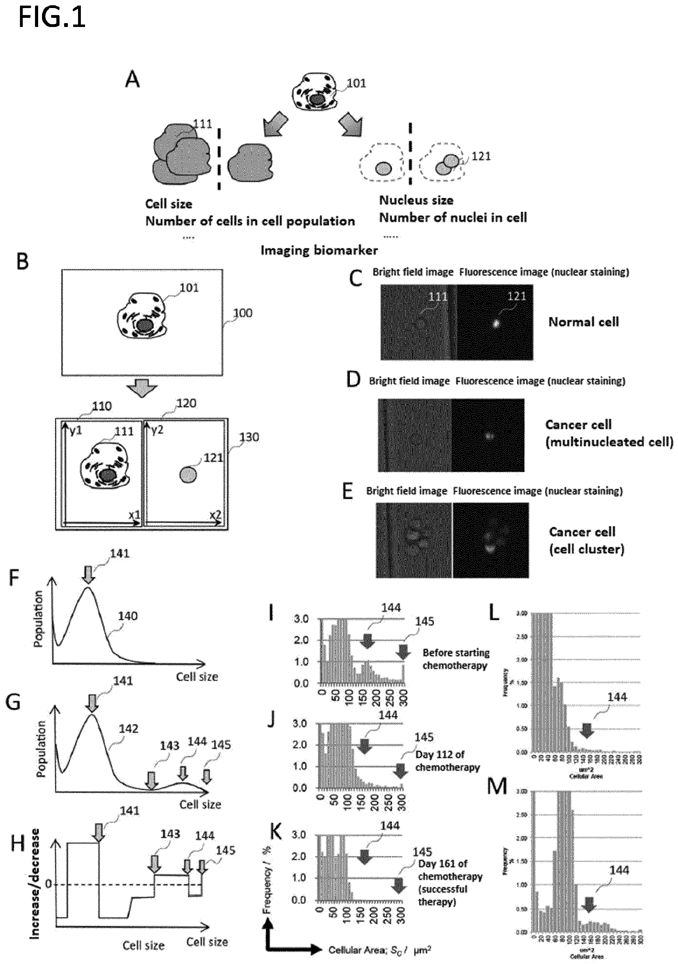

Figure **1** is a diagram that schematically shows an exemplary summary of the concept of a cell sorting technology based on an image performed by a cell analysis device system with regard to cell analysis performed using the cell analysis device system of the present disclosure.

Figure **1A** describes that a cell image **101** actually acquired by a system is directly used as an indicator (e.g., imaging biomarker) for detailed analysis of a cell from the acquired image itself. In this regard, the indicator includes not only information that can be distinguished by bright field microscope observation such as characteristics of the size and shape of cells, but also information such as the presence or absence of formation of a population, i.e., whether cells form an aggregate (cluster), and population size (number and type of constituent cells). The indicator also includes information such as information that can be distinguished by fluorescence microscope observation through nuclear staining or the like, i.e., the size of a nucleus within a cell and whether a cell is a multinucleated cell. In particular, information on the cell shape and clustering is difficult to obtain by conventional light scatter measurement, light absorption, or technologies for analyzing a change in electrical impedance, such that a technology for acquiring and analyzing an image was required.

Figure **1B** is a diagram describing cell analysis performed by using the cell analysis device system of the present disclosure in order to materialize the approach discussed in the description of Figure **1A** above. An input image **100** obtained through an objective lens is data for a cell **101** in which information on light of a plurality of wavelengths is overlaid. Meanwhile, by going through means for separating a wavelength, a bright field output image **110** and an output image **120** consisting of a fluorescence image of a fluorescently labeled nucleus, for example, can be simultaneously acquired. For this reason, the cell image **101** is simultaneously resolved into each optical wavelength at the same magnification ratio. For example, a bright field image **111** of the cell shape and a fluorescence image **121** of a fluorescently stained nuclear component in the cell are simultaneously acquired as images of respective wavelengths, which allows comparison of these images as coordinates on two parallel two dimensional relative coordinate axes x1-y1 and x2-y2 and estimation of which position of the cell **111** corresponds to the position of a nuclear component **121** with fluorescence. Use of such function can reveal, for example, whether the cell image **111** obtained in a bright field image is a nucleated cell or multinucleated cell. Further, the shape or size of a cell, presence/absence of cells in a clumped state, the rough number of cells in a cell population when forming a cell mass, or the like can be acquired in the bright field image **111.** For example, the number of nuclei inside a cell, area of each nucleus, or the like can be acquired by using a fluorescent dye that specifically stains a nucleus in the fluorescence image **121.**

Figure **1C** to Figure **1E** are microscope pictures that show an example of two images simultaneously acquired as a high speed bright field microscope image and a high speed fluorescence microscope image from fluorescently staining a nucleus by splitting a single ultrahigh speed camera light receiving surface. As described above with regard to Figure **1B****,** the present disclosure can compare where in a bright field cell image or cell cluster image a nucleus is distributed with regard to the position of a nucleus that can be identified by a fluorescence image using relative coordinates of each other by matching the relative coordinates of two images in advance. By comparing the relative coordinates, a bright field image and a fluorescence image can be simultaneously acquired as shown in, for example, the microscope pictures of Figure **1C****.** It can be understood that a single nucleus is gleaming with fluorescence indicating a normal cross sectional area size in a cell with a smooth surface and normal size in normal cells of a healthy individual. Meanwhile for cancer cells, multinucleation is an indicator of cancer cells. It can be understood that two divided nuclei are gleaming in a single isolated cell with the same shape and size as a normal cell in the left picture of bright field image by comparing relative coordinates as shown in the pictures of Figure **1D****.** Although not present in normal blood, fluorescence of a plurality of nuclei within a cell cluster is observed through the fluorescence of the cell clusters and the fluorescence of the nucleus of each cell in the cluster in blood from a cancer metastasis case as shown in the pictures of Figure **1E****.** Thus, it can be understood that this is a cluster in view of such fluorescence of a plurality of nuclei. It can be readily determined that such cells are in a form of a cluster from analysis via image acquisition as can be seen from the picture. Meanwhile, even if information showing that the size is different is obtained with conventional technologies such as light scatter or impedance measuring approaches, it is difficult to distinguish whether this is a difference in cell size, or a plurality of cells are aggregated into a cell mass. Furthermore, it is a characteristic of an image based analysis method that the positional relationship of nucleic acid and cell based on spatial information can be compared by simultaneously acquiring a bright field image and fluorescence image by separating wavelengths from the same image. To find a cell volume (here it is the two dimensionally projected area) that is accurate in both flow directions y1 and y2 for an acquired image, the length in the direction of flow is converted for an image acquired as an image so that the lengths in the direction of flow (y1, y2) and the direction that is vertical to the flow (x1, x2) would be the same to acquire an accurate two dimensional image.

The present disclosure can also distinguish a cell mass using only a bright field image by combining a procedure for extracting only an image of a cell or cell mass by subtracting background image data acquired in advance when cell is not flowing from a bright field image of a cell cluster of for example Figure **1E** by image processing, and means for acquiring the length of a boundary line of the extracted image (circumferential line of a cell or cell mass) and an area of a region surrounded by the boundary line. This effectively utilizes a characteristic of a cell sorter, i.e., the same region is observed at a fixed point, at which cells flow into sequential images. Since all pixels with the same image as the background image would be zero from subtraction, only data for each pixel of an image of a cell that is different from the background image would be automatically extracted. Thus, a conventional approach of relatively finding a boundary line between a cell in a solution and a solution by a complex contrast comparison is not needed. The actual measurement of circumferential length L of a boundary line of a cell or cell mass readily acquired from two dimensional coordinates of a boundary line where pixel data would not be zero and actual measurement of area S for the entire region of cell surrounded by the boundary line can be found by counting the number of pixels of the boundary line for the former, and by counting the total number of pixels of a region within the boundary line for the latter. The procedure for distinguishing a cell mass is, for example, the following. First, the hypothetical radius R, when assuming area S as a projected image if a cell is a true sphere with a radium R, is found from actual measurement of area S. R can be found from cross sectional area S actually measured from an image by R = (S/π)^{1/2}, and then the hypothetical circumferential length L₀, in terms of such a circle, is found by L₀ = 2πR = 2(πS)^{1/2}. If the ratio of actually measured circumferential length L to the hypothetical circumferential length L₀ is D, a cell would be a true sphere if D = L/L₀ is 1. Since the circumferential length L would be longer as the shape of a cell or cell mass deviates away from a true sphere, D would be greater. This, along with whether the size is greater than a single cell from the actually measured area S of a cell or cell mass, are checked, and if both results are satisfied, it can be determined as a cell cluster. Specifically, a cell can be determined as a cell cluster by using only a bright field image without using a fluorescence image by using an indicator of, for example, "(D > 1.1) and (S > 800 µm²)"

Likewise, the present disclosure can combine a procedure for extracting only an image of a cell or cell mass by subtracting background image data acquired in advance when a cell is not flowing from a fluorescence image of a cell cluster of for example Figure **1C, 1D,** or **1E** by image processing, and means for acquiring the length of a boundary line of the extracted image (circumferential line of a cell or cell mass) and an area of a region surrounded by the boundary line to readily measure the number or size of nuclei if nuclei are fluorescently stained. This effectively utilizes a characteristic of a cell sorter, i.e., the same region is observed at a fixed point, at which cells flow into sequential images, as discussed above in the same manner as a bright field image. Since all pixels with the same image as the background image would be zero from subtraction in the same manner, only pixel portions that are different from the background image and would not be zero is automatically extracted as data for each pixel of an image of a cell. The actual measurement of circumferential length L of a boundary line of a cell or cell mass readily acquired from two dimensional coordinates of a boundary line where pixel data would not be zero and actual measurement of area S for the entire region of cells surrounded by the boundary line can be found by counting the number of pixels of the boundary line for the former, and by counting the total number of pixels of a region within the boundary line for the latter. Since a cell or cell mass is configured to flow in a single straight line with an interval at the center of a flow so that a single cell or a single cell mass would be in a single acquired image, a single cell or a single cell mass was in a single screen for a bright field image, but a plurality of nuclei would be observed in a fluorescence image. For this reason, a plurality of independent regions (nuclei) and their respective boundary lines (circumferential lines) would be acquired in fluorescence observation. Whether each nucleus is a normal nucleus is determined from a comparative analysis of the circumferential length and cross-sectional area of each region (nucleus) by the same procedure for a bright field image. The procedure for distinguishing nucleus sizes is, for example, the following. First, the hypothetical radius R, when assuming area S as a projected image if a cell is a true sphere with a radium R, is found from actual measurement of area S. R can be found from cross sectional area S actually measured from an image by R=(S/π)^{1/2}, and then the hypothetical circumferential length L₀, in terms of a such a circle, is found by L₀ = 2πR = 2(πS)^{1/2}.

If the ratio of actually measured circumferential length L to the hypothetical circumferential length L₀ is D, a nucleus would be a true sphere if D = L/L₀ is 1. Since the circumferential length L would be longer if the shape deviates away from a true sphere, D would be greater. Thus, if D > 1.2, it can be determined that the condition of a nucleus is abnormal. For the number of nuclei, the area of each fluorescence is calculated and the center of area considered as each nucleus is acquired. If the positions of the centers of each nucleus are 3 µm or more apart, they can be determined as individual nuclei.

Figures **1F** to **1H** schematically show an example of an area size distribution diagram when cells in white blood cell components after removing red blood cell components from blood measured by the device of the present disclosure (entire cells in blood remaining after removing red blood cells) are observed with a bright field microscope. Figure **1F** schematically shows a size distribution of blood of a healthy individual. Each cell is flowing in a normal isolated state, and the distribution **140** thereof is a distribution with a single cell count peak **141** in a size region from 50 µm² to 100 µm². This corresponds to a diameter of about 10 µm as a cell size. Figure **1G** schematically shows the size distribution of white blood cell components of blood in a patient who has developed metastatic cancer. The distribution **142** has a second peak **144,** which was not seen in distribution **140** in the blood of a health individual, in a region from 150 µm² to 200 µm² after a minimum value **143** in a region from 100 µm² to 150 µm². This corresponds to a diameter of about 16 µm in terms of cell size. As a characteristic thereof, a cell size in a size region beyond 300 µm² (arrow **145)** is seen. The presence thereof can be more readily found from an increase/decrease, which is a result of differentiation of the difference in the distribution **(140** or **142)** of white blood cell components in blood as shown in Figure **1H****.** If there is an increase beyond a cell size of 150 µm² (arrow **143),** it can be inferred that the sample is blood of a metastatic cancer patient by using this graph.

Specifically, Figure **1I** to Figure **1K** show a change in cell size distribution in blood, for example, from a blood sample of a metastatic cancer model (Copenhagen rat) prior to treatment to the completion of chemotherapy. The size distribution of blood of a metastatic cancer model prior to starting chemotherapy has a distribution like that of Figure 1I. There is a clear second size peak (arrow **144)** in a region of cell size of 150 µm² to 200 µm², and many cells exceeding a cell size of 300 µm² (arrow **145).** In view of Figure **1J** showing a cell size distribution of a blood sample on day 112 after treatment with a chemotherapeutic drug (docetaxel hydrate), the clear second size peak in the region of cell size of 150 µm² to 200 µm² is eliminated, which was present in Figure **1I** prior to starting treatment, and a decrease in the number of cells with a cell size exceeding 300 µm² is also observed. In addition, metastatic cancer disappears on day 161 of starting chemotherapy from the model. As can be seen from the cell size distribution diagram of Figure **1K****,** the clear second size peak has completely disappeared in a region of cell size of 150 µm² to 200 µm², and cells with a cell size exceeding 300 µm² have also disappeared. Figure **1L** is a distribution of sizes of cells in blood of a healthy model. The size distribution of blood of a healthy model does not have a clear second size peak in a region of cell size of 150 µm² to 200 µm², or cells with a cell size exceeding 300 µm², much like the successfully treated metastatic cancer model in Figure **1K****.**

Figure **1M** is a cell size distribution of blood of an animal model (Copenhagen rat) suffering from an infection. When infected, a small second size peak appears in the region of cell size of 150 µm² to 200 µm², but a significant increase in cells with a cell size exceeding 300 µm² is not found. This indicates that metastatic cancer and infection can be distinctly determined in hematological diagnosis from the difference in the presence/absence of a clear increase in the region of cell size of 150 µm² to 200 µm² and a significant increase in cells with a cell size exceeding 300 µm².

In this manner, the presence of metastatic cancer in blood can be confirmed, and cancer cells in blood can be selectively collected from identification with a new biomarker, i.e., "image of the shape or population formation of cells, internal structure such as multinucleation, or the like", instead of conventional molecular biomarker by (1) an approach of identifying a cell cluster (mass) that is not present in healthy blood as a candidate of a cancer cell in blood, (2) an approach of identifying, and selectively collecting, a multinucleated cell that is not present in healthy blood as a candidate of a cancer cell in blood, (3) a method of identifying, and selectively collecting, a giant cell that is not present in healthy blood as a candidate of a cancer cell in blood, (4) an approach of determining that a size distribution is characteristic to a metastatic cancer patient that is different from the characteristic of a healthy individual from a size distribution diagram for white blood cells in blood (all cells remaining after removing red blood cell component from blood) and a method of selectively collecting cells in a characteristic size distribution region, or (5) an approach of identifying, and selectively collecting, cancer cells by analysis combining detection of fluorescence intensity representing the presence of a fluorescently labeled antibody, which is prepared from fluorescently labeling an antibody to one or more biomarkers (e.g., EpCam antibody, K-ras antibody, cytokeratine antibody, or the like) for a cancer cell measured from fluorescence intensity in addition to (1), (2), (3), or (4), by using means for image analysis and determination in the device of the present disclosure. Further, it is possible to subsequently identify whether a candidate of a cancer cell in blood collected by the approach described above is ultimately a cancer cell, or if the candidate is a cancer cell, what characteristic a cancer cell has by combining gene analysis means such as PCR analysis technology for small cells to measure a genetic mutation or re-culturing the cell to evaluate cellular function as described above.

With regard to (1), cells can be distinguished by evaluating a cell image from a bright field image by D > 1.1 and S > 800 µm², or evaluating the size from a bright field image and number and distribution of nuclei from a fluorescence image (i.e., the distance of the centers in images of a plurality of adjacent nuclei is 3 µm or more) and D > 1.3 for nuclei. With regard to (2), cells can be distinguished through evaluation by D < 1.1 and S < 800 µm² from a bright field image, and the number and distribution of nuclei (i.e., the distance of the centers in images of a plurality of adjacent nuclei is 3 µm or more) and D < 1.3 for nuclei. With regard to (3), cells can be distinguished from a bright field image by D < 1.1 and cell size of S > 800 µm². With regard to (4), this can be distinguished by the presence of a clear second size peak (arrow **144)** in a region of cell size of 150 µm² to 200 µm² and a large number of cells exceeding a cell size of 300 µm², as described for Figures **1F** to **1M****.** For this condition, unlike the conditions from (1) to (3), it is necessary to acquire the entire data for white blood cells in blood for the number of cells required to acquire a histogram of cell size distribution. This requires at least 5000 white blood cells. In particular, a cell with one or more matching conditions from the combination of (1) to (4) can be determined as a cancer cell.

### <2. Summary of a typical embodiment (summary of a device)>

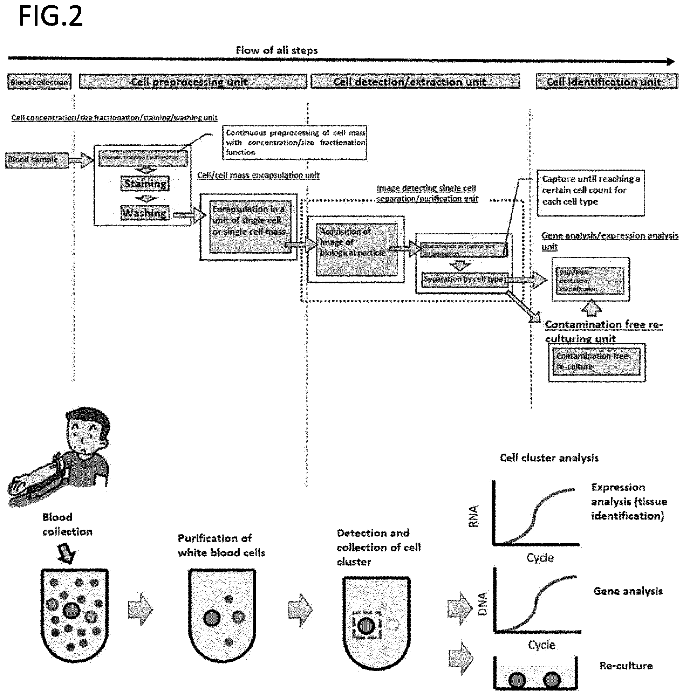

The cell analysis device system of the present disclosure generally consists of the following three elements as schematically shown in Figure **2** in order to distinguish cells based on image data described in Figure **1****.** Specifically, the system comprises:
(1) a cell preprocessing unit consisting of a cell concentration/staining/washing unit for sequentially performing processes including concentration of cells, fractionating cells by size, staining with a fluorescent antibody label (or optionally a reversible fluorescent label marker such as an aptamer for re-culture), and washing and a cell/cell mass encapsulation unit for encapsulating in a single cell or single cell mass unit;
(2) an image detecting single cell separation/purification unit (cell sorting unit) for acquiring image data for a cell image at a high speed from cells flowing through a microchannel formed on a chip substrate, performing quantitative distribution analysis on the type of cell based on a result of analyzing the image information, and separating/purifying cells in real-time at a high speed; and
(3) a control/analysis unit for controlling the operation of each of the units described above and performing the analysis described above.

To analyze fractionated cells in a unit of a single cell, the following can be added to a subsequent stage:
(4) a gene analysis/expression analysis unit for measuring the intracellular state at a single cell or single cell mass level;
(5) a contamination-free re-culturing unit for re-culturing a purified cell or cell mass each individually while preventing contamination; and the like.

A typical embodiment of the cell analysis device system of the present disclosure is characterized by consecutively combining (1) to (2) of the three modules described above in the order described above, and cells are processed continuously with a microchannel and a microcontainer. Thus, loss of a small amount of cells due to contamination or operation can be minimized.

By using the cell analysis device system of the present disclosure, cells can be detected and checked to confirm and determine whether the cells are isolated single cells that are not clustered or a clustered cell population from the size obtained from a characteristic of the shape and a two-dimensional image (volume or two dimensional image of volume) and the circumferential length by using a bright field microscope image for distinguishing the cell type. Thus, the cell analysis device system of the present disclosure can identify and count, and selectively separate/purify cells and aggregated cell populations based on an indicator, which could not be identified by conventional scattered light detecting cell sorter technologies.

By using the cell analysis device system of the present disclosure, the presence/absence of a fluorescent label of a cell by nuclear staining for distinguishing the cell type can be detected and confirmed at a single cell level, and a fluorescently labeled cell can be confirmed to be an isolated single cell that is not clustered, and the labeled cell can be checked and determined as to whether the cell is a multinucleated cell. Thus, the cell analysis device system of the present disclosure can distinguish and count, and separate/purify cells based on an indicator, such as the shape and size of a nucleus and the special arrangement of each nucleus based on imaging, which could not be identified by conventional scattered light detecting cell sorter technologies.

By using the cell analysis device system of the present disclosure, the presence/absence of a fluorescent label of a cell using a fluorescently labeled antibody for distinguishing the cell type can be detected and confirmed at a single cell level, and a fluorescently labeled cell can be confirmed to be an isolated single cell that is not clustered, and it is possible to determine whether apoptosis is occurring in a cell. Thus, the cell analysis device system of the present disclosure can separate/purify cells based on an indicator, which could not be identified by conventional scattered light detecting cell sorter technologies.

The cell analysis device system of the present disclosure can accurately determine and count, and selectively collect a specific shape, cell mass, or cell with a specific region stained in a specific shape in a unit of a single cell, and check the state of cells, such as apoptosis of cells, being collected, and combine a genetic analysis/expression analysis device with fluorescence information of each cell and information on the cell state are combined to analyze genetic information or expression information of cells.

The cell analysis device system of the present disclosure can also accurately determine and count cells with a characteristic of a specific shape or cells with a specific region stained in a specific shape in a unit of a single cell or a single mass, and acquire and analyze a distribution of the ratio of the number of cells matching each indicator to the entire number.

The outline of the configuration for materializing the function described above is described using Figure **2****.** However, the configuration shown in Figure **2** schematically shows an example of the outline of the configuration of a cell sorting technology based on an image performed by a cell analysis device system constructed by combining the three modules (1) to (3) described above. A case where the genetic analysis/expression analysis unit of (4) or the contamination-free re-culturing unit of (5) is added thereto is also shown. For example, the configuration can also be used such that only the configuration for size fractionation of (1) is optimized for use in collecting cells with a cell size exceeding 300 µm², and the selectively collected cells are used in genetic mutation analysis of (4), or used to acquire image data for a cell image of (2) at a high speed without the preprocessing of cells of (1) and perform quantitative distribution analysis on the cell type based on the result of analysis of the image information to check the presence of an abnormal cell and abnormal cell cluster in blood.

A detailed example of a cell analysis device system constructed by sequentially combining the modules of (1) to (5) in this order is described hereinafter.

First at (1), a blood sample collected from a patient is introduced into a cell concentration/size fractionation/staining/washing unit. In this regard, a procedure for concentrating and extracting only cell components from blood is performed. In particular in this case, red blood cell components and other components are continuously separated to selectively collect white blood cell components (all other cells excluding red blood cell components in blood). Alternatively, especially if it is desirable to selectively collect white blood masses, only cell clusters can be selectively collected in the preprocessing stage by setting the threshold value of cell size to a cell size greater than 300 µm² in terms of volume. After a fluorescent label agent such as a fluorescent cancer marker is added and reacted with sample cells, unreacted excessive fluorescent label agent is washed and removed.

The cells are then introduced into a cell/cell mass encapsulation unit for encapsulation in an alginic acid capsule in a unit of a single cell or single cluster. In this configuration, encapsulation of cells in an alginic acid gel capsule enables prevention of subsequent contamination of cells from the outside, and materializes additional improvement in performance that enables stable selective collection while preventing damage to cells inside a capsule by a stabilized and constant surface charge of the alginic acid capsule encapsulating the cells without being dependent on the surface charge of the cells upon selective collection using an electrophoretic force in an image detecting single cell separation and purification unit (sell sorting unit) of a cell detection and extraction unit at a later stage. However, the series of operations in an image detecting single cell separation/purification unit (cell sorting unit) in this device can be performed without encapsulation with an alginic acid capsule. Further, a function of an alginic acid capsule being attracted to a magnetic field as a superparamagnetic capsule can be added by mixing a ferromagnetic microparticle of iron, manganese, or the like pulverized to a size where a magnetic domain structure is not formed, with a particle size of 100 µm or less, to the alginic acid capsule. This can facilitate selective collection. Further, an alginic acid capsule in a solution can be simulated to be transparent by using a solution prepared to have the same refractive index as that of an alginic acid capsule consisting of alginic acid gel to facilitate optical microscope observation of cells within the capsule. Specifically, if the refractive index of an alginic acid capsule is 1.4, the same refractive index is achieved by adding 40% by weight of sucrose to the solution. In addition, buoyancy can also be adjusted by including iron nanoparticles in an alginic acid capsule.

Next, the image detecting single cell separation/purification unit (cell sorting unit) of (2) performs detection using an indicator from two optical images. First, the outer shape of cells, shape of intracellular organelle inside cells, the ratio of sizes of a nucleus and cytoplasm inside cells, and the status of cell masses are checked from a bright field microscope image at a single cell level. In addition, the presence/absence of emission of fluorescence, and the position and size thereof, based on a fluorescent label in coordinates corresponding to the position of a cell obtained in a bright field image are checked. This can confirm whether cells are target cells.

The cell detection function, when cells are captured as an image for evaluation, is structured so that a site observed by a high speed camera is provided upstream of a channel branch, and a cell separation region is optionally provided downstream therefrom. A laser or the like is irradiated onto cells passing through a channel, and scattered light from traversing cells, or fluorescence when cells are modified with fluorescence, can be detected with a photodetector without using an image. In such a case, the function is also structured so that a separation channel point, which acts as a cell separation region, is provided downstream of a detection unit.

Such an image acquisition mechanism comprises a function for simultaneously measuring a flow rate of each cell in order to reconstruct an accurate shape of a cell or cell cluster from the acquired image of cells flowing at a high speed. Accurately reconstructed cell shape information from correcting information on the cell shape to match the acquired information on the flow rate of cells is obtained. Based on this result, determination is performed using an indicator such as the cell size, circumferential length, or internal structure based on the cell shape described in, for example, Figure **1****.**

Specifically, to measure the flow rate of cells when using a high speed camera, cell images at a certain moment are simultaneously acquired with a high speed camera as individual separate images via a two wavelength image splitting optical system as images of a bright field light source from different irradiation periods at two different wavelength, and the moving speed of cells is calculated through one image acquisition from the difference in the shapes, and if, for example, the circumferential length and volume (of a two dimensional projected image) is to be obtained based on this result, the contour image and volume of the original cell can be accurately calculated by parallel translation of the boundary line of the tip on the downstream side of a flow of the acquired contour shape of cell to the upstream side of the flow by the distance of (moving speed x irradiation period of light source) . For image acquisition, a high speed camera with a temporal resolution of 1/200 second or less is used. Light emission of a light source is adjusted to match the shutter cycle of the high speed camera so that light is emitted from a light source for only a certain period among the period of cycle during which each shutter is released. If, for example, the shutter speed is 1/10,000 seconds, the precise shape of a target cell can be acquired by irradiating light onto the cell with a light source capable of high speed light emission control such as an LED light source or pulse laser light source for only 1/10 of the period. If light is emitted simultaneously from visible light sources of two wavelengths based thereon, the flow rate is calculated, for example, from the difference in the apparent full length of a cell in the direction of flow generated from the difference in irradiation periods by setting two irradiation periods of 1/100,000 seconds and 1/50,000 second.

If a high speed linear sensor is used, cross-sectional data for cells continuously detected with a linear sensor disposed perpendicularly to the direction of flow can be reconstructed and disposed in the direction of flow and acquired based on the moving speed of a cell detected with a linear sensor disposed in the direction of flow.

By analyzing, in real-time, an image acquired from reconstructing an image acquired with a high speed camera or high speed linear sensor acquired in this manner as shape information that is the same as that while still, 1) it is determined whether a cell is an isolated cell with one nucleus, a multinucleated cell, an anucleated cell, a cell undergoing cell division, or a cell constituting a cell mass with another cell, and 2) it is determined whether a cell emitting fluorescence is in a healthy state, or in a state such as apoptosis where a cell nucleus or cell shape is deformed. In accordance with the objective, healthy cells, cell masses, multinucleated cells, or cells undergoing apoptosis can be collected.

To determine and separate/purify cells, the present disclosure uses means for narrowing the channel width by adding a solution of the same speed from both sides at the upstream region from which a solution comprising cells flows to arrange the cells, in order to arrange cells or cell clusters in a straight line in the center of a channel, where the cells flow, at upstream of a region observed with a high speed camera. The present disclosure is configured to have a bifurcating structure with asymmetric channel widths downstream of the observed region, so that cells flow to a branch with a wider width including the center if an external force is not applied to the cells, and the position of cells is moved from the center to the side of a branch with a narrow width and the cells can flow in the narrow branch if an external force is applied. Subsequently, an external force is applied only to cells to be collected among cells flowing in arrangement to move the position where the cells flow so that cells are introduced to another channel of the two branched channels described above only when an external force is applied. In this regard, a pair of gel electrodes containing an electrolyte, such as sodium ion, incorporated into a region of a channel where it is desirable to apply an external force, can be utilized as a specific external force. This is advantageous in that even if a large ionic current flows by using a gel electrode, this does not result in bubbles due to electrolysis seen in a deposition electrode or the like in a channel, or a loss of a deposition electrode. As other external forces, a deposition electrode can be used if an alternating electric field is used to use a low voltage or dielectrophoretic force.

The aforementioned device configuration describes an example of a configuration intended for separation/purification of cells. If a configuration is intended for determination of cells, a configuration without means for applying an external force or a branched downstream channel is used.

Collected cells can be introduced to a high speed/small amount compatible gene analysis/expression analysis unit in the next stage capable of gene analysis or expression analysis of (4), or re-culturing while preventing contamination of (5), separately for each cell form.

If cells encapsulated in an alginic acid capsule are used as cells that have been identified and purified at this stage, the cells can be directly introduced into a gene analysis/expression analysis unit to perform high speed PCR analysis or the like while preventing contamination. When re-culturing, cells can be transferred while still being kept in an alginic acid capsule to a container for performing re-culture in a contamination free manner in a unit of a purified cell, and then a metal ion such as calcium which gelates alginic acid can be chelated with an agent such as EDTA to remove the alginic acid capsule for re-culturing.

Cells envisioned as a target of detection in the present disclosure include bacteria, as an example of small cells, and metastatic cancer cell clusters flowing in blood, as an example of large cells. Thus, the cell size is typically in the range of about 0.5 µm to about 200 µm in diameter. If a channel incorporating both a cell concentration function and cell separation function is formed on one surface of a substrate to consecutively perform cell concentration and separation, the first issue is the channel width (cross-sectional shape). A channel using a channel space of about 10 to about 200 µm in the direction of thickness of a substrate on one substrate surface is the most typical size.

Since the pressure to introduce a sample solution into a channel is the driving force for movement of the solution in the present disclosure, it is desirable to configure the pressure of a plurality of branched inlet channels and a plurality of discharge channels to be nearly equal. For this reason, the positions in the direction of height with respect to gravity at the entrance and exit are at the same height.

Algorithms for the cell recognition and separation of the present disclosure have the following characteristics. First, the flow rate of cells is found by means described above to correct and reconstruct the cell shape. Next, each pixel of a corrected and accurate cell image is binarized to find the center of luminance thereof. The center of luminance of binarized cell, area, circumferential length, major axis, and minor axis are found, and the image of each cell is numbered in the order of acquisition by using these parameters. Since it is beneficial for users to automatically save each cell image at this point as an image, the algorithm is configured to be able to auto-save.

Next, for use in cell separation, only specific cells among the numbered cells must be separated. An indicator of separation, information such as the center of luminance, area, circumferential length, major axis, and minor axis described above, or information discussed in the description of Figure **1****,** which utilizes fluorescence by concomitantly using fluorescence detection separately from an image is used. In either case, cells obtained at a detection unit are separated in accordance with the numbering. Specifically, the application timing is set to (A/v) to (A/v + t), wherein the distance from the position of a detection unit that has acquired an image of a cell to a sorting unit that applies an external force is (A) and application time is (t), in accordance with the moving speed (flow rate v) of the cells acquired above, so that the cells are sorted and separated by applying an external force as described above when a cell with a number of interest just arrives between electrodes.

With regard to means for high speed single cell genome analysis/expression analysis at the gene analysis/expression analysis unit of (4) used in the present disclosure, a reaction controlling device used comprises, for example, means for rapidly changing liquids with a plurality of different temperatures with a large thermal capacity by using a liquid with a large thermal capacity having each temperature maintained for a plurality of temperatures to be changed in changing the temperature of a sample solution, a micro reaction vessel for rapidly performing heat exchange between the liquid with a large thermal capacity and the sample solution, and a mechanism for exchanging each liquid, in order to achieve the objective described above.

When a PCR reaction is conducted while cells or cell mass are encapsulated in an alginic acid capsule at the preprocessing unit of (1) described above, the gene analysis/expression analysis unit can identify a gene or expression of cells introduced into an alginic acid capsule in a contamination free manner in a unit of a small amount of cells within a capsule as a single cell distinguished as the same cell based on information from an image detecting single cell separation/purification unit or in a unit of a population of the same cells.

In view of the lack of detection of a cell or cell mass (cluster) with an area of about 250 µm² or greater in healthy blood in the experimental result shown in for example Figure **1L****,** when a medical diagnosis is rendered, a cell or cell mass (cluster) with an area of about 250 µm² or greater is sorted with an image detecting single cell separation/purification unit (cell sorting unit), collected, and re-cultured, or a genetic mutation test or expression analysis test is performed to identify this cell cluster in order to determine whether a metastatic cancer cell is present in blood, or the cell cluster is not cancer but a cell mass produced by damage to an organ such as a liver or kidney due to a disease. Alternatively, if there is a peak of cells with an area of 150 µm² or greater and less than 250 µm², cells at 150 µm² or greater and less than 250 µm² are collected to examine whether the cells are white blood cells enlarged due to an infection, and in case of an infection, this can be utilized as a diagnostic device for elucidation including identification of the bacteria causing the infection from a gene of a foreign object incorporated into the white blood cells. In particular, for detection of metastatic cancer in blood, the degree of progression of cancer can be estimated to be in the early stage of metastasis from primary cancer if each cluster is a product of the same genetic mutation by examining a genetic mutation in a more detailed comparative analysis in a unit of each cluster. If there are many different mutation points among each cluster while the history of mutation of each cluster is the same, it can be inferred that metastatic cancer has progressed to spread into many regions.

The post-chemotherapy or hormone therapy effect on the blood of a metastatic cancer patient can also be examined. This approach, in view of the sequence of changes due to chemotherapy from Figure **1I** to Figure **1K****,** first collects a small amount of blood from a patient, acquires a cell size distribution diagram thereof, and records in particular the ratio of the presence of a peak of cells with an area of 150 µm² or greater and less than 250 µm², to the presence of a cell or cell mass (cluster) with an area of 250 µm² or greater, prior to treatment with chemotherapy. Next, after administration of each round of chemotherapy, a small amount of blood is collected from a patient, a cell size distribution diagram thereof is acquired, and in particular the ratio of the presence of a peak of cells with an area of 150 µm² or greater and less than 250 µm², to the presence of a cell or cell mass (cluster) with an area of 250 µm² or greater is recorded in the same manner to compare, after each round, the difference from the sample acquired in the previous round. If a decrease in both is found at this time, it is inferred that chemotherapy is functioning effectively. In this regard, the drug efficacy can be maximized while suppressing side effects to the minimum by reducing the dosage to the minimum amount at which anticancer function can be confirmed. Meanwhile, if a decrease is not found, administration up to the maximum tolerable dose of an agent is attempted. If there is still no effect, it is recommended to use another anticancer agent or approach.

This approach can also be used in testing for the possibility of residual cancer after a surgical operation of a metastatic cancer patient and early discovery of recurrence. Specifically, prior to a surgical operation, a small amount of blood of a patient is first collected, a cell size distribution diagram thereof is acquired, and the ratio of the presence of a peak of cells with an area of 150 µm² or greater and less than 250 µm² to the presence of cells or cell mass (cluster) with an area of 250 µm² or greater in particular is recorded. Next, after completion of the surgical operation, a small amount of blood of a patient is collected, a cell size distribution diagram thereof is acquired, and the presence/absence of a peak of cells with an area of 150 µm² or greater and less than 250 µm² to the that of cells or cell mass (cluster) with an area of 250 µm² or greater is checked in the same manner. If present, the cells are collected and subjected to a gene test to study whether there is residual metastatic cancer. If not present, blood can be periodically collected to test for the presence/absence of recurrence of metastatic cancer from comparison of a difference from the previous test at an interval of, for example, about once every six months.

Not only metastatic cancer diagnosis, but also liver diseases can be assumed if a cell cluster flowing in blood can be identified as a liver tissue fragment. If a sample can be identified as a fragment of other organs in the same manner, a disease can be assumed to be a disease of each of such organs.

With regard to especially phagocytic white blood cells such as macrophages, cells that have enlarged to a size greater than normal can be collected to identify a gene of a heterologous cell such as bacteria in the cells by a test for diagnosis of an infection in a short period of time. Alternatively, it is possible to diagnose what the immune system is responding to by selectively collecting cells with increased size or cells whose internal shape has become complex due to B cell activation and analyzing genetic information of an antibody produced by the B cells with a next generation sequencer or the like to elucidate the antigen.

### <3. Example of the overall configuration of cell analysis device system 1 that materializes the procedure shown in Figure 1 described above>

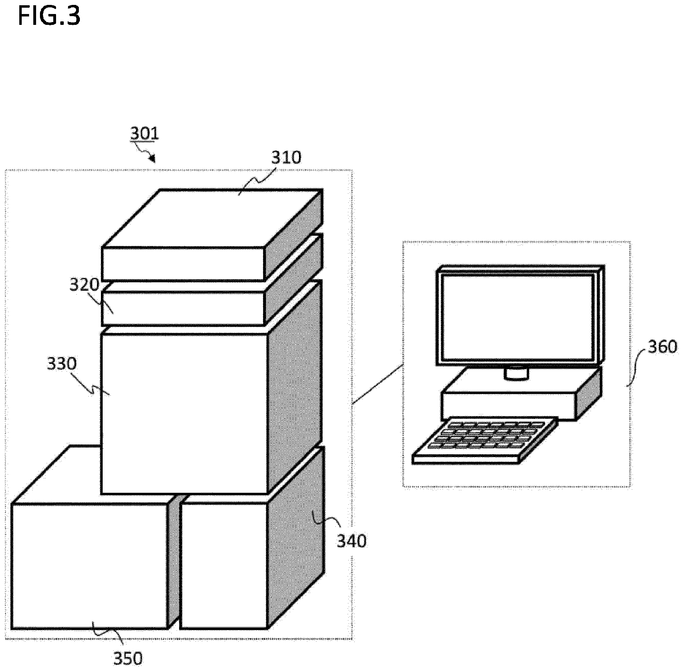

Figure **3** shows an example of the overall configuration of the cell analysis device system that materializes the procedure shown in Figure **2****.** In this example, a cell analysis device system **301** is configured so that a sample such as a blood sample flows towards the direction matching gravity from top to bottom. The system comprises a cell concentration/size fractionation/staining/washing module **310** for performing preprocessing of cells from an introduced blood sample, a cell/cell mass encapsulation module **320,** an image detecting single cell separation/purification module **330** for performing identification/purification of cells in a unit of a single cell, a gene analysis/expression analysis unit **340** for measuring an intercellular condition of purified cells at a single cell or single cell mass level, a contamination free re-culturing module **350** for re-culturing the purified cells, and a control/analysis module (computer) **360** for controlling the overall operation of the system and analyzing a result of analysis.

Specific examples of the configuration of each module in the example shows in Figure **3** are provided hereinafter.

### <4. Example of the configuration of a cell concentration/size fractionation/staining/washing unit for performing preprocessing of cells>

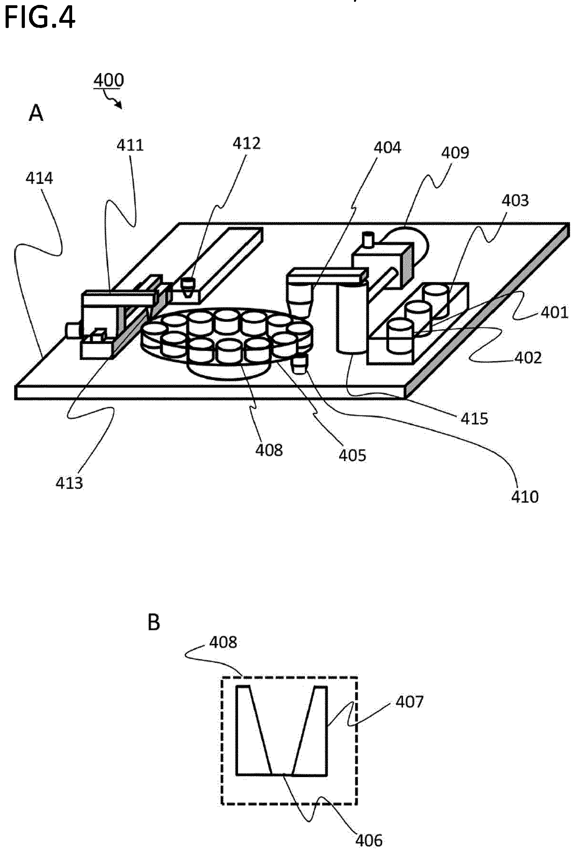

Figure **4** shows an example of the configuration of the cell concentration/staining/washing module **310** for performing preprocessing of cells from an introduced blood sample derived from a subject such as a cancer patient. In the example of Figure **4A****,** a cell concentration/size fractionation/staining/washing module **400** is disposed integrally on a chassis **414** as a cell concentration/size fractionation/staining/washing mechanism. Inside the module are containers or reservoirs **(401, 402, 403)** for retaining each of a cell sample, staining agent, and detergent, from which the solutions can be introduced into a concentration chamber **408** disposed on a turn table **405** by a dispenser head **404** attached to a rotary arm **415.** As shown in Figure **4B****,** the concentration chamber **408** consists of a sample solution retention chamber **407** with a slope toward the bottom surface where a concentration/bleaching filter **406** is disposed.

A cell sample such as blood is first introduced into the concentration chamber **408.** Cells are concentrated by discharging liquid components to a waste collection tube **410** placed below through the concentration/bleaching filter **406** from applying pneumatic pressure from the top surface of the concentration chamber with a pressure pump **409.** Next, a staining solution is introduced using the dispenser head **404** and reacted for a certain period of time, and then the staining solution is discharged again from the concentration chamber **408** with the pressure pump **409.** Next, a bleaching agent is introduced into the concentration chamber **408,** and so that excessive staining agent is washed away and discharged. The system is configured so that, subsequently, a diluent that also functions as a detergent in general is introduced to dilute the cells to a desired concentration, and the cells are introduced to a collection tube **412** through a collection head **411** comprising a collection chip **413** at the end thereof. The advantage of this approach is that a sample with few components having a size that can potentially cause clogging can be efficiently fractionated, stained, and washed by effectively utilizing a membrane filter.

### <5. Another example of the configuration of a cell concentration/size fractionation/staining/washing module for performing preprocessing of cells>

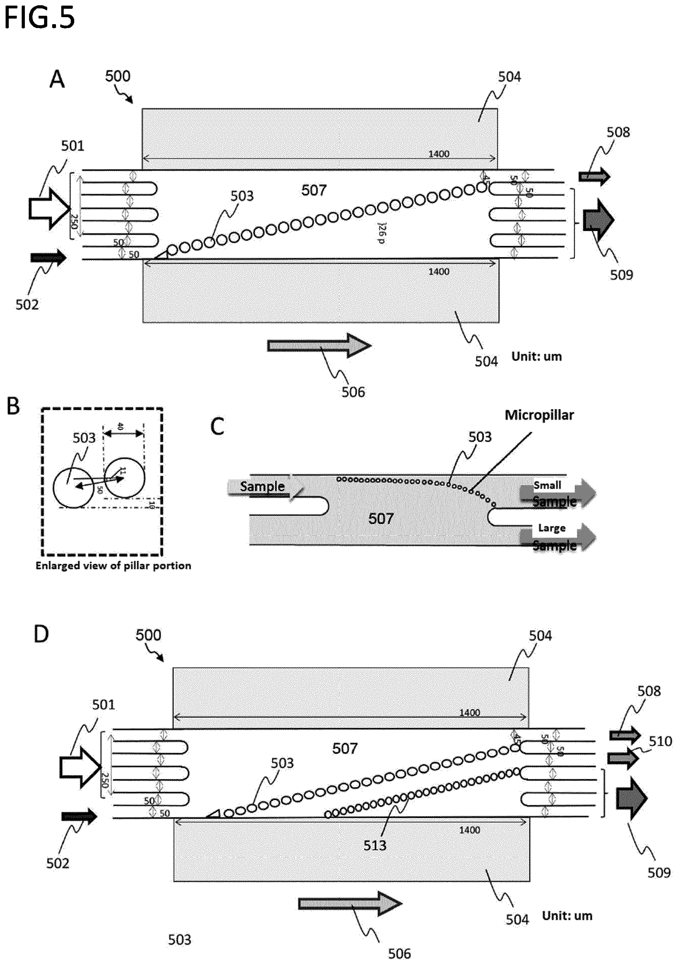

Figure **5** is an example of a cell concentration technology with a cell size fractionation function using a microchannel system for a sample with a size distribution that can potentially cause clogging or collapsing of cells, which result frequently upon purification of cells with a membrane filter as shown in Figure **4** described above. Figure **5A** is a top view of structure **500** of a microchannel for materializing this concentration technology. A cell free buffer **501** is poured in from upstream of a channel so that the buffer accounts for a region that is 3/4 of the channel width of more, and a sample solution **502** containing various sizes of cells is poured into a channel **507** in the direction of a flow **506** so that the solution accounts for a region that is the remaining 1/4 of the channel width or less. In the channel **507,** a pillar array **503** with a slope at both ends of the channel is disposed in the entire region from upstream to downstream, except for the upstream position of exit **508,** which is the final exit. As an alternating electric field application mechanism, a pair of electrodes **504** is disposed on walls of both sides that are orthogonal to the flow of the channel **506.** The system can be configured so that cells flow downstream while wavering in a sinusoidal manner by applying an alternating electric field in the direction orthogonal to the flow to the cells flowing in the channel **507** in the direction of the flow **506.** At the downstream of the channel **507,** two collection exits **508** and **509** are disposed. Particles larger than the distance (space) between adjacent columns of the pillar array **503** are captured by the pillar array when flowing the channel downstream due to a sinusoidal electric field and climb each pillar one by one like stairs to be ultimately collected at the exit **508.** Meanwhile, particles small than the distance (space) between adjacent columns of the pillar array **503** flow downstream while oscillating up and down centered around the same position as the position of entering the channel without being captured by the pillar array when flowing downstream the channel due to the sinusoidal electric field, so that the particles are ultimately collected at the exit **509.** In this regard, particles smaller than the distance (space) between adjacent columns of the pillar array **503** can be prevented from entering the exit **508,** which collects particles larger than the distance (space) between adjacent columns of the pillar array **503,** by configuring the channel width of the downstream exits **508** and **509** to be 1/4 or less and 3/4 or greater of the entire channel width, respectively. The intensity of the alternating electric field applied to cells is set so that the amplitude of oscillation of the cells is greater than the slop of adjacent pillar arrays and is 1/4 of the channel width or less.

Figure **5B** shows an example of an exploded view of the arrangement of adjacent pillars from the top. The cell sizes that can pass through can be controlled by disposing cylindrical pillars while setting the space therebetween to be the cell size to be fractionated.

Figure **5C** shows another example of the spatial arrangement of a pillar array. The arrangement is configured so that the initial slope on the upstream side is lower and the slope on the downstream side is higher to enable more efficient fractionation.

In order to solve the problem of the loss of the function of a membrane filter due to the pores on the membrane filter surface being all clogged and depleted in view of the technological design of a membrane filter fractionating sizes by capturing and preventing passage of cells with a size exceeding the pore size of the membrane, this technology enables continuous processing while preventing clogging of pores by guiding cells that could not pass through the pore size away from the pores while moving the cells to the collection channel in a step-wise manner. The technology is especially effective when collecting a small amount of cells with a size exceeding a threshold value, e.g., selectively collecting a small amount of a cell cluster.

As shown in Figure **5D****,** a plurality of different cells in a plurality of size regions can be simultaneously separated and collected by disposing a plurality of pillar arrays with different spacing in levels from those with a broader to narrower distance (space) between adjacent columns. At the downstream exit **508** for collecting large sized cells, a first level pillar array **503** set to have a larger distance between columns for such cells is disposed. This guides all cells or cell masses greater than the space of the pillar array to the downstream exit **508.** Next, a second level pillar array **513** set to have a smaller space than the first level pillar array can guide intermediate sized cells which are larger than the space between columns of the second level pillar array that cannot pass through the space to a downstream exit **510.** Cells that are smaller than either of them are all guided to the downstream exit **509.** This example shows three types of fractionation by combining pillar arrays with two types of spaces described above, but more fine and numerous size fractionation can also be materialized by arranging a greater number of pillar arrays disposed in order from a greater space between columns to smaller space between columns in the same manner.

### <6. Principle of operation of another example of a cell concentration/size fractionation/staining/washing unit for performing preprocessing of cells>

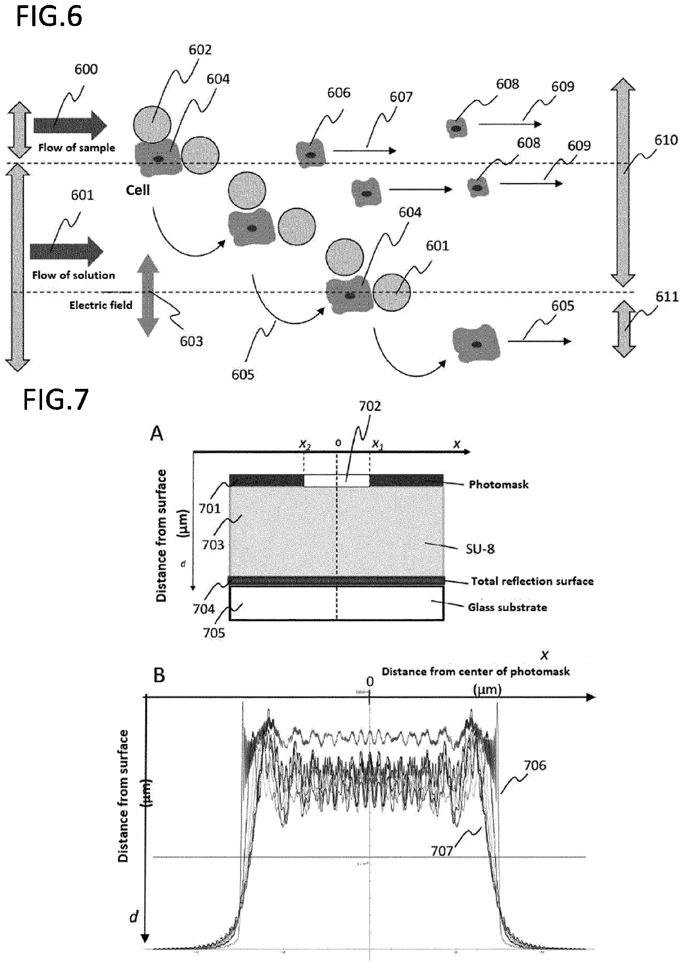

Figure **6** schematically shows the principle of the collection method using a pillar array shown in Figure **5****.** A flow **600** of a solution of a sample comprising cells to be fractionated is a part of the entire channel on the top side and is positioned with a sufficient space from the downstream exit for collecting large cells downstream. Meanwhile, a flow **601** of a cell free solution accounts for the majority of the channel on the bottom side, and a downstream exit **611** for collecting large cells is disposed in this region. A cell **604** larger than the space between columns of a pillar array would be stuck between pillars and cannot flow where a pillar array **602** is disposed diagonally with respect to the flow **600** of a sample solution. Meanwhile, cells **606** and **608** that are smaller than the space between pillars flow directly in the directions of flow **607** and **609** and are collected as cells that are smaller than the column space at a downstream exit **610.** Meanwhile, cells that are stuck between pillars due to being larger than the pillar spacing float in the direction of an electric field by applying a sinusoidal electric field that is orthogonal to the flow **600** of the sample solution, and continuously get stuck between the next pillar spaces each time to advance on the diagonally arranged pillar along the arrangement thereof (arrow **605**)**.** The cells are ultimately guided to a downstream exit **611** and fractionated thereby. In this regard, the magnitude of sinusoidal movement of cells in the direction orthogonal to the direction of the flow **600** of a sample due to an electric field **603** is about a magnitude that results in a greater amplitude than the space between adjacent pillars or greater. This allows cells to be collected at the exit **611** by advancing in steps on the diagonally arranged pillars as show by arrow **605.**

### <7. Microprocessing technology using a cell concentration/size fractionation/staining/washing unit for performing preprocessing of cells>

Figure **7** is a diagram that schematically describes an approach for making an upright pillar structure with a height of 100 µm or greater using a photocurable resin. The cell size fractionation using a pillar array shown in Figures **5** and **6** needs to consecutively dispose pillars with a height of 100 µm or greater to broaden the region where cell sizes to be fractionated can be simultaneously processed. It is important that the thickness of pillars is the same regardless of the height to enable the pillars to correctly fractionate by size. However, when using a photocurable resin such as SU8 using a photomask, the shape broadens for parts that are further away from an opening due to the light diffraction phenomenon, so that a trapezoidal shape would be pronounced at a height exceeding 100 µm. To prevent this, a total reflection surface can be placed at the bottom surface of a photocurable resin to change the shape from trapezoidal to an upright shape. Figure **7A** is an example of an arrangement for materializing the concept described above. An aperture **702** smaller than the shape of a pillar array to be constructed by about 10% is designed on a photomask **701.** For example, chromium is deposited 10 nm so that this would be a total reflection surface **704** on a glass substrate **705,** and then 200 nm of gold is deposited. A photocurable resin such as SU8 is disposed thereon to a height of about 100 µm. The photomask **701** described above can be placed to adhere thereto, and ultraviolet light can be irradiated for photocuring. Figure **7B** shows an actual simulation of a shape **707** of a region cured without total reflection from the bottom surface due to diffraction and shape **706** of a region cured with total reflection. The lines between line **707** and line **706** are each from reflectance with a value between 0% and 100%. The actual size of the aperture for materializing the shape that yields a desired diameter of a pillar is ultimately determined by checking the actual diameter of a photocured column. A microchannel shape with an upright pillar array can be effectively prepared from using another curable resin such as PDMS by using an upright photocurable resin prepared in this manner as a template.

### <8. An example of the configuration of a cell/cell mass encapsulation unit for encapsulating a cell>

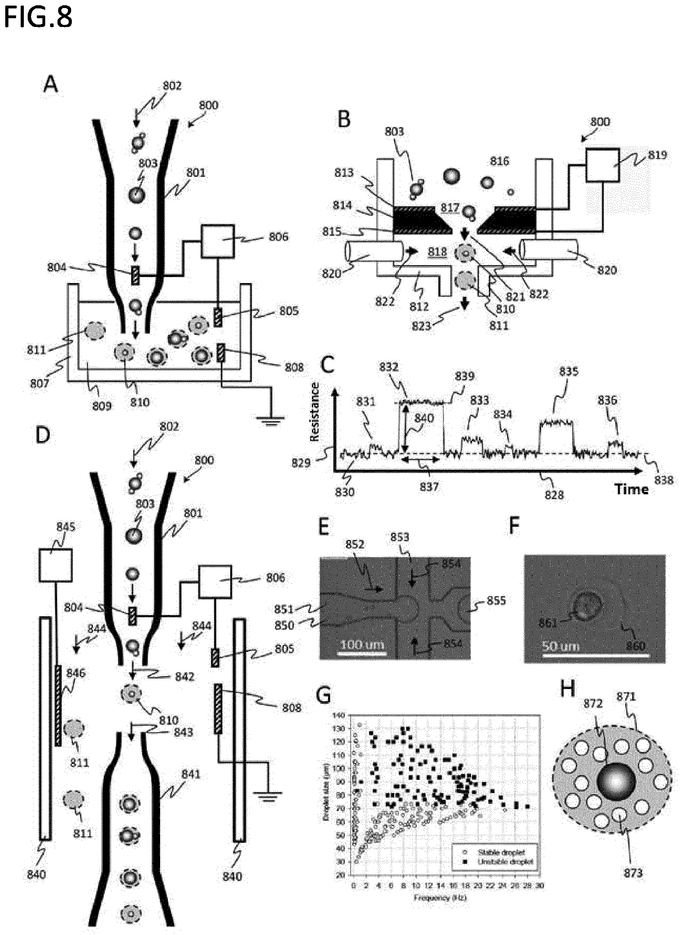

Figure **8** describes the constituent mechanism of an encapsulation unit **800** for preparing a capsule that encapsulates a cell in an alginic acid gel capsule.

Figure **8A** shows an example of an approach for encapsulating a cell as a capsule particle construction mechanism. Alginic acid gelates when a cell **803** or a cell mass in a solution **802** comprising alginic acid in a sol state flowing in a capillary tube **801** passes through the tip of the capillary tube and is discharged into a container **807** containing a solution **809** comprising a divalent ion such as calcium, so that a capsule **810** comprising a cell or a capsule **811** that does not comprise a cell can be made. In this regard, the presence/absence of a cell passing through a capillary tube, size of a cell or cell mass passing through, and actual time required for passing, as a flow rate, can be estimated from a change in the size of resistance obtained with a meter **806** from the change in the current between an electrode **804** within a capillary tube and an electrode **805** disposed in the solution **809.** A ground electrode **808** is also disposed in the solution **809** so that electric potential of each electrode can be stably measured at this time. An optimal alginic acid capsule size can be achieved by feeding back and controlling the actual discharge pressure of the solution **802** discharged from the obtained flow rate data. In this regard, a divalent ion placed in the solution **809** for making an alginic acid gel capsule can be calcium or magnesium, but barium, which has a property resulting in the inside of an alginic acid capsule to be hollow, is preferably used.

Figure **8B** shows another example of an approach for encapsulating a cell. A solution comprising the cell **803** from a region **816** of a cell solution comprising alginic acid in a sol state is gelated and discharged to a solution region **818** comprising a divalent ion for making a capsule via a region **817** that narrows down like a funnel, and the alginic acid capsule **810** comprising the prepared cell or the cell free alginic acid capsule **811** can be retrieved with a flow **823** in a structure **812** constructed by microprocessing. Two pieces of information, i.e., size of resistance and time at which resistance is increased, can be acquired when a cell is present in a cell solution being discharged in the same manner as Figure **8A** by constructing a structure in which an insulation layer **814** is sandwiched with a top conductive surface **813** and a bottom conductive surface **815** of the region **817** narrowed like a funnel for discharging and measuring a change in the amount of current therebetween with a meter **819.** In addition, the discharge rate can be adjusted by adjusting the pressure of the region **816** of a cell solution comprising alginic acid in a sol state. A channel **820** for supplying a solution comprising a divalent ion to the solution region **818** comprising a divalent ion is disposed and is configured to supply in the direction of arrow **822.**

Figure **8C** schematically shows example **830** of a result of measuring a change in resistance **829** generated when a solution comprising alginic acid in a sol state passes through a micropore with respect to elapsed time **828,** shown in Figures **8A** and **8B****.** If a cell passes through, small increases **831** and **834** in resistance occur with a cell that is small with respect to the pore size, moderate increases **833** and **836** in resistance occur with a moderate sized cell, and large increases **832** and **835** occur with a large cell. In this regard, if an increase in resistance is studied in detail, an increase consists of a resistance portion **840** determined only by the size of a cell with respect to the pore diameter and an elapsed time **837** during which a cell passes through the pore. The elapsed time during which a cell passes through the pore is a value obtained from the combination of the cell size and the flow rate at which the cell passed through the pore. Thus, if the elapsed time **837** is t, the diameter of the cell obtained from resistance is r, and the flow rate is v, t = r/v, so that the flow rate v can be found from v = r/t. Since the cell size actually found only with resistance **840** is determined to be dependent on the ion intensity of solution **802** and pore size, initial calibration is required, but once calibrated, the cell size and resistance can be unambiguously and reproducibly found as long as a solution and a capillary tube are not exchanged. A stable capsule can be constructed if v found in this regard can be fed back for control so that the size would be the desired alginic acid capsule size.

Figure **8D** schematically shows an example of a configuration constructed from adding a mechanism for collecting only a cell-containing alginic acid capsule to the alginic acid capsule construction mechanism described in Figure **8A** as a cell/capsule particle collection mechanism. As explained in the above description for Figure **8A****,** the moment at which a cell is discharged from a capillary tube can be distinguished by a change in resistance between the pair of electrodes **804** and **805** placed between solutions comprising a divalent ion inside and outside of the capillary tube **801.** For this reason, a capillary tube **841** for collecting an alginic acid capsule discharged downstream is disposed, an opening that is short to the extent that always one alginic acid capsule passes through is created between the two capillary tubes **801** and **841,** wherein an electrode **846,** a power source **845** for applying a charge thereto, and the ground electrode **808** are disposed, and a flow **844** of a solution is created in a tube **840** surrounding the two capillary tubes. By such a configuration, alginic acid capsules **811** that do not encapsulate a cell can all be discharged on a flow of the outside tube **840** by not applying an electric field when a capsule comprising a cell can be confirmed from an increase in resistance and applying an electric field otherwise.

Figure **8E** is a microscope picture of an example of actually constructing the configuration described in Figure **8B** by microprocessing. A cell solution comprising alginic acid in a sol state is discharged in direction of flow **852,** and an alginic acid capsule **855** comprising a cell can be made downstream by a flow **854** of solution comprising a divalent ion supplied from a channel **853** on both side surfaces.

Figure **8F** is one of the microscope pictures of an actually made alginic acid capsule. A single cell **861** is encapsulated in an alginic acid capsule **860.** As described in Figure **1****,** when a cell or the like encapsulated in alginic acid gel is observed as a bright field microscope image, the refractive index of a surrounding solution can be adjusted to the same value as that of the alginic acid gel so that the alginic acid gel is substantially transparent as the bright field image. Thus, only a cell or the like encapsulated in gel can be observed in the same manner as a cell that directly floats in the solution. To do so, sucrose, for example, can be added to an aqueous solution to be observed to increase the refractive index to give the same refractive index as that of the alginic acid gel capsule.

Figure **8G** is an example of an experimental result showing the relationship between the discharge speed (particle generation cycle) when a cell solution comprising alginic acid in a sol state is discharged from a pore and the size of a generated alginic acid gel capsule. As can be understood from this example, an alginic acid gel capsule with a stable particle size can be made by controlling the speed of the discharged solution. However, the relationship between the size of actually created gel and discharge speed varies depending on conditions such as the difference in the viscosity of the solution used, pore size, ion intensity in the solution, or the like. Thus, as a capsule particle size sorting mechanism, the flow rate can be optimized by feedback control through the approach described above in Figure **8A** to Figure **8C** in accordance with the optimal discharge rate matching the capsule size to be made in accordance with the actual configuration.

Figure **8H** schematically shows a superparamagnetic alginic acid cell capsule **871** encapsulating a cell **872** created by mixing ferromagnetic microparticles, such as iron or manganese, which are finely fragmented to be smaller than a magnetic domain structure so that an alginic acid solution in a sol state does not have a magnetic domain. By adding the ferromagnetic microparticles **873** that have become superparamagnetic to an alginic acid capsule, even if the specific gravity of the solution increases from adding sucrose or the like to increase the refractive index of the solution, this is balanced by an increase in the specific gravity of the alginic acid capsule.

### <9. An example of the configuration of an image detecting single cell separation/purification unit for identifying/purifying cells in a unit of a single cell>

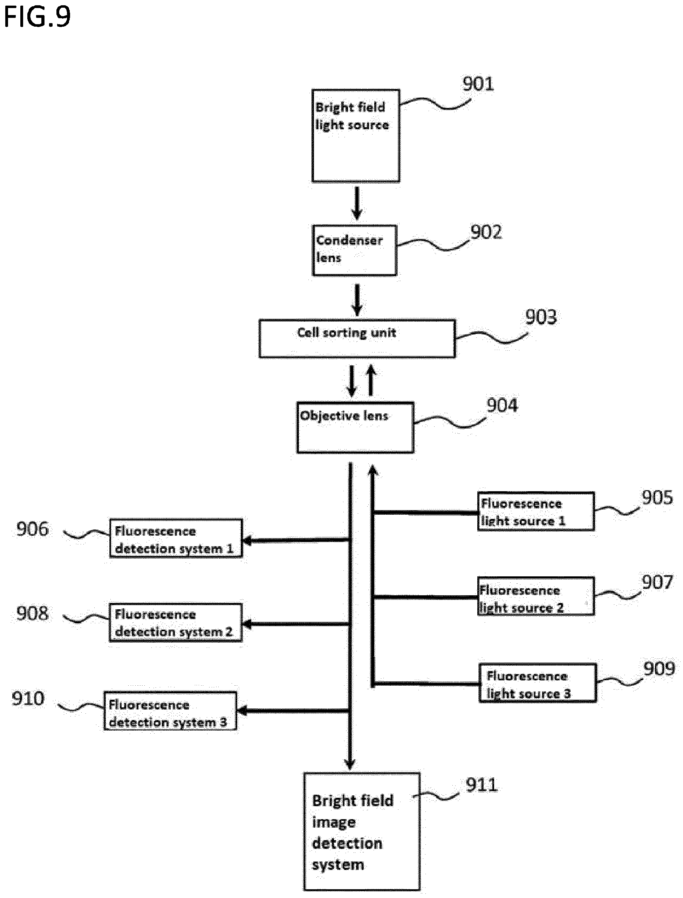

Figure **9** shows an example of the configuration of the image detecting single cell separation/purification (cell sorting) module **330** for identifying/purifying cells in a unit of a single cell in Figure **3****.** As shown in Figure **9****,** the image detecting single cell separation/purification (cell sorting) module **330,** in order to obtain a bright field image of a cell, irradiates visible light to a cell sorting unit **903** from a bright field light source **901** and a condenser lens **902** and a cell is acquired and analyzed as an image with a bright field image detection system unit **911** via an objective lens **904.** In order to obtain a fluorescence image of a cell, excitation light irradiated from fluorescent light sources **905, 907,** and **909** matching each fluorescence is irradiated to the cell sorting unit **903** via the objective lens **904,** and the resulting fluorescence image of cell can be detected as fluorescence matching excitation light at each of fluorescence intensity detection system units **906, 908,** and **910** again via the objective lens **904.** The intensity of scattered light can also be measured by making the excitation light wavelength and fluorescence wavelength the same. If fluorescence to be observed is a plurality of colors exceeding three colors in this Example, an optical path branching system can be suitably adjusted to allow a plurality of excitation lights to pass through, and then a wavelength that does not overlap with fluorescence (radiated light) wavelength for the detection of each excitation light and fluorescence is selected and light is irradiated onto cells, and a plurality of dichroic mirrors or band-pass filters can be combined in accordance with the type of fluorescence to be observed.

### <10. Another example of the configuration of an image detecting single cell separation/purification unit for identifying/purifying cells in a unit of a single cell>

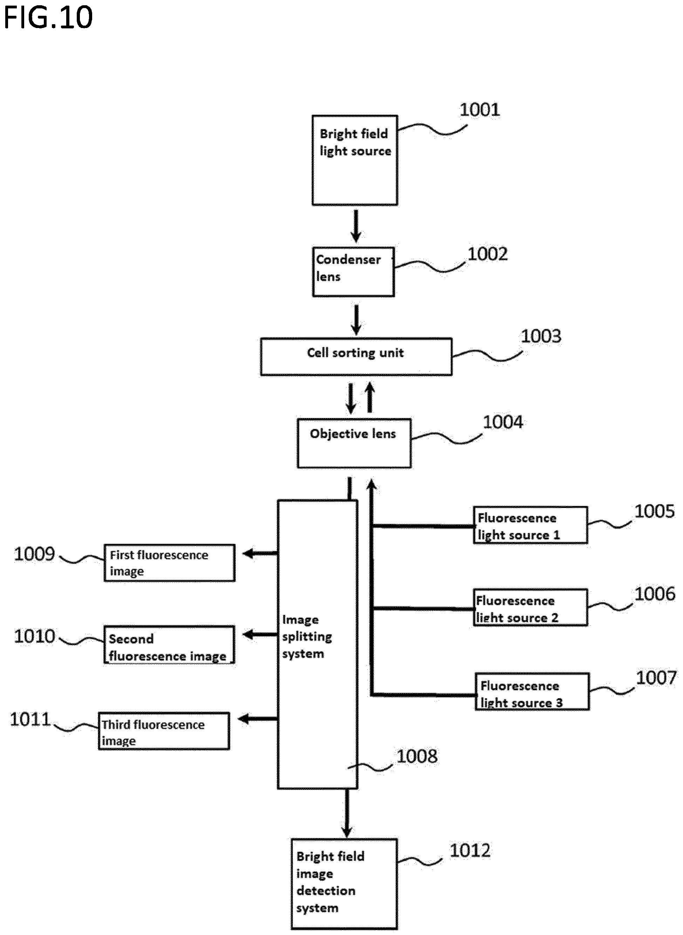

Figure **10** shows another example of the configuration of the image detecting single cell separation/purification (cell sorting) module **330** for identifying/purifying cells in a unit of a single cell in Figure **3****.** As shown in Figure **10****,** the image detecting single cell separation/purification (cell sorting) module **330,** in order to obtain a bright field image of a cell, irradiates visible light to a cell sorting unit **1003** from a bright field light source **1001** and a condenser lens **1002** and a cell is acquired and analyzed as an image with a bright field image detection system unit **1012** via an objective lens **1004** and an image splitting system unit **1008.** In order to obtain a fluorescence image of a cell, excitation light irradiated from fluorescent light sources **1005, 1006,** and **1007** matching each fluorescence is irradiated to the cell sorting unit **1003** via the objective lens **1004,** and the resulting fluorescence image (radiated light image) of cell can be acquired as fluorescence image matching excitation light at each of first fluorescence image detection system unit **1009,** second fluorescence image detection system unit **1010,** and third fluorescence image detection system unit **1011** again via the objective lens **1004** and the image splitting system unit **1008.**

In this regard, processing using a bright field microscope image or fluorescence microscope image can be concomitantly used with the processing using fluorescence intensity or scattered light intensity described in Figure **9****.** Image data obtained in a detection system can be observed by a user by displaying the data on a monitor in real-time. If the fluorescence to be observed is a plurality of colors exceeding three colors in this Example, an optical path branching system can be suitably adjusted to allow a plurality of excitation lights to pass through, and then a wavelength that does not overlap with fluorescence (radiated light) wavelength for the detection of each excitation light and fluorescence is selected and light is irradiated onto cells, and a plurality of dichroic mirrors or band-pass filters can be combined in accordance with the type of fluorescence to be observed.

When obtaining an image of a cell, the irradiation period of a light source for irradiating an acquired image can be optionally configured to be strictly "irradiation period = pixel size/flow rate of cell" to complete acquisition of an image in a light receiving screen while each point of a cell image is within the same pixel in order to prevent an image from blurring caused by a movement due to an image at each point of a cell being captured when straddling pixels during acquisition of each frame because of a cell moving in a flow. In addition, an image of a microstructure can be accurately recorded at the best resolution in principle within the range of resolutions of pixels of a light receiving screen.

### <10. Example of a combination of constituent elements of an image detecting single cell separation/purification unit for identifying/purifying cells in a unit of a single cell>

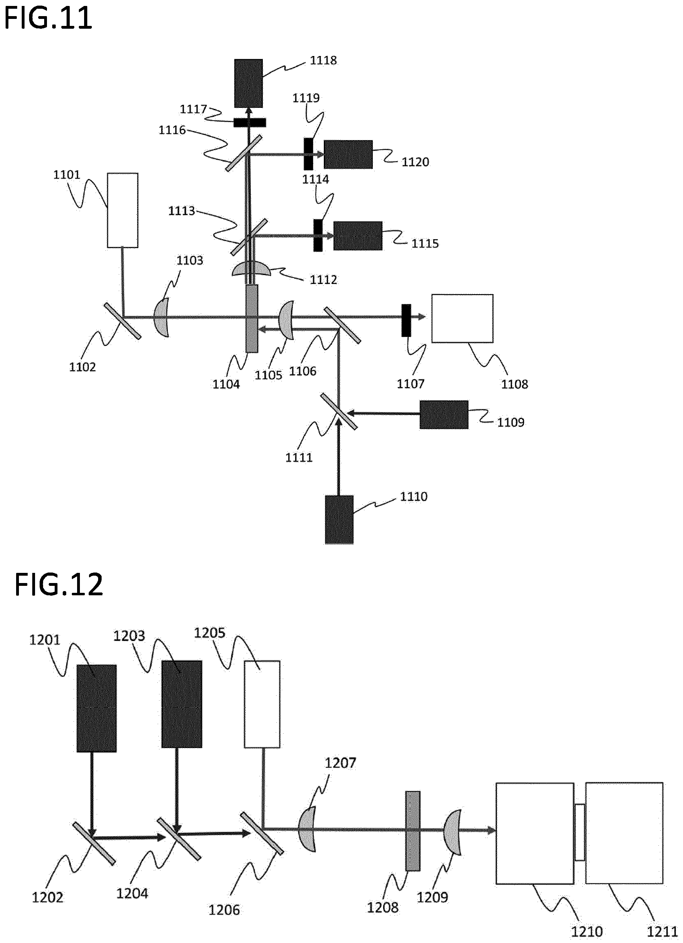

Figure **11** shows an example of constructing the configuration of the image detecting single cell separation/purification module for identifying/purifying in a unit of a single cell shown in Figure **9** by actually combining elements.

Light emitted from a bright field light source **1101** capable of periodically irradiating pulse light of 1 ms or less such as a monochromatic pulse laser or a high luminance LED visible region monochromatic light source is condensed at a channel portion of a cell sorter chip **1104** by a condenser lens **1103** after adjusting the direction of progression with a mirror **1102.** Fluorescence excitation light irradiated from a plurality of monochromatic fluorescence light sources **1109** and **1110** is condensed at a channel portion of the cell sorter chip **1104** by an objective lens **1105** via dichroic mirrors **1111** and **1106.** The light is outputted to two optical paths in this Example in order to measure a cell and cell mass flowing in a channel of the cell sorter chip **1104** with light condensed from these bright field light source and fluorescence light source. One is a scattered light measurement system using a high sensitivity light detection element side scatter photometer **1115** such as a photomultiplier for measuring light that has passed through a band-pass filter **1114,** which selectively passes only a wavelength of a bright field light source, via a dichroic mirror **1113** for measuring side scatter intensity of light with the same wavelength as a bright field light after disposing a condensing lens on a path on a side that is orthogonal to a bright field light source and a fluorescent light source, and a fluorescence intensity measuring system for measuring fluorescence with fluorescence photometers **1118** and **1120** consisting of a high sensitivity light detection element such as a photomultiplier incorporating, in the front stage, two band-pass filters **1117** and **1119** for detecting fluorescence excited by excitation light of each of the fluorescence light sources **1109** and **1110,** which passes through the dichroic mirror **1113** and is branched at the next dichroic mirror **1116.** The other is a path for observing an image of a cell and a cell mass flowing within a channel of the cell sorter chip **1104** on an optical path from a bright field light source and is configured to measure a beam of an image formed by the objective lens **1105** with a high speed camera **1108** via a band-pass filter **1107** passing light of a bright field light source.

As for irradiated light of a bright field light source, continuous light may be irradiated, but in order to improve the spatial resolution of an image so that there is no blur, this is optionally configured to generate a pulsed light for an irradiation period of 1/10 or less of a shutter cycle in each interval of a shutter cycle in synchronization with the shutter cycle of the high speed camera **1108,** so that an image with a higher spatial resolution can be acquired by preventing blur generated due to a flow of cell.

Obviously, results from processing using an image acquired by a high speed camera and processing using fluorescence or scattered light can be used together to distinguish cells for sorting.

Further, image data obtained by the high speed camera **1108** can be observed by a user by displaying the data on a monitor of an analysis device. If a plurality of fluorescence is to be observed, a filter is suitably adjusted to allow a plurality of excitation lights to pass, and then a wavelength that does not overlap with fluorescence wavelength for detecting fluorescence at a later stage is selected, and light is irradiated onto cells, and a plurality of device modules added with a configuration from a dichroic mirror to the filter and fluorescence detector can be combined in accordance with the type of fluorescence to be observed. A cell image can be acquired with fluorescence by optimizing a dichroic mirror and filter to a wavelength to be observed in this configuration in the same manner. The result of observing fluorescence can also be used as the data.

### <12. Example of combining constituent elements of an image detecting single cell separation/purification unit for identifying/purifying cells in a unit of a single cell>

Figure **12** shows an example of constructing the configuration of the image detecting single cell separation/purification module shown in Figure **10** in a unit of a single cell by actually combining elements. Bright field visible light irradiated from bright field light sources **1201** and **1203** with two different wavelengths, which are capable of periodically irradiating a pulse light of 1 ms or less such as a monochromatic pulse laser or high luminance LED visible region monochromatic light source and are each independently controllable, and fluorescence excitation light irradiated from a monochromatic fluorescence light source **1205** for irradiating a fluorescent dye excitation light are condensed at a channel portion of a cell sorter chip **1208** by a condenser lens **1207** after adjusting the direction of progression with a mirror **1202** and dichroic mirrors **1204** and **1206.** To measure a cell and cell mass flowing within a channel of the cell sorter chip **1208** with light condensed from these bright field light sources and fluorescent light source, in this example, a bright field microscope image and fluorescence microscope image formed by an objective lens **1209** are both guided to an image splitting system unit **1210** and separated into a respective image of a single wavelength, and each image is used by splitting a single high speed camera light receiving screen to simultaneously arrange and display images of a plurality of wavelengths on a single pixel screen, so that images of a plurality of wavelengths can be simultaneously processed by an image processing for a single acquired image.

While this Example was configured to dispose two bright field visible light sources with different wavelengths so that two bright field images can be captured, a detailed bright field image of a cell and moving speed of a cell in a flow can be simultaneously acquired from one image acquisition with such a configuration. Specifically, at the first bright field visible light source, a period of flash lighting from a bright field light source for irradiating an acquired image can be configured to be strictly "flash period = pixel size/flow rate of cell" to complete acquisition of an image in a light receiving screen while each point of a cell image is within the same pixel in order to prevent an image from blurring caused by a movement due to an image at each point of a cell being captured when straddling pixels during acquisition of each frame because of a cell moving in a flow. In addition, an image of a microstructure can be accurately recorded within the range of resolutions of pixels of a light receiving screen.

Meanwhile, the period of light emission of the second bright field light source can be configured to be sufficiently longer than the period of light emission of the first bright field light source to record an image of a cell, in a light receiving screen, as an extension of an image straddling pixels while a light source flash is emitted. The length of extension can be found by comparison with the length of a cell obtained by the first flash light source from "flow rate of cell = length of extension of cell/difference in length of flash light emission period of two light sources". The flow rate of cell obtained in this regard can be found, and the maximum light emission period of flash of the first bright field light source can be fed back for control. For fluorescence images, if flash can be emitted from a fluorescent light source, image blur would be minimized if the flash period can be configured to be "flash period = pixel size/flow rate of cell" in the same manner as a bright field light source described above, so that an image at the best spatial resolution at the pixel level can be acquired in principle.

While this Example described a measurement method using an image, processing using an image and processing using fluorescence or scattered light can also be concomitantly used in combination with the approaches shown in Figures **9** and **11****.** Image data obtained with a high speed camera **1211** can be observed by a user by displaying the data on a monitor. If a plurality of fluorescence is to be observed, a filter is suitably adjusted to allow a plurality of excitation lights to pass, and then a wavelength that does not overlap with fluorescence wavelength for detecting fluorescence at a later stage is selected, and light is irradiated onto cells, and a plurality of image splitting system units **1210** with a dichroic mirror and filter added thereto can be combined in accordance with the type of fluorescence to be observed.

### <13. Example that schematically shows the configuration of an analysis system for simultaneously performing high speed bright field microscope image acquisition and high speed fluorescence microscope image acquisition>

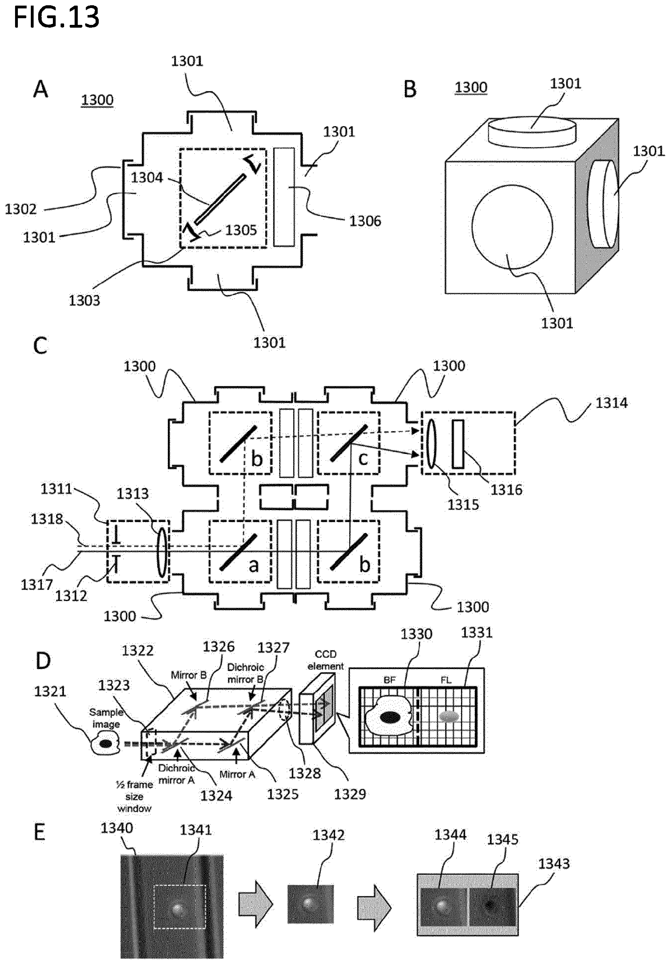

Figure **13** schematically shows an example of the configuration of an image splitting system and the configuration of an image acquisition system using the same shown in Figures **10** and **12****.** In this example, square cubes can be combined like building blocks so that the image splitting optical system can be flexibly configured or modified.

Figure **13A** schematically shows the cross-section of the inside of a cube-shaped container **1300.** The cube container **1300** has open windows **1301,** which can connect with another cube on all six surfaces and are configured to be closable with a lid **1302** when not connected to another cube. The container is configured so that a dichroic mirror **1304** having an angle adjustment mechanism **1305,** which can three-dimensionally and finely adjust the direction of reflection at a center **1303** of the cube, and a filter **1306** can be fitted in between each window **1301** and the center **1303.**

Figure **13B** schematically shows the outer appearance of the cube shown in Figure **13A****.** The windows **1301** are placed on all six surfaces of the cube.

Figure **13C** schematically shows an example of the mechanism of an image splitting optical system using this cube. First, before introducing an image into an image splitting optical system, the image obtained from an objective lens is processed by cutting out an excessive image region from the center of an optical path and introduced into an image size adjustment system unit **1311** consisting of a movable shielding plate **1312** for adjusting a plurality of split images into a shape that can be arranged in parallel on an image imaging element **1316** of a high speed camera **1314** and a lens **1313** for converting an image after adjustment of size into parallel light. An image exiting the image size adjustment system unit **1311** is introduced into an image splitting optical system constructed by combining four cubes **1300.** When, for example, monochromatic lights **1317** and **1318** of two different wavelengths are introduced into the image size adjustment system unit **1311,** the lights are first introduced into a first cube having a dichroic mirror a with a (wavelength) high-pass filter or (wavelength) low-pass filter incorporated therein as images with the same image region cut out in the same size. As the dichroic mirror, a dichroic mirror that allows passage of the monochromatic light **1317** but totally reflects the monochromatic light **1318** is selected. Next, the direction of progression of the monochromatic light **1317** is changed 90 degrees with a total reflection mirror **b.** The light is reflected with a dichroic mirror **c** with a (wavelength) high-pass filter or (wavelength) low-pass filter, which reflects the monochromatic light **1317,** but allows the monochromatic light **1318** to pass, incorporated therein, and is acquired as an image with an image imaging element **1316** via a lens **1315** for forming the light as an image again. In this regard, the position of a cell image of the monochromatic light **1317** on the image imaging element **1316** can be freely adjusted by three-dimensionally and finely adjusting the angle of the total reflection mirror **b** or the dichroic mirror **c.** Meanwhile, the monochromatic light **1318** is reflected by the dichroic mirror **a,** introduced into the other total reflection mirror **b** to change the angle 90 degrees, and then passes through the dichroic mirror **c,** so that the monochromatic light **1318** can be observed as a cell image on the image imaging element **1316** via the lens **1315.** In this regard, the position of the total reflection mirror **b** is adjusted so that the image does not overlap with the image of the monochromatic light **1317** on the image imaging element **1316.** In this Example, it is not particularly necessary to insert a filter for selecting a wavelength such as a band-pass filter as the filter **1306.** When a bright field image and a fluorescence image, or images of two wavelengths with significantly different light intensities such as two bright field images with different flash lighting periods, an absorption filter such as an ND filter is inserted on the side of the optical path with a greater intensity, which has an effect that enables adjustments of images into two images with the same light intensity for measurement on the image imaging element **1316,** and simplification of optimization of amplification of the dynamic range and cutting offset of light intensity for each location.

The Example described above shows an example of the configuration for splitting an image of two wavelengths and acquiring images with a high speed camera. Meanwhile, images of all wavelengths can be acquired from a single capture using one high speed camera by arranging a plurality of images having different wavelengths, with a size that does not overlap on a light receiving surface, so that they do not overlap by cutting out an excess region on both sides of an inputted image to the minimum size required by an image size adjustment system within an image splitting system. In this regard, even for a plurality of monochromatic lights, the positions of each image on a light receiving surface of a high speed camera can be freely adjusted by adjusting the position of a surface of the dichroic mirror **1304** with a plurality of angle adjustment mechanisms **1305** capable of three-dimensional and fine adjustments. For a plurality of bright field images or fluorescence images, images with different magnifications can be formed on a single high speed camera light receiving surface **1316** by incorporating an optical lens system at a position of a filter of a cube on an optical path of an image of a specific wavelength after separating wavelengths for magnification or contraction. This can be applied especially in decreasing the magnification of a bright field image for measurement including the surrounding state of a cell and increasing the magnification of a bright field image or fluorescence image for checking the detailed circumstances within a cell.

The optical system combining images with different magnification is not limited to applications for an imaging cell sorter, but can also be incorporated for use into a common optical bright field/fluorescence microscope system for similar observation in a static cell sample selectively collected with an imaging cell sorter.

Figure **13D** schematically shows how a cell is observed via an image splitting system unit **1322** using the configuration of Figure **13C** described above and how cells are observed as two images of a bright field image and nuclear stained fluorescence image in terms of the combination of mirrors as an image acquisition camera mechanism. An image of a cell **1321** goes through an optical system such as an objective lens and through a 1/2 sized vertically-long rectangular movable shielding plate **1323.** A fluorescence image is reflected by a first dichroic mirror **1324,** while a bright field image directly passes through, introduced to and reflected by a total reflection mirror **1325,** and reflected by a second dichroic mirror **1327** to be projected on half **1330** of an image imaging element **1329** via an image forming lens **1328.** Meanwhile, the fluorescence image reflected by the first dichroic mirror **1324** is reflected by a total reflection mirror **1326,** passes through the second dichroic mirror **1327,** and is projected onto the remaining half **1331** of the image imaging element **1329** via the image forming lens **1328.**

Figure **13E** shows an example of using the configuration of the present disclosure in absorption spectrum imaging technologies. The visible light spectrum of a light source is changed to continuous light instead of monochromatic light, and an image of a cell **1341** flowing in a microchannel **1340** is acquired with an objective lens using the light source, and then only an image **1342** of a part of a region is cut out from the actual obtained image with the movable shielding plate **1323** and introduced into an image splitting system. An image from reflecting absorption of a specific wavelength in a visible light image of a cell can be obtained by adding a band-pass filter in addition to the dichroic mirror. For example, when stained with a light absorbent reagent that selectively stains a nucleus, a cell image **1344** can be observed as a normal image in a region outside of the absorbed wavelength, but the nucleus portion is dark due to absorption in an image **1345** of a wavelength in an absorbed wavelength region of a nuclear staining reagent. Light absorption spectrum imaging images reflecting absorption at each wavelength of a plurality of wavelengths can be acquired. A cell is generally in the GO phase, and the nucleus is present, which is clearly recognized in an image as a black sphere within a cell by using this approach. Meanwhile, a nucleus is lost in a cell in a mitotic phase, so that a clear spherical nucleus cannot be found within the cell from image recognition of the cell. In particular, there are many reversible labeling reagents with low cytotoxicity as a light absorbing reagent. Thus, it was difficult to check the state of a cell with conventional labeling technologies such as antibody labels. Meanwhile, cells in a mitotic phase can be collected depending on the presence/absence of a nucleus within a cell, in addition to checking the shape of a cell, by the two wavelength bright field image comparison analysis technology of the present disclosure. In general, most normal cells flowing in blood have completed the final differentiation. Meanwhile, cells with division potential such as cancer cell in blood or stem cells can be collected by collecting cells undergoing cell division in blood by the present disclosure.

The present disclosure can also extract only an image of a cell by subtracting pre-recorded image data from when a cell is not flowing from a bright field image, e.g., an image of a flowing cell, for the plurality of obtained images. For this reason, the cell size (area) and cell circumference length can be found from the total number of pixels within a region with remaining data after subtraction and the total number of pixels at the boundary of a region with remaining data, respectively, and can be determined from only a bright field image and a cell cluster from comparison of the directly obtained circumferential length with converted circumferential length found from an estimated diameter from the cell volume as described in Figure **1****.**

Likewise, as described in Figure **1****,** a fluorescence image of a nucleus is obtained from a fluorescence image (nuclear staining) **1331,** and the number of nuclei, area of nucleus, and the overall fluorescence intensity, i.e., integrated value of luminance, can be obtained. Since a bright field image and a fluorescence image simply captured the same location at different wavelengths, the coordinate axes thereof match. For this reason, the cell shape cannot be measured with a fluorescence image, but a relative position of a stained nucleus within a cell can be estimated by utilizing the coordinates in a bright field image. For this reason, a single isolated normal cell with one nucleus can be identified, and data can be acquired in the same manner for a cell cluster of multinucleated cells. Likewise, similar processing can be performed in a system forming a plurality of images with different magnification by combining and using a relative coordinate system that takes into consideration the difference in the magnification ratio centered around the same origin (center of image).

Further, the image size preparation system unit **1311** and each cube **1300** in the present disclosure are fixed in a form that maintains an optically sealed state. A cross-sectional area of an image of incident light cut out with the movable shielding plate **1323** is adjusted to an area that is equal to or less than (total area of light receiving surface/parallel light introduction module) in the total number of acquired images of a plurality of wavelengths of a plurality of wavelengths measured last for projecting independent images for the number of modules to be linked so that they do not overlap in an image imaging element **1329** on a high speed camera.

### <14. Example that schematically shows the configuration of an analysis system for simultaneously measuring fluorescence intensity, acquiring high speed bright field microscope image and acquiring high speed fluorescence microscope image>

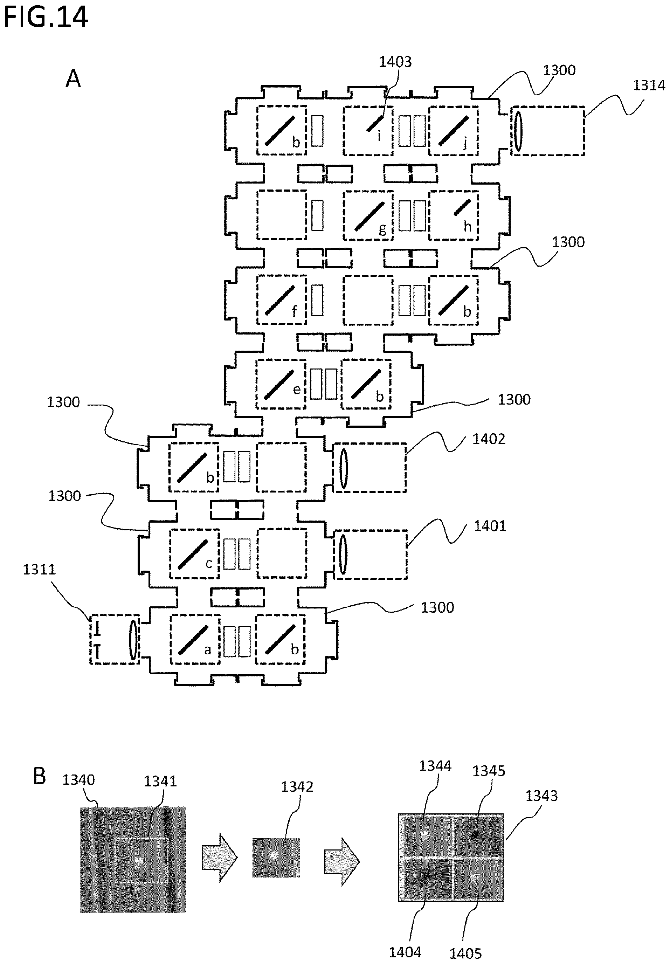

Figure **14** is a diagram that schematically shows an example of a specific configuration, which adds a configuration for acquiring images of a plurality of wavelengths shown in Figure **10** to a configuration of an analysis system for simultaneously measuring fluorescence intensity and acquiring a high speed bright field microscope image shown in Figure **9****.**

In this example, four high luminance LED flash light sources emitting monochromatic light of different visible regions are used as a bright field (high speed camera) light source to obtain light absorption spectrum imaging images of four wavelengths, and 375 nm and 488 nm lasers are used as fluorescent pigment excitation light sources. A dichroic mirror is disposed so that wavelengths are branched in steps from short wavelength light to long wavelength light. In addition, intensity of fluorescent dyes in a cell excited with 375 nm or 488 nm lasers is quantitatively measured with fluorescence photometers **1401** and **1402** consisting of a high sensitivity light detection element such as a photomultiplier, and cell images of a long wavelength region obtained from four LED flash light sources are split with an image splitting system as images of each wavelength and placed in a high speed camera. This enables simultaneous measurement of fluorescence intensities at various wavelengths in a cell sorter chip disposed in a microchip holder and bright field images of a plurality of wavelengths of cells.

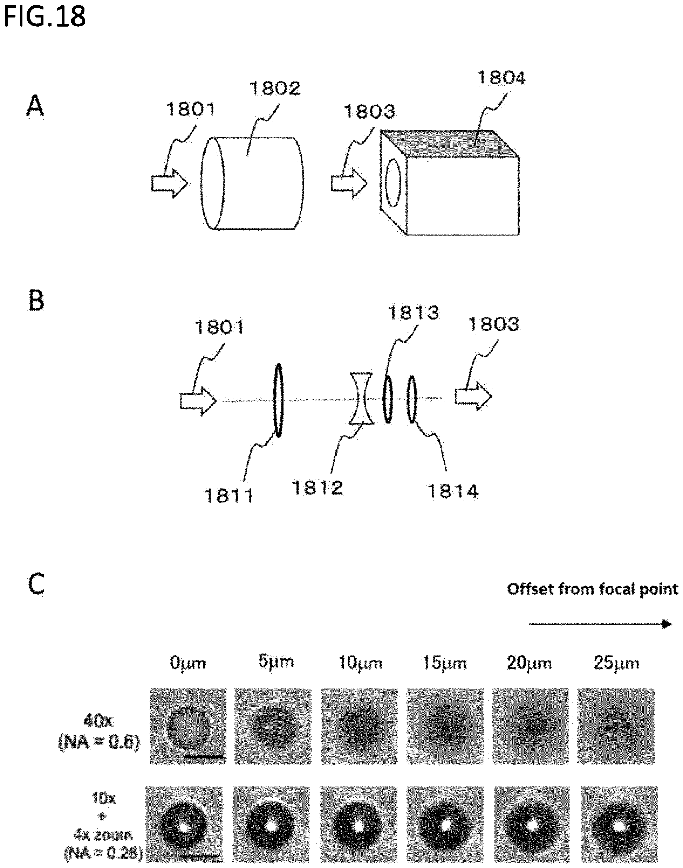

Figure **14A** is a diagram that schematically shows an example of an arrangement of the configuration of an analysis system for simultaneously measuring fluorescence intensity and acquiring a high speed bright field microscope image. Monochromatic light for observation irradiated from a bright field light source such as four LED flash light sources synchronized with a frame interval of a high speed camera is condensed with a condenser lens, and irradiated onto a cell in a cell sorting unit comprising a cell sorter chip with a cell sorting mechanism and a channel where a target cell in blood is flowing incorporated therein. The cell in the channel can be focused with an objective lens. In this regard, a depth of field improvement technology described below in Figure **18** can be optionally incorporated. Fluorescent excitation light irradiated to an objective lens from a fluorescence light source such as two monochromatic lasers can generate fluorescence from a fluorescent antibody for identifying a cancer cell such as a fluorescently labeled EpCam antibody or K-ras antibody, cytokeratin antibody, or CD antibody bound to a cell in a channel described above, a nucleus stained with a nuclear staining fluorescent dye (DAPI, Hoechst 33258, or the like), or the like. Two fluorescent lights branched from a short wavelength fluorescence with the dichroic mirror **a** in an image splitting optical system using a cube and branched with the dichroic mirror **c** adjusted to a cut-off wavelength in the middle of two fluorescence wavelengths can be quantitatively measured, one goes through a band-pass filter for removing excessive wavelengths and directly by a fluorescence photometer **1401** consisting of a fluorescence intensity measurement system such as a photomultiplier tube or photodiode, and the other fluorescence wavelength, has the optical path changed by the total reflection mirror **b,** then goes through a band-pass filter for removing excessive wavelengths, and directly by a fluorescence photometer **1402** consisting of a fluorescence intensity measurement system such as a photomultiplier tube or photodiode, respectively. In this example, two lines were described as an example of a fluorescence detection system, but a plurality can be freely combined. Furthermore, at a later stage, fluorescence intensity of a cell can be detected in this manner via an image splitting system consisting of a combination of the cubes **1300** for branching and splitting an optical microscope image exemplified in Figure **13** as images of each of a plurality of wavelength regions to simultaneously acquire a plurality of images with a single high speed camera light receiving element, while simultaneously acquiring a bright field image of a cell with a high speed camera **1314.**

The configuration of a device for simultaneously acquiring a plurality of microscope images branched by wavelengths with a single high speed camera light receiving surface is the following. Image data of light on the long wavelength side after completing branching of short wavelength light guided to a fluorescence intensity measurement unit with the dichroic mirror **a** is first introduced into the first image splitting unit via the total reflection mirror **b.** In this regard, with a specific wavelength as a cut-off wavelength, a long wavelength region or short wavelength region from said wavelength can be reflected, and introduced into the next optical branching system, with a dichroic mirror **e** with an angle adjustment function capable of three-dimensional and fine adjustment of the direction of reflection. In this regard, a dichroic mirror and filter at a later stage consist of an intensity adjustment ND filter for aligning the intensities of images of each wavelength on a high speed camera to a certain degree, a band-pass filter for obtaining a sharper image of a wavelength bandwidth, or the like. The angle can be adjusted with a dichroic mirror, disposed within each cube **1300,** with an angle adjustment function capable of three-dimensional and fine adjustment of the direction of reflection, so that the plurality of obtained split images do not overlap on the light receiving surface of a high speed camera. All optical paths are adjusted to be the same so as not to create a difference in the optical paths for the light handled. Further branching into a plurality of wavelengths can be promoted by incorporating half-sized dichroic mirrors **i** and **h.**

Figure **14B** shows an example of four branched images that were actually obtained. Images **1344, 1345, 1404,** and **1405** in four absorption wavelength bandwidths obtained from branching of wavelengths can be obtained as a single image on an image imaging element. In this diagram, three image splitting units consisting of similar configurations were combined, but a required number of split images can be combined by further combining the cubes **1300.**

This Example shows an example of an optical system for splitting and acquiring bright field images of a plurality of wavelengths, but fluorescence images of a plurality of wavelengths can also be acquired by adjusting the configurations of a dichroic mirror and band-pass filter for branching wavelengths.

### <15. Schematic outer appearance of another example of the configuration of an optical module portion of an analysis system for simultaneously measuring fluorescence intensity, acquiring high speed bright field microscope image and acquiring high speed fluorescence microscope image>

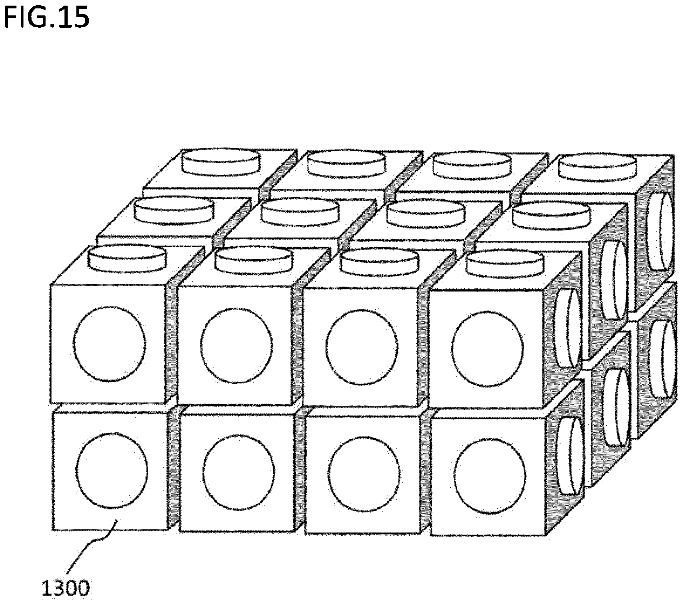

Figure **15** schematically shows the outer appearance of an image splitting system combining a plurality of cubes **1300** described above in Figures **13** and **14****.** Branched light can be efficiently processed in parallel by three-dimensionally arranging the cubes.

### <16. Example that schematically shows an example of the configuration of the chip of an image detecting single cell separation/purification (cell sorting) module>

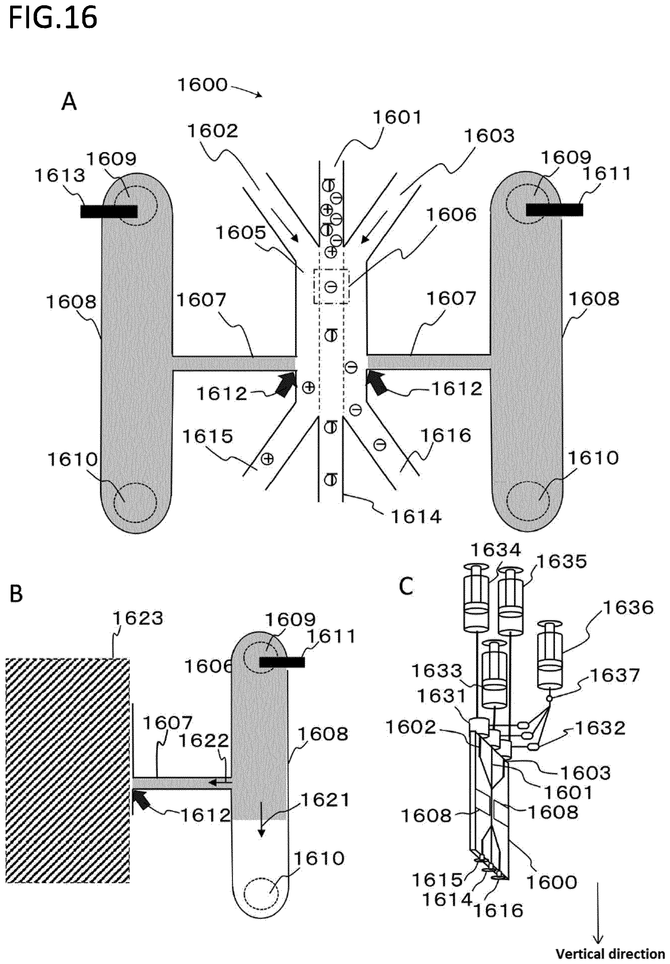

Figure **16** schematically shows an example of the configuration of a cell sorting unit of the image detecting single cell separation/purification (cell sorting) module of the cell analysis device system of the present disclosure shown in Figures **9** and **10****.**

Figure **16A** shows an example of a combination of constituent elements of a cell sorter chip for separating cells with the cell sorting unit of the present disclosure that can also be used in the cell analysis device system of the present disclosure. At a cell sorter chip **1600,** three axially symmetric channels are disposed symmetrically on the upstream side (**1601, 1602,** and **1603**) and downstream side **(1614, 1615,** and **1616)** on a chip substrate. At the merging point of the three channels, the three channels merge while maintaining the laminar flow, and branch into three downstream channels while maintaining the same state. Thus, the upstream side **1601** of the center channel where a sample flows is guided to the downstream center channel **1614,** and two side sheath flows, i.e., the upstream channel **1602** and the upstream channel **1603,** are guided to the downstream channel **1615** and the downstream channel **1616,** respectively.

As shown in Figure **16C****,** the cell sorter chip **1600** and each channel are configured to be vertically installed, so that a sample flows in the direction of gravity. The inlets of the three upstream channels are connected to a reservoir **1631** filled with a sheath fluid at the opening of each inlet. In particular, the upstream center channel **1601** can continuously introduce a sample solution comprising cells via the center reservoir with a syringe **1633.** A sheath fluid can be added and supplied to each reservoir connected to the channels **1602** and **1603,** which are channels flowing on the sides of the upstream side from syringes **1634** and **1635** filled with a sheath fluid. This enables continuous processing of a large amount of samples. By incorporating a fluid level measurement sensor using measurement of the presence/absence of conductance on the wall surface of the sample solution introducing syringe **1633,** sheath fluid introducing syringes **1634** and **1635,** and each reservoir, each sample solution can be supplied via the syringe until reaching a certain height when the fluid level is at or below a certain level. A fluid level measurement sensor can be composed of an electrode or electrode pair disposed at the upper limit and lower limit of a desired fluid level. The top portions of the three reservoirs **1631** are connected to a pneumatic syringe **1636** that functions as a pneumatic pressure source for applying pneumatic pressure for adjusting the air pressure of the fluid surface via a distribution valve **1632.** Pressurized air obtained from a pneumatic syringe goes through a pressure sensor **1637,** and pressure can be more flexibly distributed using the three distribution valves **1632.** When the heights of fluid surfaces of three reservoirs **1631,** i.e., sample reservoir and sheath fluid reservoir, are aligned and a flow rate is generated by adding pressure to the fluid surfaces using pressurized air after placing a cap on the top surface of the reservoirs for generating and controlling the flow rate, an ideal laminar flow is generated at the merging point. Thus, the shape of a channel cross-section and the distance from the merging point to the fluid inlet are optimally the same for each of the three channels on the upstream side and downstream side from the merging point, so that the flow rate of the three channels are the same. It is also desirable that the ratio of the cross-sectional area of a side sheath flow reservoir and the cross-sectional area of a sample reservoir are the same. If the change in the fluid surface heights of each reservoir is different, the ratio of decrease in the height of a fluid surface would be different, which ultimately disrupts the generation of a laminar flow at the merging point. If this is generalized, it is desirable that the ratio of the total cross-sectional areas of channels connecting to each reservoir matches the ratio of the cross-sectional area of each reservoir.

At a point at which three laminar flows without a wall, where all six channels including the three upstream channels and three downstream channels merge shown in the center of Figure **16A****,** a region **1606** for observing cells flowing in the center is disposed, and a pair of electrodes **1607** is disposed downstream in a manner that opposes the side surfaces of the channel. Electrodes are typically composed of a gel electrode. Examples of the gel used include agarose gel in which NaCl is dissolved so that an electrolyte would be a current carrier. Agarose gel in a sol state is added to a wide channel **1608** for loading gel from an inlet **1609** so that the tip of gel can directly contact the channel. Since the gel can move towards an outlet **1610,** the gel mainly moves toward the outlet **1610** and extends to the electrode **1607,** which is a small tube, due to capillary action and stops at the boundary surface due to surface tension without entering the cell sorter channel **1605.** Use of a gel electrode is advantageous in that a high ionic current can be applied to a liquid in a channel because bubbles are not generated and an electrode does not elute out even if the voltage is raised to a voltage that results in bubbles in a channel or elution of a metal electrode when using a normal metal electrode or higher at the boundary between a gel electrode in contact with the channel and sheath fluid in a cell channel by inserting electric wires **1611** and **1613** such as platinum wires connected to a power source for applying an electric field at the gel introduction point.

Next, the procedure for collecting cells in a sample in a cell sorter chip is the following. The sample solution flow **1601** flowing from upstream is sandwiched by the flows **1602** and **1603** of two side sheath solutions and proceeds to the cell observation region **1606** in one line while maintaining the arrangement at the center of the channel. Then, the shape of each cell is distinguished, the presence/absence of a fluorescent label, etc. is checked, and the cells are separated downstream based on the result thereof. One of two approaches is used for collection. One is an approach of not applying an electric field on cells to be collected and applying an electric field to other cells. In this regard, when cells to be collected are flowing, the cells are allowed to flow directly to a sorted sample collection channel **1614** downstream, and when cells or microparticles to be discarded are flowing, the cells or microparticles can be moved to one of two side sheath flows **1605** and discarded by applying a voltage to the two opposing gel electrodes **1607** regardless of whether the charge thereof is positive or negative.

The other collection approach is an approach of applying an electric field when a cell or cell mass to be collected has arrived. When cells to be collected are flowing in such a case, the cells are moved to one of the two side sheath flows **1605** and collected by applying a voltage to the two opposing gel electrodes **1607.** Meanwhile, cells that are not to be collected can be allowed to flow directly to the sorted sample collection channel **1614** and discarded. In this regard, cells generally have a negative surface charge, so that this charge is utilized. If an alginic acid capsule is used, cells can be collected by utilizing a strong surface charge more stably.

When effectively applying an external force on cells with an external electric field, the composition of a solution with an ionic strength resulting in the conductivity of an aqueous sample solution of 10² µS/cm or less is desirable. Such a composition facilitates movement of microparticles in a sample solution with an electric field. Specifically, it is important that the composition of the solution maintains osmotic pressure while reducing ionic strength when sorting especially viable cells. For example, it is preferable to use a molecule that does not directly contribute to an increase in ionic strength, such as a saccharide or polymer, as a sample solution upon purification of cells.

When capturing cells as an image for evaluation, cells are separated accurately by providing a site for observing the channel portion **1606** after merging with a CCD camera and expanding the range of measurement to a plane to distinguish and track cells by image recognition. What is important at this time is the image capturing rate. Cells are miscaptured as an image with a common camera with a 30 frames/second video rate. Cells flowing at a significant rate in a channel can be recognized with at least a 200 frames/second capture rate.

The first step of image processing method is cell recognition. As described above, the moving speed of cells varies by cells, and cells can pass other cells in some cases. To prevent passing, it is important that a sample solution is sandwiched by two side sheaths to arrange the cells in one line. Next, each cell is numbered when a cell first appears on an image frame, and are managed thereafter with the same number until the cell disappears from the image frame. Specifically, the status of movement of a cell image in a plurality of consecutive frames is managed with a number. Cells in each frame transition to the downstream side in order from the cells that are upstream, and the cells between frames are linked under the condition that the moving speed of a specific numbered cell recognized in an image is within a certain range. For cell numbering, a cell image is first binarized, and the center thereof is found. The center of luminance, area, circumferential length, major axis, and minor axis of the binarized cells are found, and each cell is numbered using these parameters. Auto-saving each cell at this point as an image is beneficial to users, so that auto-saving is enabled.

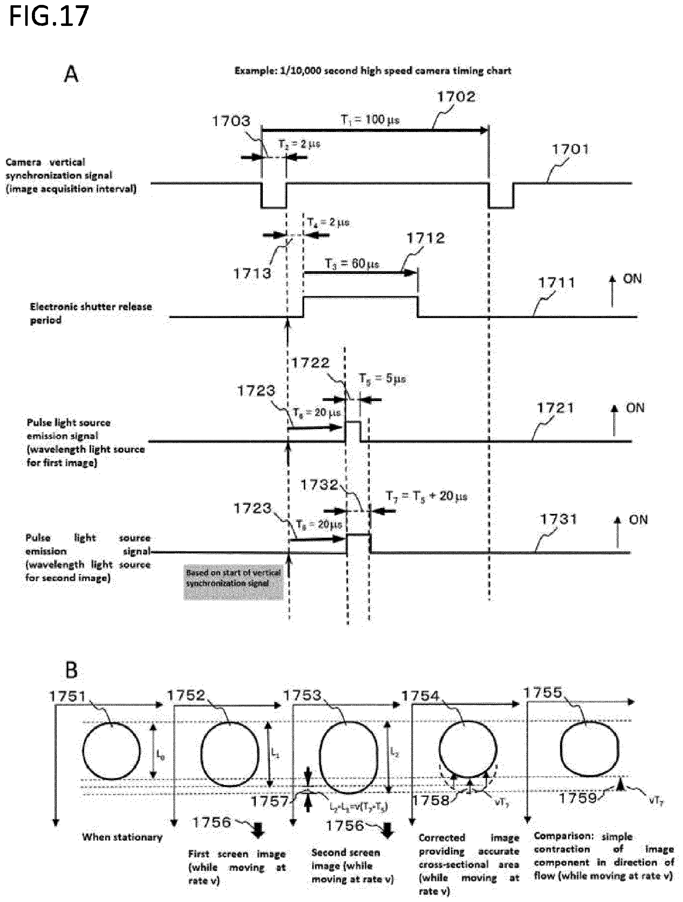

Next, if this is used in cell separation, only specific cells among the numbered cells must be separated. An indicator for separation can be information such as the center of luminance, area, circumferential length, major axis, and minor axis described above, or information utilizing fluorescence by concomitantly using fluorescence detection in addition to an image can be used. In either case, cells obtained at a detection unit are separated in accordance with the numbering. Specifically, the moving speed (v) of cells acquired by an approach detailed in Figure **17** is calculated, and the application timing is set to (A/v) to (A/v + T), wherein the distance from a detection unit to a sorting unit is (A) and application time is (T) with respect to the cell moving speed (v), so that the cells are electrically sorted and separated when a cell with a number of interest just arrives between electrodes.

As described above, a cell separation/purification module is constructed in the cell sorter chip **1600.** A microchannel is embedded inside a cell sorter chip substrate. An opening is provided on each end of a channel to provide an opening for supplying a sample or required buffer (medium) or for collecting sorted cells. A channel can be created by the so-called injection molding, which pours in plastic such as PMMA into a mold, or by adhesion of a plurality of glass substrates. Examples of the size of a cell sorter chip include, but are not limited to, 50 × 70 × 1 mm. When using PMMA plastic so that cells flowing in a channel of a groove carved into the inner surface of a cell sorter chip can be observed with a high magnification optical microscope, an adhesive emitting fluorescence is not used, but for example a laminate film with a thickness of 0.1 mm is used through thermocompression. For glass, 0.1 mm of glass is similarly used by optical adhesion. For example, cells flowing within a channel can be observed through a laminate film with a thickness of 0.1 mm by using an objective lens with a numerical aperture of 1.4 and magnification of 100x. If plastic with high light transmittance is used as the plastic, cells can also be observed from the top side of a chip substrate. Cells envisioned by the present disclosure include bacteria as small cells and cancer cell clusters and the like as large cells. Thus, the cell size is typically in the range of about 0.5 µm to 200 µm, but the cell size is not strictly limited to this range. Any size of cell can be used as long as the present disclosure is effectively used. When cell concentration and cell separation are consecutively performed using a channel incorporated into a surface of a substrate, the first issue is the channel width (cross-sectional shape). The channel **1605** is typically created in a substantially two-dimensional plane in a space of 10 to 100 µm inside and outside in the direction of thickness of the substrate on one of the substrate surface. In view of the cell size, a suitable size would be 5 to 10 µm in the direction of thickness for bacteria, and 50 to 100 µm in the direction of thickness for animal cells.

### <17. Example that schematically shows the relationship between an electronic shutter and light emission timing of a high speed flash light source in an image detecting single cell separation/purification (cell sorting) module>

Figure **17** is an operation timing chart that schematically shows the relationship between the irradiation period and timing of a light source and an interval of image acquisition when using a high speed camera that can actually obtain images at an interval of 1/10000 seconds.

First, Figure **17A** shows the temporal relationship of timing signals for a vertical synchronization signal **1701** of a camera, release period **1711** of an electronic shutter, a light emission period **1721** for an emission signal of a monochromatic pulse light source of a first bright field image, and emission period **1731** for an emission signal of a monochromatic pulse light source of a second bright field image, as a cell flow rate acquisition mechanism. A shown by the vertical synchronization signal **1701** of a camera, an interval **1702** of image acquisition, with an interval of 100 µs, starts operation by a 2 µs start timing signal **1703** with this camera. Next, an electronic shutter is released for only the release period **1711** after the 2 µs of the timing signal **1703.** The release period can be freely adjusted between, for example, 27 µs to 96 µs during a period in the interval of the camera, but this Example is set to release the shutter for 60 µs, for example, so that an image can be acquired. Next, light is emitted from bright field light sources of monochromatic light with two different wavelengths during the electronic shutter release period. The first light source emits light for a short light emission period **1721** and is used for acquiring mainly a microstructure of a cell. For example, in this example, light is emitted for a short period **1722** of 5 µs at 20 µs after the timing signal **1703.** The second light source is mainly used to find the flow rate of moving cells by emitting light for a longer period than the first light source. Light is emitted for a relatively long period **1732** of 25 µs, by adding 20 µs of the light mission period of the first light source, at 20 µs after the timing signal **1703** in the same manner as the first light emission. This allows the flow rate of a cell to be instantaneously measured by acquiring two bright field images from two bright field light sources with different wavelengths simultaneously in split regions of a light receiving screen of a single high speed camera, and comparing and measuring how much the cells have been extended in the direction of flow and exposed to light by the difference in the irradiation periods from the light sources. An image with the least amount of blur due to movement can be acquired by setting the light emission period of the first light source so that the period of movement of cells exposed to light during the light emission period of the first light source would satisfy "flash period **(1722)** of first light source = pixel size/flow rate", from the acquired flow rate of cells, to fit within the range of sizes of a single pixel of a light receiving pixel.

If, for example, the pixel size of a 1/10,000 second camera is 12 µm × 12 µm, the pixel resolution when observed using a 20× objective lens is 0.6 µm/pixel. Thus, an image without blur can be actually acquired if an LED light source that can fire a 5 µs flash is used when the cells flow at 12 cm/s.

Furthermore, the best bright field image from the first light source without blur due to a flow can always be acquired by using an image blur suppression mechanism, which utilizes the flow rate of cells obtained by comparing light emission of the first bright field light source with that of the second bright field light source and finely adjusts the light emission period of the first light source from feedback control so that "flash period **(1722)** of first light source = pixel size/flow rate" is satisfied.

Figure **17B** is a diagram that schematically describes the principle of an approach of simultaneously acquiring a cell image obtained by a pulse light source described above and thereby acquiring a highly detailed image, and a moving speed of cells as a cell shape correction mechanism. If there is a spherical sample **1751** observed as a circle with a diameter of L₀ when stationary, this is assumed to be moving in a downward direction **1756** at a flow rate of v. When the electronic shutter is released for exposure to light at this time, and then light is irradiated from a bright field light source for only a period of T₅ from a pulse light source, a circular shape is overlaid for the amount of light emission period from a light source and becomes a shape **1752** in which a portion that is parallel to the direction of progression of the circle is elongated, so that the length in only the direction of flow reaches L₁ as a whole. If, at this time, (L₁-L₀) is less than the size of a pixel when projected to a high speed camera capturing an image, an image that is the same as a static cell as an image can be acquired. However, if the irradiation period of a light source is reduced, a pixel capturing an image cannot obtain a sufficient amount of light. Thus, it is preferable to select a long period of irradiation by a light source to the extent that (L₁-L₀) would be the same as the pixel size as much as possible, together with increasing the light source intensity. Next, if light is irradiated at the same timing as the start of irradiation of T₅ during T₇, which is a longer period than T₅, from a bright field light source with a second wavelength, an image **1753** from overlaying spheres due to movement of a sphere for the irradiation period is acquired in the same manner as irradiation of the first bright field light source described above. (L₂-L₁) **(1757** in the figure) can be found from the length L₂ extended in the direction of flow. The (L₂-L₁) should match v(T₇-T₅), wherein v is the exact speed at which a sphere flows, so that this can be found from v = (L²-L¹) / (T⁷-T⁵). In this regard, the image splitting system unit **1008** described in Figure **10** can be used to simultaneously acquire the two images **1752** and **1753.**

When finding the accurate size of a sphere (area and circumferential length) by using the obtained speed v of a sphere that is flowing, the size can be accurately found by, for example, the procedure described below from the image **1753.** Specifically, if the end point (dotted line), located on the downstream side from the location of the maximum diameter orthogonal to the flow in the image **1753** obtained by irradiation from a bright field light source for T₇, is translated by only a distance **1758** of ᵥT₇ as the new end point position, while keeping the shape of the end point at the upstream side the same, an image **1754** that is the same as an image when stationary can be obtained based on an overlaid image during light exposure.

If the image **1753** is simply contracted by the amount of extension obtained from flow rate v as an axial component in the direction of flow, this does not result in an accurate original shape that is the same as an image when stationary, but instead results in a shape of the image **1753** compressed in the direction of flow, as can be seen from image **1755.** Thus, the correct original contour, circumferential length, and area cannot be found.

It can be understood, in view of the above, that finding an accurate flow rate of a sample flowing in a cell sorter chip is essential not only for acquiring the shape of a cell in high detail to the same extent as the shape in a stationary state, but also for acquiring an accurate shape of a cell. For image acquisition with a high speed camera, it is also extremely important to simultaneously find the moving speed of a cell to be analyzed by an image from only one image acquisition for a high precision and high throughput operation. Since the present technology can simultaneously acquire an image of an observed cell for analysis and the precise flow rate of the cell, the timing of a cell separation operation downstream can be determined as an accurate time to materialize highly precise cell separation.

Although the above Example described an example using a bright field light source and a bright field image, it is obvious that an exact same mechanism can also be constructed using a fluorescent light source and a fluorescence image.

### <18. Diagram that schematically shows an example of the configuration of an optical system for preventing image blur in an image detecting single cell separation/purification (cell sorting) module>

Figure **18** is a diagram that schematically shows an example of the configuration of an optical system for preventing image blur in an image detecting single cell separation/purification (cell sorting) module. When observing a target particle using the optical system of a microscope, the magnification of an image of a sample is generally determined only by the magnification of an objective lens. In such a case, the depth of focus and depth of field of the optical system would be dependent on the numerical aperture and magnification of the objective lens, so that the depth of focus and depth of field of the optical system would become shallow as the magnification of the objective lens is increased.

When observing a sample in a microchannel and the sample is a cell, the width and depth of the channel need to be of a sufficient size for a sample with a maximum size to flow in order for samples with various sizes to flow, such as small samples with a size of about several microns to a cluster with a size of 10's of microns. However, it is preferable that the resolution of an image is higher in order to distinguish the type of sample from image recognition. In general, an objective lens with a higher numerical aperture is used to increase the magnification with an optical microscope. Meanwhile, this was problematic in that if such means is used, the depth of focus would be shallow, resulting in the depth of field in a channel to also be shallow. In order to increase the magnification of a target sample and configure the depth of field to be about the height of a channel for identifying a sample from a more highly detailed image with an image recognition cell sorter, an objective lens with a numerical aperture resulting in the depth of focus and the depth of field of the objective lens to be about the height of a channel can be selected, and a zoom lens can be placed in the later stage of the objective lens. Specifically, as shown in Figure **18A****,** an image **1801** from an objective lens can be magnified with a zoom lens **1802** at a later stage of the objective lens to capture the magnified image **1803** with an image acquisition device **1804** such as a high speed camera.

Figure **18B** shows an example of the configuration of an optical element of a zoom lens system used in the present Example. The optical element is configured so that the magnification of the image **1801** entering from an objective lens is changed by a combination a convex lens (focusing lens) **1811,** concave lens (variator) **1812,** and two convex lenses, i.e., (compensators) **1813** and (master lens) **1814,** to output the magnified image **1803.** When composed of four lenses in this manner, the magnification can be flexibly changed while maintaining the full length of the zoom lens constant through an adjustment by moving the concave lens **1812** in the middle without moving the positions of the convex lenses **1811** and **1814** at both ends, and moving and adjusting the movable convex lens **1813** to correct movement in the focal point associated therewith.

Figure **18C** is a diagram showing a comparison of exemplary images of microparticles observed with a conventional optical system with images of microparticles observed with the optical system of the present disclosure. Specifically, it can be understood that an image is already blurry at about 5 µm with an objective lens with a numerical aperture of 0.6 and magnification of 40. Meanwhile, it can be understood that an image can be acquired without any problems up to about 25 µm, when measuring an image capturable height, with the same level of final magnification by using a 10× objective lens with a numerical aperture (NA) of 0.28 and combining a 4x zoom lens optical system therewith. This result shows an example of a configuration that can capture an image without blur in the direction of height of a channel, which is optimal for cell sorting, if a 10× objective lens and a 4x zoom lens are combined when an image with the same magnification as an image observed conventionally with a 40x objective lens is obtained with an image processing cell sorter system.

### <19. Diagram that schematically shows an example of the configuration of a high speed continuous image acquisition system using a line sensor set in an image detecting single cell separation/purification (cell sorting) module>

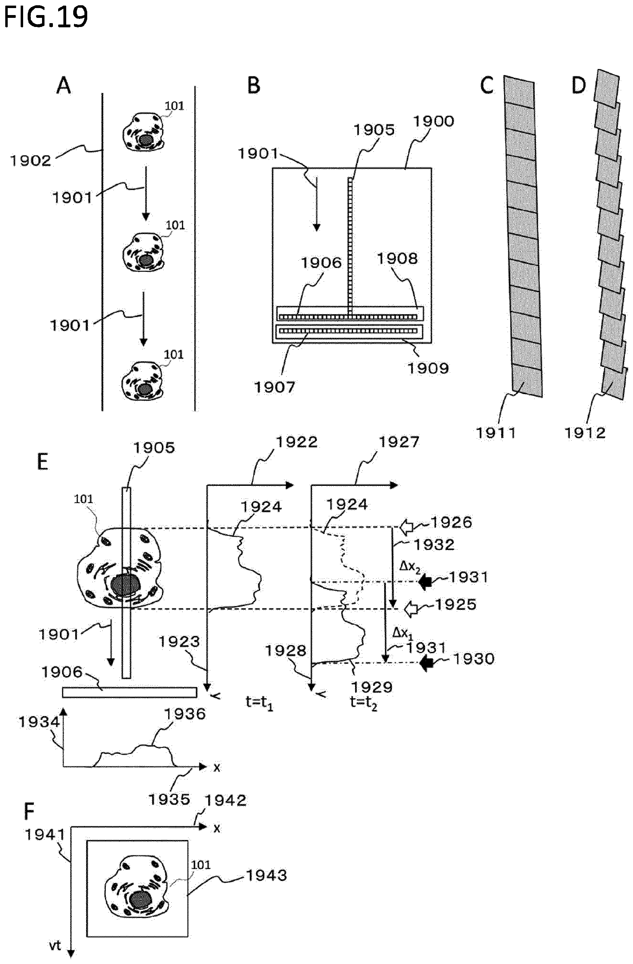

Figure **19** schematically shows an example of the configuration of a high speed continuous image acquisition system using a line sensor set as another example of image acquisition in an image detecting single cell separation/purification (cell sorting) module.

Figure **19A** schematically illustrates the concept of this approach.

Figure **19B** schematically shows an example of the configuration of a high speed continuous image acquisition system using a line sensor set. An image of a cell **101** is formed on a surface on an image acquisition plate **1900** via an optical system unit **1903** in a channel disposed so that the cells flow in one line in the direction of arrow **1901** at the center of a channel **1902** of a cell sorting unit. A flow rate detecting one dimensional sensor array **1905** is disposed in the first flow direction along the direction of the flow of the cell on the plate surface. A bright field image acquiring one dimensional sensor array **1906** with a length that can cover the entire width of the channel is disposed in the direction that is orthogonal to the flow. Optionally, a second image acquiring one dimensional sensor array **1907** can be disposed on the downstream side of the first image acquiring one dimensional sensor array **1906.** In this regard, a band-pass filter **1908** that allows transmission of only bright field light is placed above the sensor array **1906,** and a band-pass filter **1909** that allows transmission of only fluorescence is disposed above the sensor array **1907,** so that the first image acquiring one dimensional sensor array **1906** can acquire a bright field image, and the second image acquiring one dimensional sensor array **1907** can acquire a fluorescence image as an image splitting mechanism **1.** Since a high speed camera consisting of a two dimensional sensor could only acquire a two dimensional image at a certain interval, there was a void in time between intervals, so that cells needed to flow in series with a greater interval than such a temporal interval. Meanwhile, when the one dimensional sensor array of this Example is used, information can be obtained from each sensor continuously without a gap. For this reason, a cell shape can be reconstructed and obtained from consecutive signals from an image acquiring one dimensional sensor array if the flow rate of a cell can be found by means such as a flow rate detecting one dimensional sensor array.

Figure **19C** schematically shows the structure of a one dimensional sensor array. Information is continuously acquired as intensity information when light is irradiated onto each element by disposing each optical sensor element **1911** one-dimensionally. Such a one dimensional sensor array in this arrangement is used in each of the flow rate detecting one dimensional sensor array **1905** and image acquiring one dimensional sensor arrays **1906** and **1907** described above.

Figure **19D** shows an arrangement of each element **1912** in the one dimensional sensor array shown in Figure **19B** with a slope instead of in a plane. The size of depth of field can be improved by such an arrangement. A one dimensional sensor array with such an arrangement can be used especially in the flow rate detecting one dimensional sensor array **1905** described above.

Figure **19E** is a schematic diagram describing the role and function of each one dimensional sensor array disposed on the image acquisition plate **1900** which has actually formed an image of the cell **101.** From an image of the cell **101** moving on the flow rate detecting one dimensional sensor array **1905,** an intensity spectrum **1924** of cells as shown in the graph at time t₁ (light intensity axes **1922** and **1927** of cells, and axes **1923** and **1928** of one dimensional sensor array position) can be acquired, and then an intensity distribution **1928** of cells as shown in graph **1927** at time t₂ can be acquired. The moving speed v can be found by v = Δx₁/(t₂-t₁) or v = Δx₂/ (t₂-t₁) from the amount of movement Δx₁ (arrow **1931)** from tip portion positions **1925** to **1930** and the amount of movement Δx₂ (arrow **1932)** from end portion positions **1926** to **1931** when the intensity distribution **1924** has moved to the intensity distribution **1928.** When the image of the cell **101** reaches the image acquiring one dimensional sensor array **1906,** a one dimensional light intensity distribution **1936** shown in the graph consisting of an axis (x axis) **1935** for the position of the sensor array and light intensity **1934** measured by each sensor can be obtained. An image of the cell **101** can be reconstructed as a two dimensional image **1943** shown in Figure **19F** by reconstructing this intensity distribution as an image reconstruction mechanism as a vt axis **(1941)** combining the speed v and the spatial distribution (x axis) **1942** obtained from a flow rate sensor array in a spatial arrangement with respect to the respective acquisition time t.

### <20. Diagram that schematically shows an example of the configuration of a line sensor array set for acquiring a plurality of images on an image formation surface and the configuration of a line sensor array set for simultaneously acquiring a polychromatic fluorescence image>

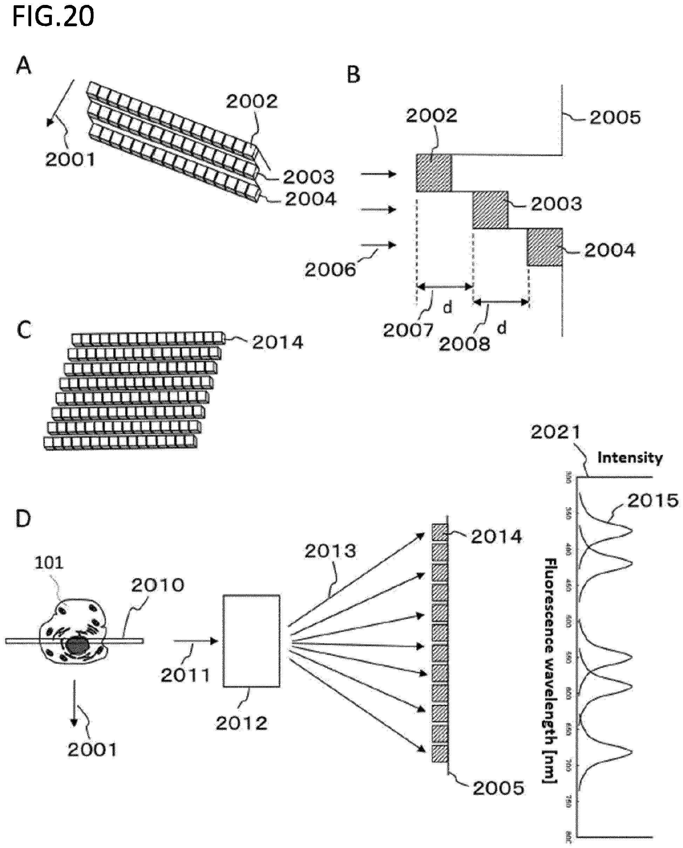

Figure **20** is a diagram that schematically shows an example of the configuration of a line sensor array, which can simultaneously acquire a plurality of images of different heights of image formation surfaces and the configuration of a line sensor array for simultaneously acquiring polychromatic fluorescence images in an image detecting single cell separation/purification (cell sorting) module.

Figure **20A** schematically shows the configuration in which image acquiring one dimensional sensor arrays **2002, 2003,** and **2004** described in Figure **19** are arranged in parallel in the direction of flow **2001** of cells at different heights on the image acquisition plate **1900** as an image blur suppression mechanism for preventing image blur. Figure **20B** shows the cross-section of Figure **20A****.** Substantially, images at a plurality of heights of image formation surfaces can be simultaneously acquired by disposing the one dimensional array **2004** above a bottom surface **2005** of the image acquisition plate so that the height is different from the bottom surface **2005** of an image acquiring plate by a height of d, and disposing the second one dimensional array **2003** at a position **2008** which is higher by just a height of d, and disposing the third one dimensional array **2002** at a position **2007** which is higher by just a height of d.

Figures **20C** and **20D** schematically show the configuration of a light receiving element array for branching an image of a cell by wavelengths and simultaneously acquiring images. As a wavelength spectrum separation mechanism, the same number of image acquiring one dimensional sensor arrays **2014** as the number of wavelengths to be acquired are disposed in parallel as shown in Figure **20C****,** and beams are acquired in a line that is orthogonal to a flow from an image **2011** of a cross-section **2010** that is orthogonal to the flow of the cell **101** flowing in the direction of arrow **2001** as shown in Figure **20D** and are transmitted to a wavelength branching mechanism to deploy a wavelength spectrum one-dimensionally as shown in graph **2021.** In accordance therewith, the one dimensional sensor array **2014** can be disposed on the bottom surface **2005** on the image acquisition plate **1900** to simultaneously acquire cell images for each wavelength component as an image splitting mechanism **2** by the same procedure as the description for Figure **19F****.**

### <21. Process of image processing after simultaneously acquiring a high speed bright field microscope image and a high speed fluorescence microscope image>

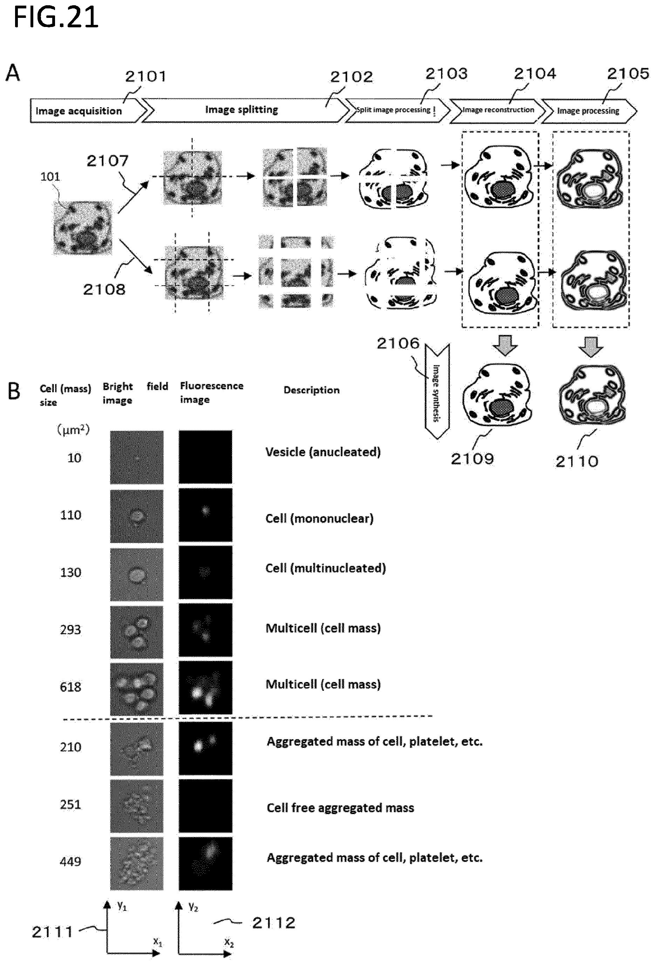

Figure **21** is a diagram that schematically shows the process of image processing after simultaneously acquiring a high speed bright field microscope image and a high speed fluorescence microscope image, and pictures that show exemplary images from simultaneously acquiring a high speed bright field microscope image and a high speed fluorescence microscope image from fluorescently staining a nucleus.

Figure **21A** is a schematic diagram illustrating the process of processing an image of a cell for actually analyzing an image. The cell image **101** acquired in image acquisition process **2101** is first divided into two or more different image splittings **2017** and **2018** in an image splitting process **2102.** Next, the offset is adjusted for each microimage divided at a split image processing process **2013,** and the maximum value and the minimum value of intensity are reconfigured to maximize the resolution of images. This is because optimization that selects the maximum value and minimum value and offset are averaged for larger images, so that a value that is optimal locally is not used. The probability of the optimization including the best split is improved by splitting images with a plurality of different patterns. When the processing of split images is completed in this manner, each of the split images can be returned again to the whole image at an image reconstruction process **2104.** The circumferential shape of a cell or microstructure within a cell can be extracted by binarization or differentiation of each of the whole images obtained in this manner at an image processing process **2105.** Further, a more accurate cell image can be acquired by synthesizing each of the whole reconstructed images and processed images obtained through the image splitting **2107** or **2108** at an image synthesis process **2106.**

Figure **21B** is a picture that shows exemplary images from simultaneously acquiring a high speed bright field microscope image and a high speed fluorescence microscope image from fluorescently staining a nucleus on a single high speed camera light receiving surface in the cell analysis device system of the present disclosure. As described above, the relative coordinates of two images can be matched in advance to compare which site in an image of a cell or cell cluster of a bright field image the nucleus is distributed for positions of nuclei that can be identified from a fluorescence image by using the relative coordinates of each other. It can be understood that a single nucleus is gleaming with fluorescence in a cell with a smooth surface and normal size in normal cells by comparing the relative coordinates. Meanwhile for cancer cells, multinucleation is an indicator of cancer cells. It can be understood that a plurality of nuclei are gleaming in a cell that have enlarged to be greater than normal cells from comparing the relative coordinates, as shown in the picture. Although not present in normal blood, fluorescence of a plurality of nuclei is observed within a cluster due to the fluorescence of a cell cluster and the nucleus of each cell in the cluster in blood in the case of a cancer metastasis. Thus, it can be understood that this is a cell cluster in view of such fluorescence of a plurality of nuclei. It is also possible to distinguish whether this is a cluster or an aggregated mass of cells and platelets or the like from the bright field image of the cluster.

In this manner, cancer cells in blood can be identified, and selectively collected, using a new biomarker, i.e., "image of the shape or population formation of cells, internal structure such as multinucleation, or the like", instead of conventional molecular biomarkers by (1) an approach of identifying, and selectively collecting, a cell cluster (mass) that is not present in healthy blood as a cancer cell candidate in blood, (2) an approach of identifying, and selectively collecting, a multinucleated cell that is not present in healthy blood as a cancer cell candidate in blood, (3) a method of identifying, and selectively collecting, a giant cell that is not present in healthy blood as a cancer cell candidate in blood, or (4) an approach of identifying, and selectively collecting, cancer cells by analysis combining detection of the presence of fluorescence intensity of a fluorescent antibody to one or more biomarkers (e.g., EpCam antibody, K-ras antibody, cytokeratine antibody, or the like) for a cancer cell measured from fluorescence intensity in addition to (1), (2), or (3), by using the device of the present disclosure.

Further, it is possible to subsequently identify whether a cancer cell candidate in blood collected by the approach described above is ultimately a cancer cell or, in case of a cancer cell, what characteristic of genetic mutation a cancer cell has by combining gene analysis means such as PCR analysis technology for small cells, and measuring a genetic mutation. With regard to (1), a candidate can be distinguished by, as described in the explanation of Figure **1****,** evaluating the circumferential length of a cell or cell mass from a bright field image, or evaluating the size from a bright field image and the number and distribution of nuclei from a fluorescence image (i.e., the distance of the centers of images of a plurality of adjacent nuclei are, for example, 3 µm or more from each other). With regard to (2), a candidate can be distinguished by, as described in the explanation of Figure **1****,** evaluating the circumferential length of a cell or cell mass from a bright field image, and evaluating the number and distribution of nuclei (i.e., the distance of the centers of images of a plurality of adjacent nuclei are, for example, 3 µm or more from one another). With regard to (3), a candidate can be distinguished from a bright field image by, as described in the explanation of Figure **1****,** evaluating the circumferential length of a cell or cell mass from a bright field image, and determining that the cell size exceeds, for example, 20 µm in terms of the diameter. Alternatively, a cell with one or more matching conditions from the combination of (1) to (3) can be determined as a cancer cell.

As can be understood from the examples in Figures **1** and **21****,** when the number of nuclei in a mass of cells (cluster) was measured from a fluorescence microscope image, samples with three or more fluorescent nuclei were only found in cells (Positive) in blood when cancer tissue was translated, while such samples could not be found in an image of cells (Control) in healthy blood for comparison. Thus, if three or more nuclei are found in a mass of cells (cluster), the cell (cluster) can be determined as a cancer cell. However, this indicates that cancer cells and normal cells cannot be identified from a sample with two or less nuclei as described above, so that three of more nuclei in a cell (cluster) is only one of the conditions sufficient for indicating the presence of a cancer cell cluster.

In view of these results, one of the following three determination conditions:
(1) an area of a nucleus of about 150 µm² or greater of a cell (cluster) is measured from an acquired image;
(2) an area of about 250 µm² or greater of a cell (cluster) is measured from an acquired image; and
(3) the presence of three of more nuclei of a cell (cluster) is measured from an acquired image;
or a combination of the three conditions described above, i.e., (1) and (2), (1) and (3), (2) and (3), or (1) and (2) and (3), can be used as criteria for determining the presence of a cancer cell in blood.

### (Cell analysis method)

The summary of the cell analysis device of the present disclosure and a system using the device is described above. Hereinafter, a method that can be achieved by using the device of the present disclosure or a part thereof is described.

The present disclosure provides a method of analyzing a cell derived from a subject, the method comprising the steps of:
a) acquiring an image of the cell;
b) generating flow rate data for the cell from the acquired image;
c) generating accurate cell shape data based on the flow rate data;
d) continuously analyzing information on a cell based on the cell shape data;
e) outputting a distribution of cell information on the entire test sample from information on a cell based on the cell shape data; and
f) distinguishing an abnormality in a cell of the subject from the distribution of the cell information.

An image of a cell can be acquired, for example, as described in Figure **1****.** In one embodiment, as described in Figure **1C** to Figure **1E****,** a high speed bright field microscope image and a high speed fluorescence microscope image from fluorescently staining a nucleus can be acquired as microscope pictures of two images simultaneously acquired by splitting one high speed camera light receiving surface. In one embodiment, a cell mass can be distinguished using only a bright field image by combining a procedure for extracting only an image of a cell or cell mass by subtracting background image data acquired in advance when the cell is not flowing from a bright field image of a cell cluster of for example Figure **1E** by image processing, and means for acquiring the length of a boundary line of the extracted image (circumferential line of a cell or cell mass) and an area of a region surrounded by the boundary line.

In one embodiment, the flow rate of each cell can be simultaneously measured in order to reconstruct an accurate shape of a cell or cell cluster from the acquired image of cells flowing at a high speed. Accurately reconstructed cell shape information from correcting information on the cell shape to match the information on the acquired flow rate of cells is acquired, and based on this result, determination is performed using an indicator such as the cell size, circumferential length, or internal structure based on the cell shape described in for example Figure **1****.** The flow rate of cells can be measured as described for the image acquisition mechanism described above.

In one embodiment, after acquiring information on cells as described above, an area size distribution diagram from observing white blood cell components after removing red blood cell components from blood (entire cells in blood remaining after removing red blood cells) with a bright field microscope can be generated as schematically shown in, for example, Figures **1F** to **1H****.** If this graph is used, a sample can be estimated to be blood of a metastatic cancer patient when, for example, the cell size increases at a point beyond 150 µm² (arrow **143**).

In one embodiment, as shown in Figure **1I** to **1K****,** the change in the cell size distribution in blood from before treatment of a blood sample of a metastatic cancer model (Copenhagen rat) to the completion of chemotherapy can be generated. It can be determined whether the size distribution is a size distribution for blood of a healthy model or a size distribution for blood of a metastatic cancer model, and the presence/absence of therapeutic effect can be determined by using such a graph.

In one embodiment, as shown in Figure **1M****,** the cell size distribution for blood of an animal model (Copenhagen rat) suffering from infection can be generated. Metastatic cancer and infection can be distinctly determined in blood diagnosis from the difference in the presence/absence of a clear increase in the region of cell size of 150 µm² to 200 µm² and a significant increase in cells with a cell size exceeding 300 µm².

In this manner, the presence of metastatic cancer in blood can be confirmed, and cancer cells in blood can be identified, and selective collected, with a new biomarker, i.e., "image of the shape or population formation of cells, internal structure such as multinucleation, or the like", instead of with a conventional molecular biomarker by (1) an approach of identifying a cell cluster (mass) that is not present in healthy blood as a cancer cell candidate in blood, (2) an approach of identifying, and selectively collecting, a multinucleated cell that is not present in healthy blood as a cancer cell candidate in blood, (3) a method of identifying, and selectively collecting, a giant cell that is not present in healthy blood as a cancer cell candidate in blood, (4) an approach of determining that a size distribution is characteristic to a metastatic cancer patient that is different from the characteristic of a healthy individual from a size distribution diagram for white blood cells in blood (all cells remaining after removing red blood cell component from blood) and a method of selectively collecting cells in a characteristic size distribution region, or (5) an approach of identifying, and selectively collecting, cancer cells by analysis combining detection of fluorescence intensity representing the presence of a fluorescently labeled antibody, which is prepared from fluorescently labeling an antibody to one or more biomarkers (e.g., EpCam antibody, K-ras antibody, cytokeratine antibody, or the like) for a cancer cell measured from fluorescence intensity with (1), (2), (3), or (4), from an image of a cell by using a cell sorting technology based on an image performed by a cell analysis device system for cell analysis performed using the cell analysis device system of the present disclosure. The present disclosure also provides a computer program for causing a computer to execute the above method, a recording medium storing such a program, and a system for executing such a method.

Specifically, another aspect of the present disclosure provides a computer program for causing a computer to execute processing of a method of analyzing a cell derived from a subject, the method comprising the steps of:
a) causing the computer to acquire an image of the cell;
b) causing the computer to generate flow rate data for the cell from the acquired image;
c) causing the computer to generate accurate cell shape data based on the flow rate data;
d) causing the computer to continuously analyze information on a cell based on the cell shape data;
e) causing the computer to output a distribution of cell information on the entire test sample from information on a cell based on the cell shape data; and
f) causing the computer to distinguish an abnormality in a cell of the subject from the distribution of the cell information.

Another aspect of the present disclosure provides a recording medium for storing a computer program for causing a computer to execute processing of a method of analyzing a cell derived from a subject, the method comprising the steps of:
a) causing the computer to acquire an image of the cell;
b) causing the computer to generate flow rate data for the cell from the acquired image;
c) causing the computer to generate accurate cell shape data based on the flow rate data;
d) causing the computer to continuously analyze information on a cell based on the cell shape data;
e) causing the computer to output a distribution of cell information on the entire test sample from information on a cell based on the cell shape data; and
f) causing the computer to distinguish an abnormality in a cell of the subject from the distribution of the cell information.

Another aspect of the present disclosure provides a system for analyzing a cell derived from a subject, comprising:
a) means for acquiring an image of the cell;
b) means for generating flow rate data for the cell from the acquired image;
c) means for generating accurate cell shape data based on the flow rate data;
d) means for continuously analyzing information on a cell based on the cell shape data;
e) means for outputting a distribution of cell information on the entire test sample from information on a cell based on the cell shape data; and
f) means for distinguishing an abnormality in a cell of the subject from the distribution of the cell information.

In view of the above, cells derived from a subject can be analyzed to determine the presence/absence of an abnormal cell by using the cell analysis device of the present disclosure or a module which is a part thereof.

Specifically, one aspect of the present disclosure provides a method of analyzing a cell derived from a subject, comprising the steps of:
(A) processing a cell contained in a cell sample solution derived from a subject;
(B) preparing a capsule particle by encapsulating the processed cell in a capsule;
(C) acquiring an image of the processed cell or the cell encapsulated in a capsule particle; and
(D) performing the method of analyzing a cell derived from a subject described above on the image for determination.

Another aspect of the present disclosure provides a method of determining the presence/absence of an abnormal cell in a cell sample derived from a subject, comprising the steps of:
(A) processing a cell contained in a cell sample solution derived from a subject;
(B) preparing a capsule particle by encapsulating the processed cell in a capsule; and
(C) determining the presence/absence of an abnormal cell in a cell sample derived from the subject, wherein the determination comprises the steps of:
   a) acquiring an image of the processed cell or the cell encapsulated in a capsule particle;
   b) generating flow rate data for the cell from the acquired image;
   c) generating accurate cell shape data based on the flow rate data;
   d) continuously analyzing information on a cell based on the cell shape data;
   e) outputting a distribution of cell information on the entire test sample from information on a cell based on the cell shape data; and
   f) distinguishing an abnormality in a cell of the subject from the distribution of the cell information.

In one embodiment, a cell processing can comprise sequentially performing processes including concentrating cells, fractionating cells by size, staining with a fluorescent antibody label (or optionally a reversible fluorescent label marker such as an aptamer for re-culture), and washing. In one embodiment, encapsulation of a cell can comprise encapsulating in a unit of a single cell or single cell mass.

In one embodiment, cell processing can, for example, concentrate and extract only cell components from blood collected from a patient, and continuously separate red blood cell components and other components to selectively collect white blood cell components (all other cells excluding red blood cell components in blood). Alternatively, especially if it is desirable to selectively collect white blood cell masses, only cell clusters can be selectively collected in the preprocessing stage by setting the threshold value of cell size to a cell size greater than 300 µm² in term of volume. After a fluorescent label agent such as a fluorescent cancer marker is added thereto and reacted with sample cells, unreacted excessive fluorescent label agent is washed and removed.

In one embodiment, cells can be encapsulated, for example, in a unit of a single cell or single cluster in an alginic acid capsule. Encapsulation of cells in an alginic acid gel capsule enables prevention of subsequent contamination of cells from the outside, and materializes additional improvement in performance that enables stable selective collection while preventing damage to cells inside a capsule by a stabilized and constant surface charge of the alginic acid capsule encapsulating the cells without being dependent on the surface charge of the cells upon selective collection using an electrophoretic force in an image detecting single cell separation/purification unit (cell sorting unit) of a cell detection/extraction unit at a later stage.

In one embodiment, detection can be performed using an indicator from two optical images for acquisition of an image of a cell. First, the outer shape of cells, the shape of intracellular organelle inside cells, the ratio of sizes of a nucleus and cytoplasm inside cells, and the status of cell masses can be checked from a bright field microscope image at a single cell level. In addition, the presence/absence of emission of fluorescence, and position and size thereof, based on a fluorescent label in coordinates corresponding to the position of a cell obtained in a bright field image can be checked.

In this manner, cells are consecutively treated with a cell sorting technology based on an image performed by a cell analysis device system and preprocessing technology for cells subjected to the cell sorting technology with regard to cell analysis performed using the cell analysis device system of the present disclosure, so that loss of a small amount of cells due to contamination or operation can be minimized, and cells can be detected and checked at a single cell level to confirm and determine whether the cells are isolated single cells that are not clustered or a clustered cell population from the size obtained from a characteristic of the shape and a two-dimensional image (volume of two dimensional image of volume) and the circumferential length by using a bright field microscope image for distinguishing the cell type. Further, the presence/absence of a fluorescent label of a cell with a fluorescently labeled antibody for distinguishing the cell type can be detected and confirmed at a single cell level, a fluorescently labeled cell can be confirmed to be an isolated single cell that is not clustered, and it is possible to determine whether apoptosis is occurring in a cell. The present disclosure also provides a computer program for causing a computer to execute the above method, a recording medium storing such a program, and a system for executing such a method.

Specifically, another aspect of the present disclosure provides a computer program for causing a computer to execute processing of a method of determining the presence/absence of an abnormal cell in a cell sample derived from a subject, the method comprising the steps of:
(A) causing the computer to process a cell contained in a cell sample solution derived from a subject;
(B) causing the computer to prepare a capsule particle by encapsulating the processed cell in a capsule; and
(C) causing the computer to determine the presence/absence of an abnormal cell in a cell sample derived from the subject, wherein the determination comprises the steps of:
   a) causing the computer to acquire an image of the processed cell or the cell encapsulated in a capsule particle;
   b) causing the computer to generate flow rate data for the cell from the acquired image;
   c) causing the computer to generate accurate cell shape data based on the flow rate data;
   d) causing the computer to continuously analyze information on a cell based on the cell shape data;
   e) causing the computer to output a distribution of cell information on the entire test sample from information on a cell based on the cell shape data; and
   f) causing the computer to distinguish an abnormality in a cell of the subject from the distribution of the cell information.

Another aspect of the present disclosure provides a recording medium for storing a computer program for causing a computer to execute processing of a method of determining the presence/absence of an abnormal cell in a cell sample derived from a subject, the method comprising the steps of:
(A) causing the computer to process a cell contained in a cell sample solution derived from a subject;
(B) causing the computer to prepare a capsule particle by encapsulating the processed cell in a capsule; and
(C) causing the computer to determine the presence/absence of an abnormal cell in a cell sample derived from the subject, wherein the determination comprises the steps of:
   a) causing the computer to acquire an image of the processed cell or the cell encapsulated in a capsule particle;
   b) causing the computer to generate flow rate data for the cell from the acquired image;
   c) causing the computer to generate accurate cell shape data based on the flow rate data;
   d) causing the computer to continuously analyze information on a cell based on the cell shape data;
   e) causing the computer to output a distribution of cell information on the entire test sample from information on a cell based on the cell shape data; and
   f) causing the computer to distinguish an abnormality in a cell of the subject from the distribution of the cell information.

Another aspect of the present disclosure provides a system for determining the presence/absence of an abnormal cell in a cell sample derived from a subject, comprising:
(A) means for processing a cell contained in a cell sample solution derived from a subject;
(B) means for preparing a capsule particle by encapsulating the processed cell in a capsule; and
(C) means for determining the presence/absence of an abnormal cell in a cell sample derived from the subject, wherein the determination comprises:
   a) means for acquiring an image of the processed cell or the cell encapsulated in a capsule particle;
   b) means for generating flow rate data for the cell from the acquired image;
   c) means for generating accurate cell shape data based on the flow rate data;
   d) means for continuously analyzing information on a cell based on the cell shape data;
   e) means for outputting a distribution of cell information on the entire test sample from information on a cell based on the cell shape data; and
   f) means for distinguishing an abnormality in a cell of the subject from the distribution of the cell information.

### (Note)

As described above, the present disclosure is exemplified by the use of its preferred embodiments. However, it is understood that the scope of the present disclosure should be interpreted solely based on the Claims. It is also understood that any patent, any patent application, and any other references cited herein should be incorporated herein by reference in the same manner as the contents are specifically described herein. The present application claims priority to Japanese Patent Application No. 2019-151212 filed on August 21, 2019 with the Japan Patent Office. The entire content thereof is incorporated herein by reference in the same manner as if the contents are specifically described herein.

### [Industrial Applicability]

The present disclosure can purify a small amount of target cells in blood in a unit of a single cell to materialize accurate analysis of genetic information and expression information on the target cells.

The present disclosure can identify whether cells targeted for a test is clustered (whether the cells are single isolated cell).

The present disclosure can determine whether apoptosis has occurred in a cell.

The present disclosure can separate/purify and collect only a target cell or cell population in real-time.

The present disclosure can measure the intracellular state at a single cell level to perform genome analysis and expression analysis at a single cell level, for only collected cells.

The present disclosure can re-culture only collected cells.

The present disclosure can obtain detailed information on cells such as the difference in sizes of cells, the ratio of the sizes of the nucleus to the cytoplasm inside a cell and distinguish cells based on the result thereof to purify cells.

The present disclosure can collect cells that are undergoing cell division in blood.

The present disclosure can effectively collect a multinucleated cell or cell cluster, which would be a candidate for a cancer cell circulating in blood.

The present disclosure enables simultaneous excitation of cells labeled with fluorescent antibodies of a plurality of wavelengths with excitation light of a plurality of wavelengths and enables simultaneous detection of a plurality of emitted fluorescent lights, so that target cells can be effectively collected.

The present disclosure can quantitatively identify and collect a cancer cell and a diseased organ tissue section from image data of cells in blood.

The present disclosure can selectively collect an immune cell in blood engulfing a foreign object or a foreign object in blood to diagnose an infection from genome analysis thereon.

Specifically, the present disclosure is useful for acquiring microparticles in a solution as an image and distinguish specific relevant microparticles from the shape and light absorbance property thereof and an image of a fluorescence property to selectively collect the microparticles.

The present disclosure is useful for purifying a small amount of target cells in blood in a unit of a single cell and performing accurate analysis on genetic information or expression information or the like on the target cells.

The present disclosure is also useful as a technology for identifying and/or collecting a cancer cell circulating in blood. The present disclosure is also useful for purifying a small amount of target cells causing an infection in a unit of a single cell and performing accurate analysis of genetic information or expression information or the like on the target cells at a high speed.

### [Reference Signs List]

**100:** input image
**101:** cell
**111:** bright field output image
**121:** output image of fluorescence
**301:** cell analysis device system 301
**310:** cell concentration/size fractionation/staining/washing module
**320:** cell/cell mass encapsulation module
**330:** image detecting single cell separation/purification module
**340:** genetic analysis/expression analysis unit
**350:** contamination free re-culturing module
**360:** control/analysis module (computer)
**400:** cell concentration/size fractionation/staining/washing unit
**401:** cell sample reservoir
**402:** staining agent reservoir
**403:** detergent reservoir
**406:** concentration/bleaching filter
**500:** microchannel of a cell concentration/size fractionation/staining/washing unit
**503:** pillar array
**504:** pair of electrodes
**507:** channel
**800:** cell/cell mass encapsulation unit
**1300:** cube container
**1301:** window
**1304:** dichroic mirror
**1305:** angle adjustment mechanism
**1306:** filter
**1311:** image size adjustment system unit
**1312:** movable shielding plate
**1313:** lens
**1314:** high speed camera
**1315:** lens
**1316:** image imaging element
**1401:** fluorescence photometer
**1402:** fluorescence photometer
**1600:** image detecting single cell separation/purification unit
**1605:** side sheath flow
**1606:** cell observation region
**1607:** gel electrode
**1608:** channel
**1609:** inlet
**1610:** outlet
**1611:** electric wire
**1613:** electric wire
**1900:** image acquisition plate
**1902:** channel
**1905:** flow rate detection one dimensional sensor array
**1906:** first image acquiring one dimensional sensor array
**1907:** second image acquiring one dimensional sensor array
**1908:** band-pass filter
**1909:** band-pass filter
**1911:** optical sensor element
**1912:** one dimensional sensor array element

## Claims

1. A cell analysis device system, comprising:
(A) a first device for processing of purification, concentration, stain, and/or wash of cells in a candidate size region from a cell sample solution from a subject;
(B) a second device for preparing a capsule particle encapsulating the cells processed by the first device in a capsule;
(C) a third device for acquiring and distinguishing images of the cells processed by the first device or the cells encapsulated in a capsule particle by the second device, continuously acquiring a flow rate of the cells as flow rate data, acquiring an accurate cell shape based on the flow rate data, continuously analyzing information in the images of the cells based on the cell shape, outputting a distribution of cell information for an entire amount of test sample, and distinguishing and collecting a target cell; and
(D) a control/determination unit for controlling an operation of each of the first to third devices to perform determination on the cell sample solution.

2. The cell analysis device system of claim 1, wherein
(a) the first device comprises a chamber comprising a membrane filter for concentrating, staining, and washing cells obtained from a cell sample solution from a subject, containers respectively housing a solution comprising the cells, a staining solution, and a detergent, and a cell concentration/staining/washing mechanism for sequentially introducing each solution in each of the containers into the chamber, or
(a') the first device comprises an alternating electric field application mechanism comprising: a pillar array capable of selectively and continuously fractionating the cells in the cell sample solution by size, wherein the pillar array has a space adjusted to match a cell size to be fractionated and is disposed in a microchannel with a slope with respect to a flow in a channel; and a pair of electrodes, which can apply a sinusoidal alternating electric field to a microchannel, disposed to oppose both side wall surfaces that are orthogonal to the microchannel;
(b) the second device comprises a capsule particle construction mechanism for constructing capsule particles of alginic acid comprising cells by discharging a solution comprising alginic acid in a sol state and the cells from an outlet of a microtube into a solution comprising a divalent ion, and comprises a capsule particle size sorting mechanism for applying a small current to the inside and outside of the microtube, and measuring and controlling a discharge rate from a change in a value of resistance to align particle sizes of capsule particles of alginic acid, and a cell/capsule particle collection mechanism for distinguishing a capsule particle comprising a cell and a capsule particle that does not comprise a cell to selectively collect a capsule particle comprising a cell; and
(c) the third device is a module having an image detecting single cell separation/purification unit (cell sorting unit) comprising a channel for allowing a sample solution of cells comprising a target cell or capsule particle containing a target cell to flow, the channel comprising a merging region where the channel merges with a sheath solution from both sides for allowing cells or capsule particles arranged in one line to flow downstream, a detection/sorting region where the cells or capsule particles aligned in one line are detected and the target cell is sorted, a combination of channels for collecting target cells by applying an ionic current to move cells or capsule particles comprising a cell and selectively moving the cells or capsule particles to a branched channel continuing from the merging region, and an ionic current application tool for applying an ionic current, wherein the module comprises, at the merging region, an optical tool for determining a flow rate of a cell and an analysis tool for acquiring and analyzing a characteristic from an image of a cell corrected based on an optically obtained flow rate of a cell.

3. The cell analysis device system of claim 1 or 2, wherein
the third device is a module having an image detecting single cell separation/purification unit comprising a channel for allowing a sample solution comprising a target cell or capsule particle to flow, the channel having a merging region where the channel merges with a sheath solution from both sides for allowing the cells or capsule particles arranged in one line to flow downstream, and an observation region where the cells or capsule particles aligned in one line are detected,
wherein the module comprises: a light source that can be temporally controlled so that light is emitted during an irradiation period in an irradiation region for irradiating light of two or more different wavelengths for a shorter period of time than an interval of image capture time with a camera, with each different length of time; a condenser optical system for irradiating light from the light source onto the irradiation region; an image capturing camera mechanism for splitting an obtained image of two or more different wavelengths by a difference in wavelengths and acquiring images as images of each wavelength; a cell flow rate acquisition mechanism for acquiring a flow rate of a cell from comparing a difference in irradiation periods of the light source and lengths of the resulting images of two or more wavelengths in a direction of flow of a cell; a cell shape correction mechanism for correcting obtained cell shape information from the acquired flow rate of a cell; a cell analysis tool for acquiring and analyzing a characteristic of a cell from a shape of a cell corrected based on an optically obtained flow rate of a cell; and an image blur suppression mechanism for suppressing an image blur by controlling a flash time of a light source in a relation of "flash time of a light source = pixel size of a camera acquiring an image/obtained flow rate of a cell" from the obtained flow rate.

4. The cell analysis device system of claim 3, wherein
the third device is a module having an image detecting single cell separation/purification unit comprising, downstream of the observation region, a combination of channels for collecting target cells or capsule particles comprising target cells by applying an ionic current to move cells or capsule particles and selectively moving the cells or capsule particles to a branched channel continuing from the merging region, and an ionic current application tool for applying an ionic current,
wherein the module comprises an application timing controlling tool for controlling a timing of applying an ionic current to a cell or capsule particle to be collected based on a flow rate acquired by the cell flow rate acquisition mechanism in the merging region.

5. The cell analysis device system of claim 3 or 4, wherein the third device uses fluorescence for the light source and an observed image.

6. The cell analysis device system of claim 1 or 2, wherein
the third device is a module having an image detecting single cell separation/purification unit comprising a channel for allowing a sample solution comprising target cells or capsule particles to flow, the channel having a merging region where the channel merges with a sheath solution from both sides for allowing the cells arranged in one line to flow downstream, and an observation region where the cells or capsule particles aligned in one line are detected,
wherein the module comprises an image reconstruction mechanism having a condenser optical system for continuously irradiating light for observing cells or capsule particles onto the irradiation region; a flow rate measuring one dimensional photosensor array disposed along a flow of cells or capsule particles on an image acquisition surface for forming the resulting image; and a cell image acquiring one dimensional photosensor array with a length that can cover a channel width in an orientation that is orthogonal to a flow of a cell and acquire all cell images at a bottom end thereof, wherein a flow rate is computed from measured moving rate information on a cell image of the flow rate measuring one dimensional photosensor array and the rate information and data acquisition time are combined to reconstruct two dimensional image information from information of the cell image acquiring one dimensional photosensor array.

7. The cell analysis device system of claim 6, wherein
the third device is a module having an image detecting single cell separation/purification unit comprising, downstream of the observation region, a combination of channels for collecting target cells or capsule particles comprising target cells by applying an ionic current to move cells or capsule particles and selectively moving the cells or capsule particles to a branched channel continuing from the merging region, and an ionic current application tool for applying an ionic current,
wherein the module comprises an application timing controlling tool for controlling a timing of applying an ionic current to cells or capsule particles to be collected based on a flow rate acquired by calculating the flow rate in the merging region.

8. The cell analysis device system of claim 6 or 7, wherein the third device uses fluorescence for the light source and an observed image.

9. The cell analysis device system of any one of claims 6 to 8, wherein the third device has an image blur suppression mechanism that can simultaneously acquire images at different image formation heights by disposing and arranging in parallel at one or more different heights, in addition to the cell image acquiring one dimensional photosensor array, on the image acquisition surface.

10. The cell analysis device system of any one of claims 6 to 9, wherein the third device has an image splitting mechanism **1** that can simultaneously acquire images of a plurality of different wavelength bands by splitting a wavelength of the light source into a plurality of wavelengths, and disposing a plurality of cell image acquiring one dimensional photosensor arrays on the image acquisition surface in addition to the cell image acquiring one dimensional photosensor array and disposing a band-pass filter that allows only light with a specific wavelength to pass through on each one dimensional photosensor array.

11. The cell analysis device system of any one of claims 6 to 10, wherein the third device has a wavelength spectrum separation mechanism for separating a wavelength of the light source into a plurality of wavelengths and separating a linear light of a band-like region that is orthogonal to an obtained flow as a wavelength spectrum, and an image splitting mechanism **2** that can simultaneously acquire images of a plurality of different wavelength bands by disposing the wavelength spectrum and each cell image acquiring one dimensional photosensor array at a position of respective wavelength spectrum on the image acquisition surface.

12. A cell analysis method for measuring a distribution of sizes, circumferential lengths, and/or particle amount ratios of an internal microstructure of a shape of a cell or microparticle in a solution at a full amount to determine the presence/absence of an abnormality from a change in the distribution by using the cell analysis device system of claims 1 to 11.

13. A method of analyzing a cell derived from a subject, the method comprising the steps of:
a) acquiring an image of the cell;
b) generating flow rate data for the cell from the acquired image;
c) generating accurate cell shape data based on the flow rate data;
d) continuously analyzing information on a cell based on the cell shape data;
e) outputting a distribution of cell information on the entire test sample from information on a cell based on the cell shape data; and
f) distinguishing an abnormality in a cell of the subject from the distribution of the cell information.

14. A computer program for causing a computer to execute processing of a method of analyzing a cell derived from a subject, the method comprising the steps of:
a) causing the computer to acquire an image of the cell;
b) causing the computer to generate flow rate data for the cell from the acquired image;
c) causing the computer to generate accurate cell shape data based on the flow rate data;
d) causing the computer to continuously analyze information on a cell based on the cell shape data;
e) causing the computer to output a distribution of cell information on the entire test sample from information on a cell based on the cell shape data; and
f) causing the computer to distinguish an abnormality in a cell of the subject from the distribution of the cell information.

15. A recording medium for storing a computer program for causing a computer to execute processing of a method of analyzing a cell derived from a subject, the method comprising the steps of:
a) causing the computer to acquire an image of the cell;
b) causing the computer to generate flow rate data for the cell from the acquired image;
c) causing the computer to generate accurate cell shape data based on the flow rate data;
d) causing the computer to continuously analyze information on a cell based on the cell shape data;
e) causing the computer to output a distribution of cell information on the entire test sample from information on a cell based on the cell shape data; and
f) causing the computer to distinguish an abnormality in a cell of the subject from the distribution of the cell information.

16. A system for analyzing a cell derived from a subject, comprising:
a) means for acquiring an image of the cell;
b) means for generating flow rate data for the cell from the acquired image;
c) means for generating accurate cell shape data based on the flow rate data;
d) means for continuously analyzing information on a cell based on the cell shape data;
e) means for outputting a distribution of cell information on the entire test sample from information on a cell based on the cell shape data; and
f) means for distinguishing an abnormality in a cell of the subject from the distribution of the cell information.

17. Use of at least one indicator selected from the group consisting of a size of a cell, a shape of a cell, presence/absence of formation of a population, i.e., whether cells form an aggregate (cluster), a population size (number and type of constituent cells), a size of a nucleus within cells, and presence/absence of a multinucleated cell, for cell analysis.

18. A method of determining whether a cell is a nucleated cell and/or a multinucleated cell, comprising simultaneously acquiring an image of a bright field cell shape of the cell and a fluorescence image in the cell as an image of at least one wavelength.

19. A method of distinguishing a cell mass, comprising combining:
acquiring background image data from when cells are not flowing;
acquiring bright field image data from when cells are flowing;
extracting an image of only a cell mass by subtracting the background image data from the bright field image data; and
acquiring a length of a boundary line of the extracted image (circumferential line of a cell or a cell mass) and an area of a region surrounded by the boundary line;
to extract data for a cell mass.

20. A method of identifying a cancer cell in blood, comprising at least one step selected from the group consisting of:
(1) identifying a cell cluster (mass), which is not present in healthy blood, as the presence of a cancer cell in blood;
(2) identifying a multinucleated cell, which is not present in healthy blood, as the presence of a cancer cell in blood;
(3) identifying a giant cell, which is not present in healthy blood, as the presence of a cancer cell in blood; and
(4) identifying a size distribution that is characteristic to a metastatic cancer patient, which is different from a characteristic of a healthy individual, from a size distribution diagram of white blood cells in blood (all cells remaining after removing red blood cell components from blood) as the presence of a cancer cell; and optionally
(5) identifying a cancer cell by analysis combining the presence of a fluorescence intensity of a fluorescent antibody to one or more biomarkers (e.g., EpCam antibody, K-ras antibody, cytokeratine antibody, or the like) of a cancer cell measured from fluorescence intensity.

21. A method of analyzing a cell derived from a subject, comprising the steps of:
(A) processing a cell contained in a cell sample solution derived from a subject;
(B) preparing capsule particles by encapsulating the processed cell in a capsule;
(C) acquiring an image of the processed cell or the cell encapsulated in a capsule particle; and
(D) performing the method of claim 13 on the image for determination.

22. The method of claim 21, wherein the step of processing comprises purifying, concentrating, staining, and/or washing a cell of a candidate size region.

23. The method of claim 21 or 22, wherein the step of processing selectively and continuously fractionates cells in the cell sample solution by size.

24. The method of any one of claims 21 to 23, wherein the step of preparing capsule particles constructs capsule particles of alginic acid comprising a cell by mixing a solution comprising alginic acid in a sol state and the cell in a solution comprising a divalent ion.

25. The method of any one of claims 21 to 24, wherein the step of preparing capsule particles aligns particle sizes of the capsule particles of alginic acid, and distinguishes a capsule particle comprising a cell and a capsule particle that does not comprise a cell to selectively collect a capsule particle comprising a cell.

26. The method of claim 25, wherein the collection collects a target cell or capsule particle comprising a cell by applying an ionic current to a cell or capsule particle comprising a cell.

27. The method of any one of claims 21 to 26, wherein the step of distinguishing optically determines a flow rate of a cell, and acquires and analyzes a characteristic from an image of a cell corrected based on the optically obtained flow rate of a cell.

28. The method of any one of claims 21 to 27 for measuring a distribution of sizes, circumferential lengths, and/or particle amount ratios of an internal microstructure of a shape of a cell in the cell sample solution at a full amount to determine the presence/absence of an abnormality from a change in the distribution.

29. The method of any one of claims 21 to 28 for separating/identifying an abnormal cell in a cell sample derived from a subject.

30. A method of determining the presence/absence of an abnormal cell in a cell sample derived from a subject, comprising the steps of:
(A) processing a cell contained in a cell sample solution derived from a subject;
(B) preparing capsule particles by encapsulating the processed cell in a capsule; and
(C) determining the presence/absence of an abnormal cell in a cell sample derived from the subject, wherein the determination comprises the steps of:
a) acquiring an image of the processed cell or the cell encapsulated in a capsule particle;
b) generating flow rate data for the cell from the acquired image;
c) generating accurate cell shape data based on the flow rate data;
d) continuously analyzing information on a cell based on the cell shape data;
e) outputting a distribution of cell information on the entire test sample from information on a cell based on the cell shape data; and
f) distinguishing an abnormality in a cell of the subject from the distribution of the cell information.

31. The method of claim 30, wherein the step of processing comprises purifying, concentrating, staining, and/or washing a cell of a candidate size region.

32. The method of claim 30 or 31, wherein the step of processing selectively and continuously fractionates cells in the cell sample solution by size.

33. The method of any one of claims 30 to 32, wherein the step of preparing capsule particles constructs capsule particles of alginic acid comprising a cell by mixing a solution comprising alginic acid in a sol state and the cell in a solution comprising a divalent ion.

34. The method of any one of claims 30 to 33, wherein the step of preparing capsule particles aligns particle sizes of the capsule particles of alginic acid, and distinguishes a capsule particle comprising a cell and a capsule particle that does not comprise a cell to selectively collect a capsule particle comprising a cell.

35. The method of claim 34, wherein the collection collects a target cell or capsule particle comprising a cell by applying an ionic current to a cell or capsule particle comprising a cell.

36. The method of any one of claims 30 to 35, wherein the step of distinguishing optically determines a flow rate of a cell, and acquires and analyzes a characteristic from an image of a cell corrected based on the optically obtained flow rate of a cell.

37. A computer program for causing a computer to execute processing of a method of determining the presence/absence of an abnormal cell in a cell sample derived from a subject, the method comprising the steps of:
(A) causing the computer to process a cell contained in a cell sample solution derived from a subject;
(B) causing the computer to prepare a capsule particle by encapsulating the processed cell in a capsule; and
(C) causing the computer to determine the presence/absence of an abnormal cell in a cell sample derived from the subject, wherein the determination comprises the steps of:
a) causing the computer to acquire an image of the processed cell or the cell encapsulated in a capsule particle;
b) causing the computer to generate flow rate data for the cell from the acquired image;
c) causing the computer to generate accurate cell shape data based on the flow rate data;
d) causing the computer to continuously analyze information on a cell based on the cell shape data;
e) causing the computer to output a distribution of cell information on the entire test sample from information on a cell based on the cell shape data; and
f) causing the computer to distinguish an abnormality in a cell of the subject from the distribution of the cell information.

38. A recording medium for storing a computer program for causing a computer to execute processing of a method of determining the presence/absence of an abnormal cell in a cell sample derived from a subject, the method comprising the steps of:
(A) causing the computer to process a cell contained in a cell sample solution derived from a subject;
(B) causing the computer to prepare a capsule particle by encapsulating the processed cell in a capsule; and
(C) causing the computer to determine the presence/absence of an abnormal cell in a cell sample derived from the subject, wherein the determination comprises the steps of:
a) causing the computer to acquire an image of the processed cell or the cell encapsulated into a capsule particle;
b) causing the computer to generate flow rate data for the cell from the acquired image;
c) causing the computer to generate accurate cell shape data based on the flow rate data;
d) causing the computer to continuously analyze information on a cell based on the cell shape data;
e) causing the computer to output a distribution of cell information on the entire test sample from information on a cell based on the cell shape data; and
f) causing the computer to distinguish an abnormality in a cell of the subject from the distribution of the cell information.

39. A system for determining the presence/absence of an abnormal cell in a cell sample derived from a subject, comprising:
(A) means for processing a cell contained in a cell sample solution derived from a subject;
(B) means for preparing a capsule particle by encapsulating the processed cell in a capsule; and
(C) means for determining the presence/absence of an abnormal cell in a cell sample derived from the subject, wherein the determination comprises:
a) means for acquiring an image of the processed cell or the cell encapsulated into a capsule particle;
b) means for generating flow rate data for the cell from the acquired image;
c) means for generating accurate cell shape data based on the flow rate data;
d) means for continuously analyzing information on a cell based on the cell shape data;
e) means for outputting a distribution of cell information on the entire test sample from information on a cell based on the cell shape data; and
f) means for distinguishing an abnormality in a cell of the subject from the distribution of the cell information.
